# EUROPÄISCHE PATENTSCHRIFT

(11) **EP 3 117 470 B1**
(45) Veröffentlichungstag und Bekanntmachung des Hinweises auf die Patenterteilung: **23.12.2020**
(21) Anmeldenummer: 15705914.8
(22) Anmeldetag: 18.02.2015
(51) Int. Cl.: H01L 51/50

(54) **ORGANISCHE ELEKTROLUMINESZENZVORRICHTUNG**
ORGANIC ELECTROLUMINESCENT DEVICE
DISPOSITIF ÉLECTROLUMINESCENT ORGANIQUE

(30) Priorität: 13.03.2014 EP 14000911
(43) Veröffentlichungstag der Anmeldung: 18.01.2017
(73) Patentinhaber: Merck Patent GmbH, 64293 Darmstadt (DE)
(72) Erfinder: STOESSEL, Philipp, 60389 Frankfurt am Main (DE); PARHAM, Amir Hossain, 60486 Frankfurt am Main (DE); PFLUMM, Christof, 64291 Darmstadt (DE); JATSCH, Anja, 60489 Frankfurt am Main (DE); KAISER, Joachim, 64289 Darmstadt (DE)
(86) Internationale Anmeldenummer: PCT/EP2015/000360
(87) Internationale Veröffentlichungsnummer: WO 2015/135624

(56) Entgegenhaltungen:
- WO-A1-2014/013947
- US-A1- 2012 248 968

## Beschreibung

Die vorliegende Erfindung betrifft organische Elektrolumineszenzvorrichtungen, welche in der emittierenden Schicht eine Mischung aus einem lumineszenten Material mit einem geringen Singulett-Triplett-Abstand und einem fluoreszierenden Emissionsmaterial mit einer hohen sterischen Abschirmung enthalten.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei insbesondere auch metallorganische Iridium- und Platinkomplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich.

Trotz der guten Ergebisse, die mit metallorganischen Iridium- und Platinkomplexen erzielt werden, weisen diese jedoch auch eine Reihe von Nachteilen auf: So handelt es sich bei Iridium und Platin um seltene und teure Metalle. Es wäre daher zur Ressourcenschonung wünschenswert, die Verwendung dieser Metalle vermeiden zu können. Weiterhin gibt es insbesondere bei der Lebensdauer phosphoreszierender OLEDs, welche Ir- oder Pt-Emitter enthalten, Verbesserungsbedarf, wenn es beim Betreiben der OLED zu höheren Temperaturen kommt, wie dies bei einigen Anwendungen der Fall ist.

Eine alternative Entwicklung ist die Verwendung von Emittern, die thermisch aktivierte verzögerte Fluoreszenz (thermally activated delayed fluorescence, TADF) zeigen (z. B. H. Uoyama et al., Nature 2012, Vol. 492, 234). Hier handelt es sich um organische Materialien, bei denen der energetische Abstand zwischen dem niedrigsten Triplettzustand T₁ und dem ersten angeregten Singulettzustand S₁ ausreichend klein ist, so dass der S₁-Zustand aus dem T₁-Zustand thermisch zugänglich ist. Aus quantenstatistischen Gründen entstehen bei elektronischer Anregung in der OLED die angeregten Zustände zu 75 % im Triplettzustand und zu 25 % im Singulettzustand. Da rein organische Moleküle üblicherweise nicht effizient aus dem Triplettzustand emittieren können, können 75 % der angeregten Zustände nicht für die Emission genutzt werden, wodurch prinzipiell nur 25 % der Anregungsenergie in Licht umgewandelt werden kann. Ist nun jedoch der energetische Abstand zwischen dem niedrigsten Triplettzustand und dem niedrigsten angeregten Singulettzustand ausreichend klein, so ist aus dem Triplettzustand durch thermische Anregung der erste angeregte Singulettzustand des Moleküls zugänglich und kann thermisch besetzt werden. Da dieser Singulettzustand ein emissiver Zustand ist, aus dem Fluoreszenz möglich ist, kann dieser Zustand zur Erzeugung von Licht verwendet werden. Somit ist prinzipiell die Umwandlung von bis zu 100 % der elektrischen Energie in Licht bei der Verwendung rein organischer Materialien als Emitter möglich. So wird im Stand der Technik eine externe Quanteneffizienz von mehr als 19 % beschrieben, was in derselben Größenordnung wie für phosphoreszierende OLEDs liegt. Somit ist es mit derartigen rein organischen Materialien möglich, sehr gute Effizienzen zu erreichen und gleichzeitig die Verwendung seltener Metalle wie Iridium oder Platin zu vermeiden.

Im Stand der Technik wird die Verwendung verschiedener Matrixmaterialien in Kombination mit Emittern, die thermisch aktivierte verzögerte Fluoreszenz zeigen (im Folgenden TADF-Verbindung genannt), beschrieben, beispielsweise Carbazolderivate (H. Uoyama et al., Nature 2012, 492, 234; Endo et al., Appl. Phys. Lett. 2011, 98, 083302; Nakagawa et al. Chem. Commun. 2012, 48, 9580; Lee et al. Appl. Phys. Lett. 2012, 101, 093306/1), Phosphinoxid-Dibenzothiophenderivate (H. Uoyama et al., Nature 2012, 492, 234) oder Silanderivate (Mehes et al., Angew. Chem. Int. Ed. 2012, 51, 11311; Lee et al., Appl. Phys. Lett. 2012, 101, 093306/1).

Den im Stand der Technik beschriebenen Elektrolumineszenzvorrichtungen ist gemeinsam, dass die TADF-Verbindung als emittierende Verbindung in der emittierenden Schicht eingesetzt wird. Da eine Voraussetzung für das Vorliegen einer TADF-Verbindung ein geringer Abstand zwischen dem T₁-und dem S₁-Niveau sind, ist man in der Wahl der TADF-Verbindung limitiert. So kann es schwierig sein, TADF-Verbindungen mit jeder gewünschten Emissionsfarbe bereit zu stellen. Daher wäre es wünschenswert, die Vorteile von TADF zu nutzen, aber dennoch die üblicherweise verwendeten Grundstrukturen fluoreszierender Emitter verwenden zu können, um sich deren positive Eigenschaften zu Nutze machen zu können. Fluoreszierende Emitter sind in allen Emissionsfarben verfügbar, so dass dies auch zu einer größeren Auswahl an emittierenden Verbindungen führen würde. Außerdem weisen TADF-Verbindungen häufig ein breites Emissionsspektrum auf, so dass es wünschenswert wäre, für eine größere Farbreinheit emittierende Verbindungen mit einem schmaleren Emissionsspektrum zur Verfügung zu haben, wie dies häufig bei gängigen fluoreszierenden Emittern gegeben ist. Außerdem sind weitere Verbesserungen der Lebensdauer wünschenswert.

Überraschend wurde gefunden, dass organische Elektrolumineszenzvorrichtungen, die in der emittierenden Schicht eine TADF-Verbindung und eine fluoreszierende Verbindung aufweisen, wobei die fluoreszierende Verbindung eine hohe sterische Abschirmung gegenüber ihrer Umgebung aufweist, wie unten genauer definiert, diese Aufgabe lösen. Durch diesen Deviceaufbau lassen sich organische Elektrolumineszenzvorrichtungen bereitstellen, die in allen Emissionsfarben emittieren, indem die Grundstrukturen bekannter fluoreszierender Emitter verwendet werden können, die aber dennoch die hohe Effizienz von Elektrolumineszenzvorrichtungen mit TADF zeigen. Derartige organische Elektrolumineszenzvorrichtungen sind daher der Gegenstand der vorliegenden Erfindung.

In US 2012/248968 wird eine organische Elektrolumineszenzvorrichtung beschrieben, die in der emittierenden Schicht eine TADF-Verbindung und eine fluoreszierende Verbindung enthält. Es ist nicht offenbart, dass die fluoreszierende Verbindung sterisch abgeschirmt ist. Hier sind weitere Verbesserungen, insbesondere in Bezug auf die Effizienz, wünschenswert. Ahnliche organische Elektrolumineszenzvorrichtungen werden ebenfalls in WO 2014/013947 A1 beschrieben.

Gegenstand der vorliegenden Erfindung ist eine organische Elektrolumineszenzvorrichtung, enthaltend Kathode, Anode und mindestens eine emittierende Schicht, die eine sterisch abgeschirmte fluoreszierende Verbindung mit einem Abschirmparameter S ≤ 0.6 enthält, dadurch gekennzeichnet, dass die emittierende Schicht oder eine an die emittierende Schicht angrenzende Schicht eine lumineszente organische Verbindung enthält, die einen Abstand zwischen dem niedrigsten Triplettzustand T₁ und dem ersten angeregten Singulettzustand S₁ von ≤ 0.30 eV aufweist (TADF-Verbindung), wobei die Peakemissionswellenlänge der sterisch abgeschirmten fluoreszierenden Verbindung größer oder gleich der Peakemissionswellenlänge der TADF-Verbindung ist.

Die sterisch abgeschirmte fluoreszierende Verbindung wird in der folgenden Beschreibung auch lediglich als fluoreszierende Verbindung bezeichnet. Was im Sinne der vorliegenden Erfindung unter einer sterisch abgeschirmten Verbindung verstanden wird und wie bestimmt werden kann, ob es sich um eine sterisch abgeschirmte Verbindung handelt, ist in der folgenden Beschreibung sowie im Beispielteil ausführlich erläutert.

Wie die Peakemissionswellenlänge im Sinne der vorliegenden Anmeldung bestimmt wird, ist im Beispielteil allgemein erläutert.

In einer bevorzugten Ausführungsform der Erfindung ist die Peakemissionswellenlänge der sterisch abgeschirmten fluoreszierenden Verbindung mindestens 10 nm größer als die der TADF-Verbindung, besonders bevorzugt mindestens 20 nm größer und ganz besonders bevorzugt mindestens 30 nm größer.

In einer Ausführungsform der Erfindung enthält die emittierende Schicht eine Mischung aus der sterisch abgeschirmten fluoreszierenden Verbindung und der TADF-Verbindung.

In einer weiteren Ausführungsform der Erfindung enthält die Elektrolumineszenzvorrichtung angrenzend an die emittierende Schicht auf Anodenseite eine Schicht, welche die TADF-Verbindung enthält.

In nochmals einer weiteren Ausführungsform der Erfindung enthält die Elektrolumineszenzvorrichtung angrenzend an die emittierende Schicht auf Kathodenseite eine Schicht, welche die TADF-Verbindung enthält.

In einer bevorzugten Ausführungsform der Erfindung enthält die emittierende Schicht außer den oben aufgeführten Materialien weiterhin mindestens eine weitere Verbindung. Diese weitere Verbindung wird im Folgenden als Matrixverbindung bzw. Matrixmaterial bezeichnet. Es kann sich dabei um eine weitere TADF-Verbindung im Sinne der oben aufgeführten Definition handeln. Im Allgemeinen ist die Matrixverbindung aber keine TADF-Verbindung.

Im Folgenden wird die lumineszente Verbindung, die einen Abstand zwischen dem niedrigsten Triplettzustand T₁ und dem ersten angeregten Singulettzustand S₁ von ≤ 0.30 eV aufweist, näher beschrieben. Hierbei handelt es sich um eine Verbindung, die TADF (thermally activated delayed fluorescence) zeigt. Diese Verbindung wird in der folgenden Beschreibung mit "TADF-Verbindung" abgekürzt.

Bei der TADF-Verbindung handelt es sich bevorzugt um eine organische Verbindung. Eine organische Verbindung im Sinne der vorliegenden Erfindung ist eine kohlenstoffhaltige Verbindung, die keine Metalle enthält. Insbesondere ist die organische Verbindung aus den Elementen C, H, D, B, Si, N, P, O, S, F, Cl, Br und I aufgebaut.

Eine lumineszente Verbindung im Sinne der vorliegenden Erfindung ist eine Verbindung, die in der Lage ist, unter optischer Anregung in einer Umgebung, wie sie in der organischen Elektrolumineszenzvorrichtung vorliegt, bei Raumtemperatur Licht zu emittieren. Dabei weist die Verbindung bevorzugt eine Lumineszenzquanteneffizienz von mindestens 40 % auf, besonders bevorzugt von mindestens 50 %, ganz besonders bevorzugt von mindestens 60 % und insbesondere bevorzugt von mindestens 70 %. Dabei wird die Lumineszenzquanteneffizienz bestimmt in einer Schicht, wie sie in der organischen Elektrolumineszenzvorrichtung verwendet werden soll, die jedoch die fluoreszierende Verbindung nicht enthält. Wie die Bestimmung der Lumineszenzquantenausbeute im Sinne der vorliegenden Erfindung durchgeführt wird, ist im Beispielteil ausführlich allgemein beschrieben.

Weiterhin ist es bevorzugt, wenn die Schicht, welche die TADF-Verbindung enthält, eine kurze Abklingzeit aufweist. Dabei ist die Abklingzeit bevorzugt ≤ 50 µs, besonders bevorzugt ≤ 20 µs, ganz besonders bevorzugt ≤ 10 µs und insbesondere bevorzugt ≤ 5 µs. Wie die Bestimmung der Abklingzeit im Sinne der vorliegenden Erfindung durchgeführt wird, ist im Beispielteil ausführlich allgemein beschrieben.

Die Energie des niedrigsten angeregten Singulettzustands (S₁) und des niedrigsten Triplettzustands (T₁) werden durch quantenchemische Rechnung bestimmt. Wie diese Bestimmung im Sinne der vorliegenden Erfindung durchgeführt wird, ist im Beispielteil ausführlich allgemein beschrieben.

Wie oben beschrieben, darf der Abstand zwischen S₁ und T₁ maximal 0.30 eV betragen, damit es sich bei der Verbindung um eine TADF-Verbindung im Sinne der vorliegenden Erfindung handelt, wobei es umso besser ist, je kleiner der Abstand ist. Bevorzugt ist daher der Abstand zwischen S₁ und T₁ ≤ 0.25 eV, besonders bevorzugt ≤ 0.15 eV, ganz besonders bevorzugt ≤ 0.10 eV und insbesondere bevorzugt ≤ 0.08 eV.

Bei der TADF-Verbindung handelt es sich bevorzugt um eine aromatische Verbindung, die sowohl Donor- wie auch Akzeptorsubstituenten aufweist, wobei das LUMO und das HOMO der Verbindung räumlich nur schwach überlappen. Was unter Donor- bzw. Akzeptorsubstituenten verstanden wird, ist dem Fachmann prinzipiell bekannt. Geeignete Donorsubstituenten sind insbesondere Diaryl- bzw. -heteroarylaminogruppen sowie Carbazolgruppen bzw. Carbazolderivate, die jeweils bevorzugt über N an die aromatische Verbindung gebunden sind. Dabei können diese Gruppen auch weiter substituiert sein. Geeignete Akzeptorsubstituenten sind insbesondere Cyanogruppen, aber auch beispielsweise elektronenarme Heteroarylgruppen, die auch weiter substituiert sein können, wie beispielsweise substituierte oder unsubstituierte Triazingruppen.

Wenn die emittierende Schicht außer der TADF-Verbindung und der sterisch abgeschirmten fluoreszierenden Verbindung eine Matrixverbindung enthält, ist es bevorzugt, um Exziplexbildung in der emittierenden Schicht zu vermeiden, wenn für LUMO(TADF), also das LUMO der TADF-Verbindung, und das HOMO(Matrix), also das HOMO der Matrixverbindung, gilt:
LUMO(TADF) - HOMO(Matrix) ≥ S₁(TADF) - 0.4 eV;
besonders bevorzugt:
LUMO(TADF) - HOMO(Matrix) ≥ S₁(TADF) - 0.3 eV;
und ganz besonders bevorzugt:
LUMO(TADF) - HOMO(Matrix) ≥ S₁(TADF) - 0.2 eV.

Dabei ist S₁(TADF) der erste angeregte Singulettzustand S₁ der TADF-Verbindung.

Im Folgenden werden die bevorzugten Dotierkonzentrationen der TADF-Verbindung in der emittierenden Schicht beschrieben. Aufgrund der unterschiedlichen Herstellung der organischen Elektrolumineszenzvorrichtung wird die Dotierkonzentration bei Herstellung der emittierenden Schicht durch Aufdampfen in Vol.-% und bei Herstellung der emittierenden Schicht aus Lösung in Gew.-% angegeben. Die Dotierkonzentrationen in Vol.-% und Gew.-% sind im Allgemeinen sehr ähnlich.

In einer bevorzugten Ausführungsform der Erfindung liegt bei Herstellung der emittierenden Schicht durch Aufdampfen die TADF-Verbindung in einer Dotierkonzentration von 1 bis 25 Vol.-% in der emittierenden Schicht vor, besonders bevorzugt von 2 bis 20 Vol.-%, ganz besonders bevorzugt von 5 bis 15 Vol.-% und insbesondere 7 bis 12 Vol.-%.

In einer bevorzugten Ausführungsform der Erfindung liegt bei Herstellung der emittierenden Schicht aus Lösung die TADF-Verbindung in einer Dotierkonzentration von 1 bis 25 Gew.-% in der emittierenden Schicht vor, besonders bevorzugt von 2 bis 20 Gew.-%, ganz besonders bevorzugt von 5 bis 15 Gew.-% und insbesondere 7 bis 12 Vol.-%.

Es gehört zum allgemeinen Fachwissen des Fachmanns, welche Materialien sich generell als TADF-Verbindungen eignen. Folgende Referenzen offenbaren beispielsweise Materialien, die potentiell als TADF-Verbindungen geeignet sind:
- Tanaka et al., Chemistry of Materials 25(18), 3766 (2013).
- Lee et al., Journal of Materials Chemistry C1(30), 4599 (2013).
- Zhang et al., Nature Photonics advance online publication, 1 (2014), doi: 10.1038/nphoton.2014.12.
- Serevicius et al., Physical Chemistry Chemical Physics 15(38), 15850 (2013).
- Li et al., Advanced Materials 25(24), 3319 (2013).
- Youn Lee et al., Applied Physics Letters 101(9), 093306 (2012).
- Nishimoto et al., Materials Horizons 1, 264 (2014), doi: 10.1039/C3MH00079F.
- Valchanov et al., Organic Electronics, 14(11), 2727 (2013).
- Nasu et al., ChemComm, 49, 10385 (2013).
Weiterhin offenbaren die folgenden Patentanmeldungen potentielle TADF Verbindungen: WO 2013/154064, WO 2013/133359, WO 2013/161437, WO 2013/081088, WO 2013/081088, WO 2013/011954, JP 2013/116975 und US 2012/0241732.

Weiterhin kann der Fachmann diesen Veröffentlichungen Designprinzipien für TADF-Verbindungen entnehmen. Z.B. zeigen Valchanov et al., wie sich die Farbe von TADF-Verbindungen anpassen lässt.

Beispiele für geeignete Moleküle, die TADF zeigen, sind die in der folgenden Tabelle aufgeführten Strukturen.

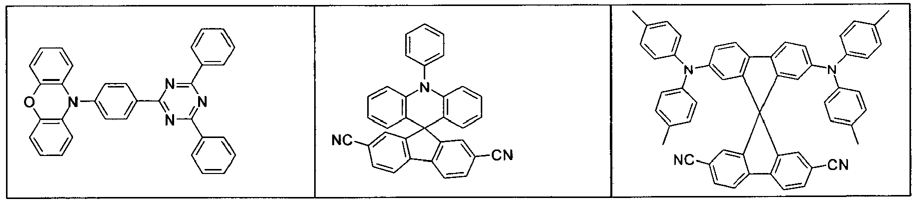

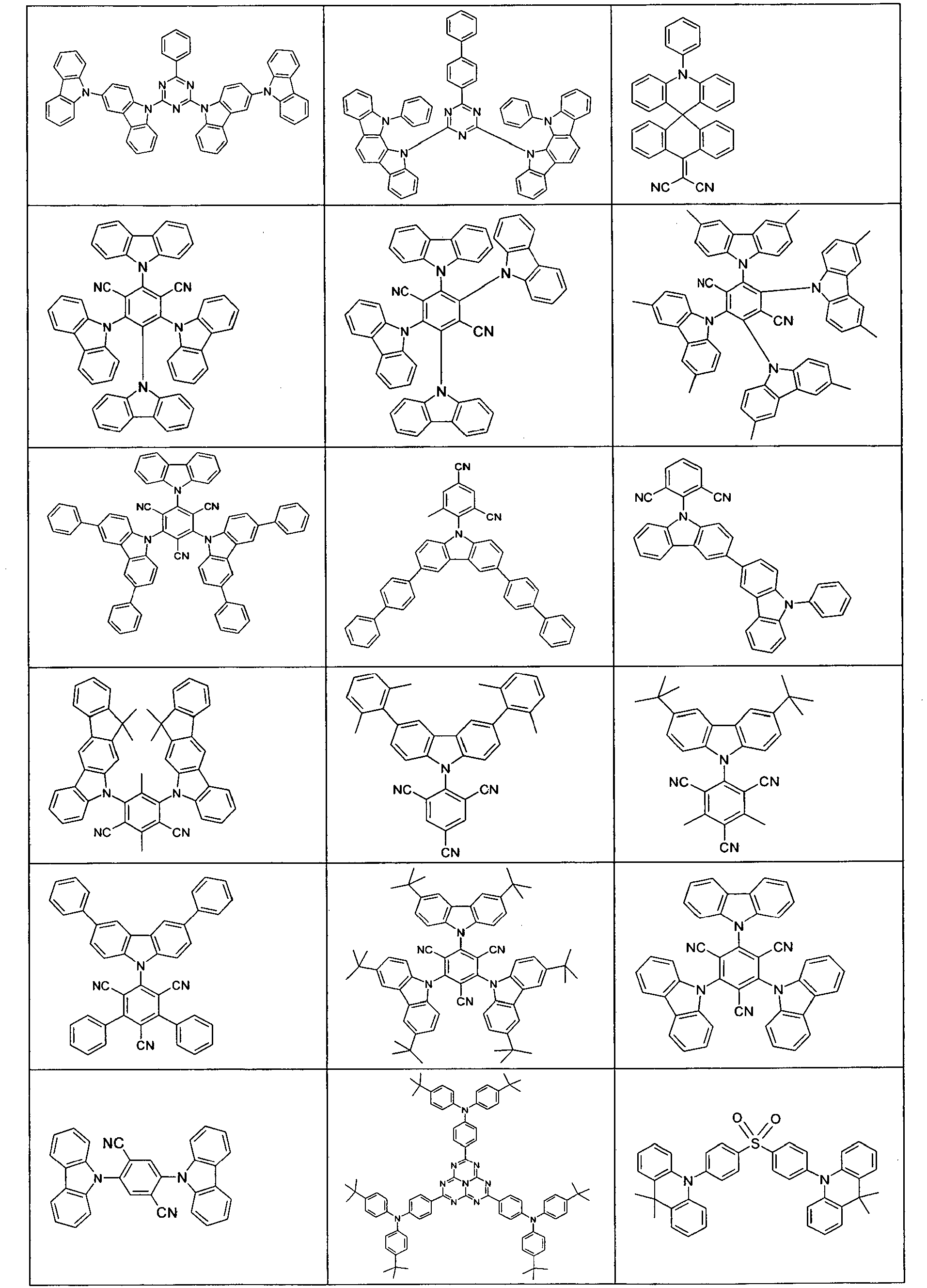

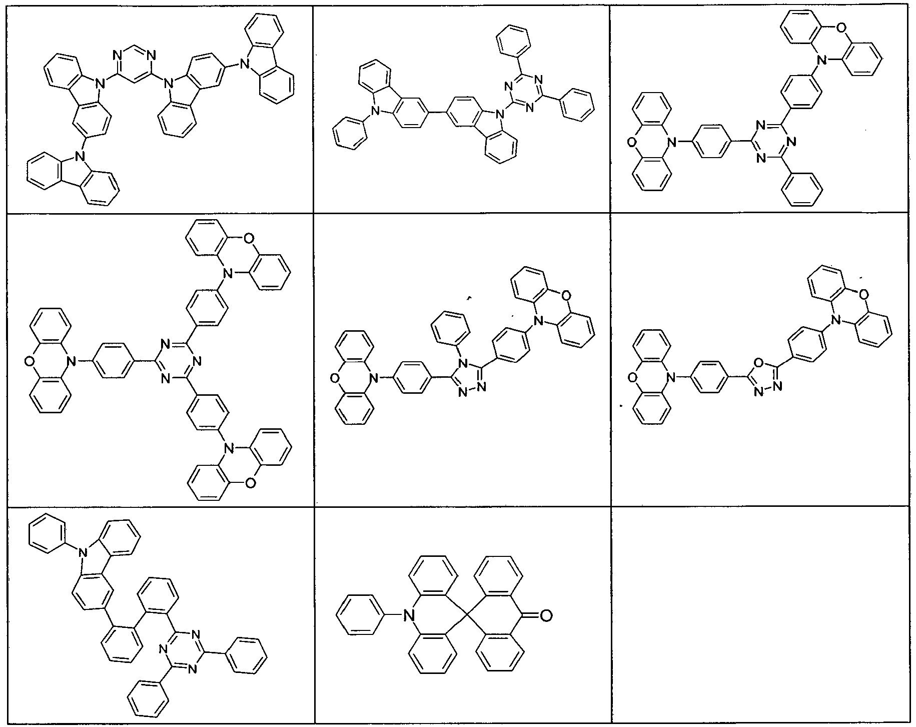

Im Folgenden wird die sterisch abgeschirmte fluoreszierende Verbindung genauer beschrieben.

Bei der fluoreszierenden Verbindung handelt es sich bevorzugt um eine organische Verbindung. Eine organische Verbindung im Sinne der vorliegenden Erfindung ist eine kohlenstoffhaltige Verbindung, die keine Metalle enthält. Insbesondere ist die organische Verbindung aus den Elementen C, H, D, B, Si, N, P, O, S, F, Cl, Br und I aufgebaut. In einer weiteren Ausführungsform der Erfindung kann die fluoreszierende Verbindung auch ein fluoreszierender Metallkomplex sein, beispielsweise ein Aluminiumkomplex.

Eine fluoreszierende Verbindung im Sinne der vorliegenden Erfindung ist eine Verbindung, die in der Lage ist, unter optischer Anregung in einer Umgebung, wie sie in der organischen Elektrolumineszenzvorrichtung vorliegt, bei Raumtemperatur Licht zu emittieren, wobei die Emission aus einem angeregten Singulettzustand erfolgt. Dabei weist die Verbindung bevorzugt eine Lumineszenzquanteneffizienz von mindestens 60 % auf, besonders bevorzugt von mindestens 80 %, ganz besonders bevorzugt von mindestens 90 % und insbesondere bevorzugt von mindestens 95 %. Dabei wird die Lumineszenzquanteneffizienz in einer Lösung in Toluol bestimmt. Wie die Bestimmung der Lumineszenzquantenausbeute im Sinne der vorliegenden Erfindung durchgeführt wird, ist im Beispielteil ausführlich allgemein beschrieben.

In einer bevorzugten Ausführungsform liegt die Peakemissionswellenlänge der fluoreszierenden Verbindung zwischen 430 und 650 nm. Wie die Bestimmung der Peakemissionswellenlänge im Sinne der vorliegenden Erfindung durchgeführt wird, ist im Beispielteil ausführlich allgemein beschrieben.

Aufgrund der unterschiedlichen Herstellung der organischen Elektrolumineszenzvorrichtung wird die Dotierkonzentration der fluoreszierenden Verbindung bei Herstellung der emittierenden Schicht durch Aufdampfen in Vol.-% und bei Herstellung der emittierenden Schicht aus Lösung in Gew.-% angegeben.

In einer bevorzugten Ausführungsform der Erfindung liegt bei Herstellung der emittierenden Schicht durch Aufdampfen die fluoreszierende Verbindung in einer Dotierkonzentration von 0.1 bis 25 Vol.-% in der emittierenden Schicht vor, bevorzugt von 1 bis 20 Vol.-%, besonders bevorzugt von 2 bis 12 Vol.-%, ganz besonders bevorzugt 5 bis 10 Vol.-% und insbesondere 7 bis 10 Vol.-%.

In einer bevorzugten Ausführungsform der Erfindung liegt bei Herstellung der emittierenden Schicht aus Lösung die fluoreszierende Verbindung in einer Dotierkonzentration von 0.1 bis 25 Gew.-% in der emittierenden Schicht vor, bevorzugt von 1 bis 20 Gew.-%, besonders bevorzugt von 2 bis 12 Gew.-%, ganz besonders bevorzugt 5 bis 10 Gew.-% und insbesondere 7 bis 10 Vol.-%.

Dabei ist es möglich, dass insbesondere bei einer geringen Dotierkonzentration der fluoreszierenden Verbindung die OLED eine Mischemission aus der fluoreszierenden Verbindung und einer Restemission der TADF-Verbindung zeigt. Dies kann auch gezielt zur Erzeugung von Mischfarben genutzt werden.

Als Grundgerüste für die fluoreszierende Verbindung können alle Verbindungen verwendet werden, wie sie gemäß dem Stand der Technik für fluoreszierende OLEDs eingesetzt werden.

Die sterische Abschirmung dieser Verbindungen erfolgt dabei durch elektronisch inerte, sterisch anspruchsvolle Substituenten, die den elektronisch aktiven Kern der fluoreszierenden Verbindung umgeben und so weitgehend vom Kontakt zu benachbarten Molekülen in der Schicht abschirmen.

Der Grad der sterischen Abschirmung kann durch einen Abschirmparameter S bestimmt werden. Wie dieser Abschirmparameter im Sinne der vorliegenden Anmeldung über quantenchemische Rechnung bestimmt wird, ist im Beispielteil allgemein beschrieben. In diesen sterischen Abschirmparameter gehen unterschiedliche Moleküleigenschaften ein. Zunächst wird die fluoreszierende Verbindung in aktive Teile und eine isolierende Hülle eingeteilt. Diese Einteilung erfolgt darüber, zu welchem Anteil die Atome an HOMO oder LUMO der Verbindung bzw. an anderen elektronisch aktiven Molekülorbitalen beteiligt sind. Wenn deren Beteiligung an den relevanten Molekülorbitalen geringer ist als ein Grenzwert, der wie hinten definiert ist, so werden diese Atome definitionsgemäß der isolierenden Hülle zugeordnet. Dann wird die so genannte Connolly-Oberfläche der fluoreszierenden Verbindung bestimmt. Dies erfolgt dadurch, dass eine Kugel einer bestimmten Größe, im vorliegenden Fall 4 Å, auf der Oberfläche der fluoreszierenden Verbindung gerollt wird. Dies simuliert, wie gut ein anderes Molekül, beispielsweise die TADF-Verbindung, sich an die fluoreszierende Verbindung annähern kann, was wichtig ist für die Bestimmung, ob ein Dexter-Energietransfer zwischen den Verbindungen stattfinden kann oder nicht. Dexter-Energietransfer kann dabei nur an dem Teil der Oberfläche der fluoreszierenden Verbindung stattfinden, die ausreichend nah an dem aktiven Kern der fluoreszierenden Verbindung liegt, also nicht ausreichend abgeschirmt ist. Unter der Abschirmung im Sinne der vorliegenden Erfindung wird das Verhältnis der Oberfläche, an der ein Dexter-Energietransfer stattfinden kann, zur gesamten Oberfläche der fluoreszierenden Verbindung angesehen, wobei diese Größe nicht nur von der fluoreszierenden Verbindung selbst, sondern auch von der verwendeten TADF-Verbindung abhängt. Wenn dieser Anteil kleiner einem bestimmten Grenzwert ist, wie er hinten definiert ist, so wird die Verbindung im Sinne der vorliegenden Erfindung als sterisch abgeschirmt angesehen.

Definitionsgemäß gilt eine Verbindung als sterisch abgeschirmt im Sinne der vorliegenden Erfindung, wenn der Abschirmparameter S, wie er hinten im Beispielteil definiert ist, ≤ 0.6 ist, bevorzugt ≤ 0.55, besonders bevorzugt ≤ 0.5, ganz besonders bevorzugt ≤ 0.4, noch stärker bevorzugt ≤ 0.3 und insbesondere bevorzugt ≤ 0.2.

Wie oben beschrieben, können als fluoreszierender Kern der fluoreszierenden Verbindung alle Grundstrukturen eingesetzt werden, wie sie üblicherweise als fluoreszierende Emitter in organischen Elektrolumineszenzvorrichtungen eingesetzt werden. Bevorzugt als fluoreszierende Verbindungen sind steife π-Systeme, die mit großen aliphatischen oder cycloaliphatischen Resten substituiert sind. Weiterhin kommen auch aromatische Substituenten, die durch aliphatische oder cycloaliphatische Reste substituiert sein können, in Frage, wenn diese sterisch so angeordnet sind, dass es sich nicht um aktive Gruppen im Sinne der vorliegenden Erfindung handeln, dass deren Anteil an den relevanten Molekülorbitalen also unter einem Grenzwert, wie hinten beschrieben, liegt. Dagegen sind beispielsweise Arylaminosubstituenten oder Heteroarylgruppen zu stark an HOMO oder LUMO beteiligt und eignen sich somit nicht als abschirmende Gruppen.

Beispiele für geeignete aromatische Grundkörper fluoreszierender Verbindungen sind die im Folgenden aufgeführten Gruppen der Formeln (1) bis (50),

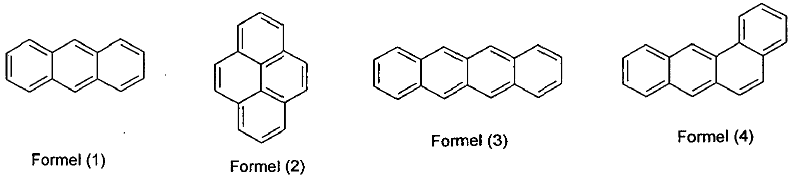

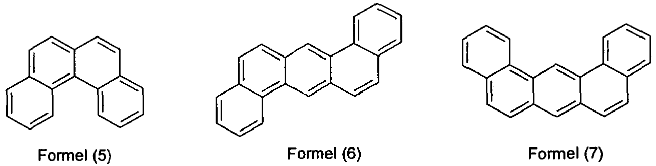

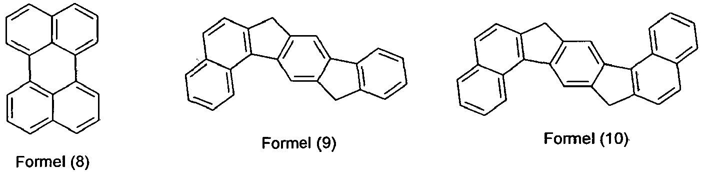

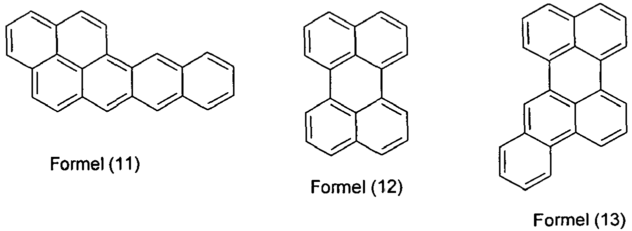

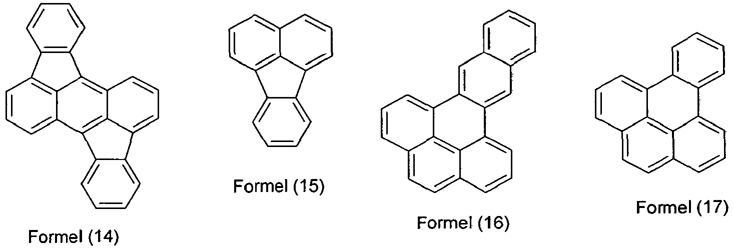

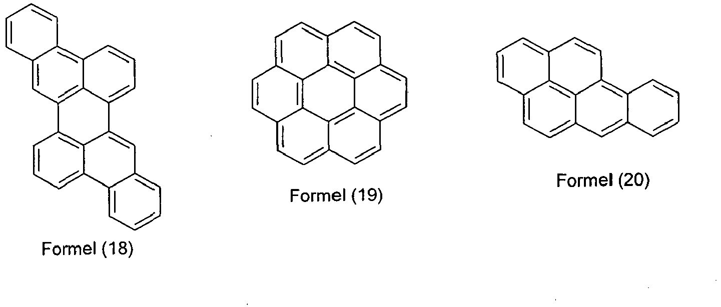

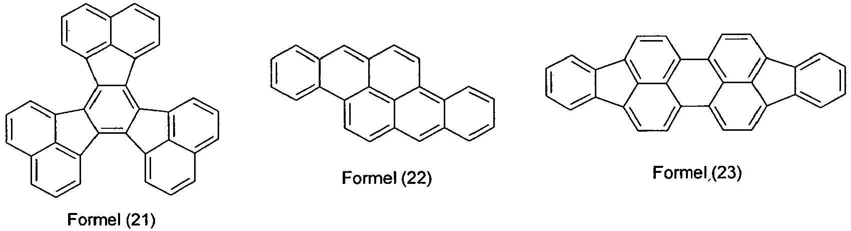

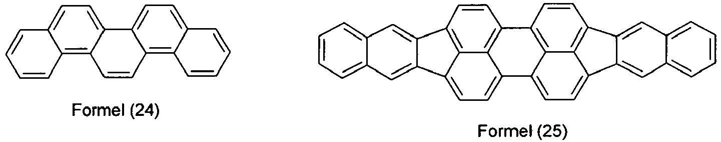

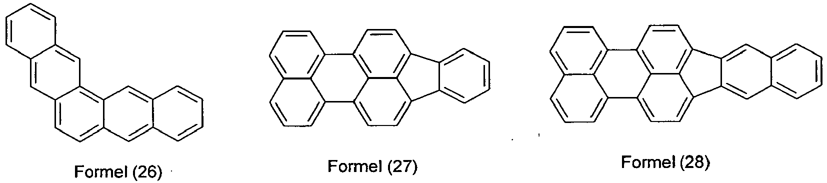

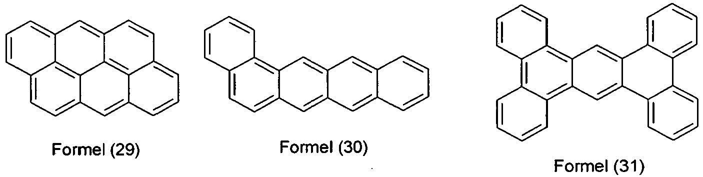

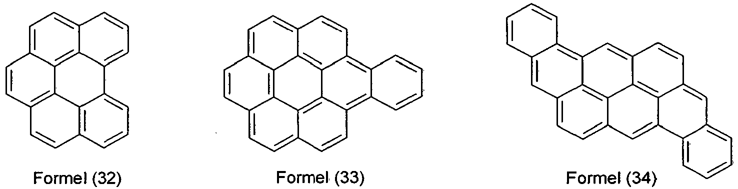

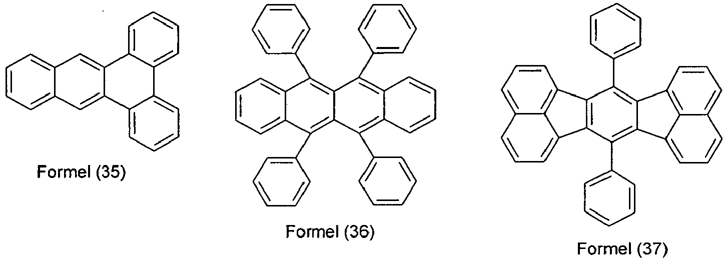

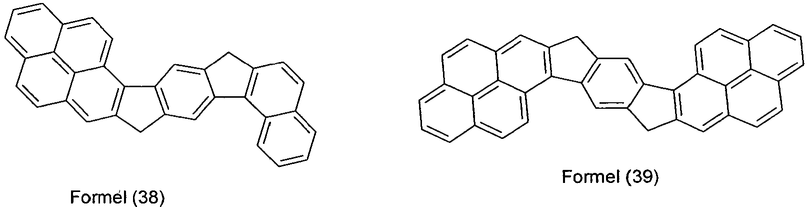

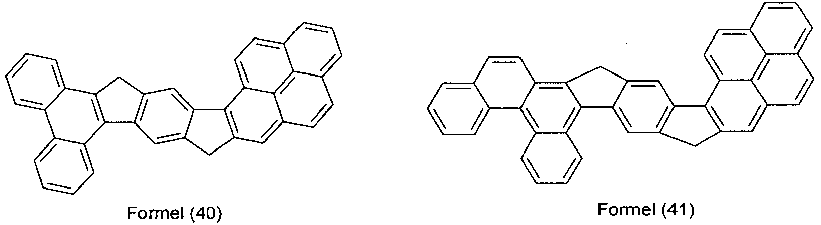

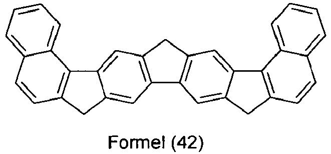

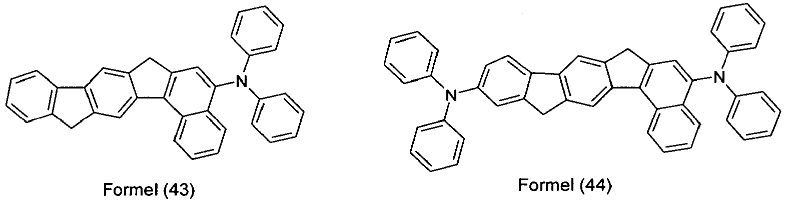

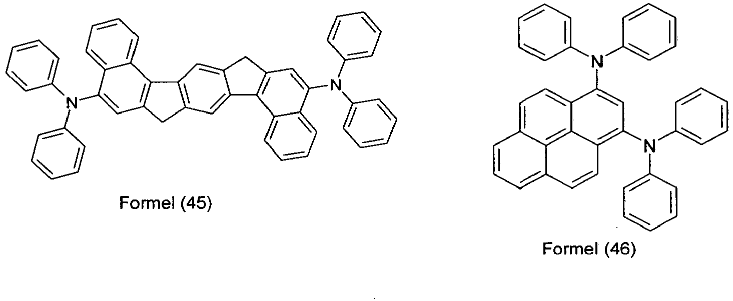

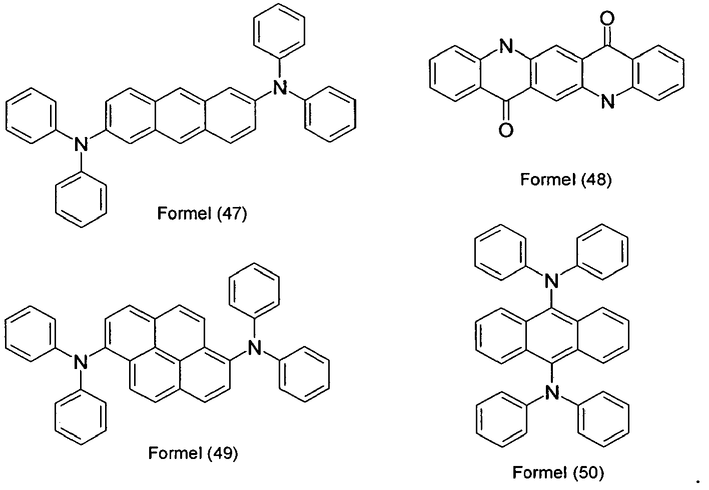

Beispiele für geeignete heteroaromatische Grundkörper fluoreszierender Verbindungen sind die oben genannten Gruppen der Formeln (1) bis (50), in denen ein, zwei, drei oder vier Kohlenstoffatome des kondensierten aromatischen Kerns durch Stickstoff ersetzt sind. Bevorzugt sind ein, zwei oder drei Kohlenstoffatome durch Stickstoff ersetzt, besonders bevorzugt ein oder zwei Kohlenstoffatome und ganz besonders bevorzugt genau ein Kohlenstoffatom.

Beispiele für geeignete heteroaromatische Grundkörper fluoreszierender Verbindungen sind weiterhin die im Folgenden aufgeführten Gruppen der Formeln (51) bis (70),

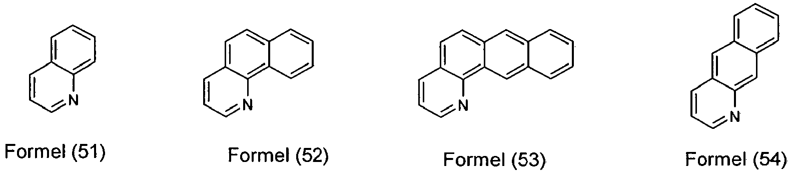

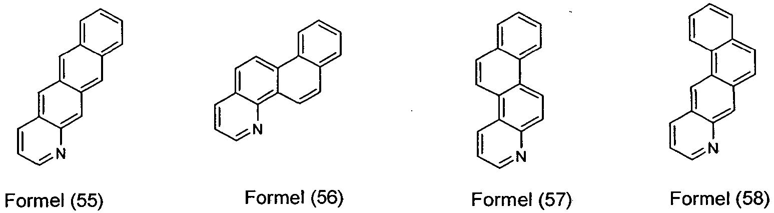

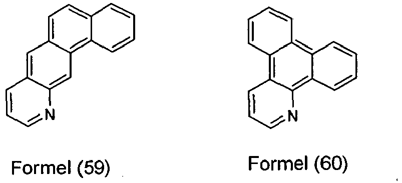

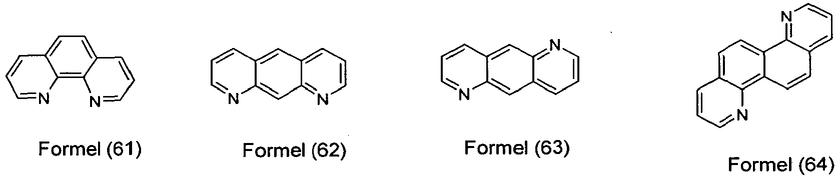

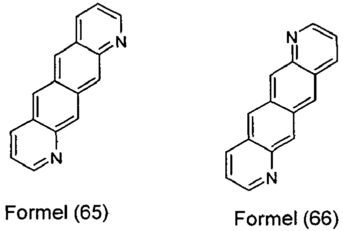

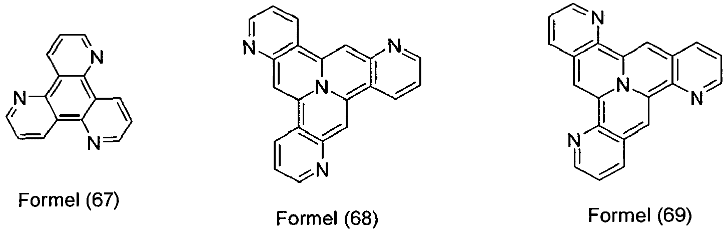

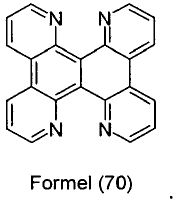

Dabei sind diese Strukturen, wie oben beschrieben, durch sterisch anspruchsvolle Substituenten substituiert und können auch durch weitere Substituenten, die nicht als sterisch anspruchsvoll angesehen werden können, substituiert sein, sofern diese Substituenten nicht elektronisch aktiv sind bzw. wiederum durch sterisch abgeschirmte Substituenten substituiert sind.

Im Folgenden werden geeignete, sterisch anspruchsvolle Substituenten beschrieben, die verwendet werden können, um die fluoreszierenden Kerne, z. B. die oben genannten Aromaten und Heteroaromaten, zu substituieren und so zu sterisch abgeschirmten fluoreszierenden Verbindungen zu gelangen.

Geeignete sterisch anspruchsvolle Substituenten sind beispielsweise Alkylgruppen, insbesondere mit 3 bis 20 C-Atomen, bevorzugt mit 4 bis 10 C-Atomen, in denen auch H-Atome durch F ersetzt sein können, Alkoxygruppen, insbesondere mit 3 bis 20 C-Atomen, bevorzugt mit 4 bis 10 C-Atomen, Aralkylgruppen, insbesondere mit 7 bis 30 C-Atomen, und aromatische Ringsysteme, insbesondere mit 6 bis 30 C-Atomen, wobei in den Aralkylgruppen und aromatischen Ringsystemen die Arylgruppen auch durch ein oder mehrere Alkylgruppen mit 1 bis 10 C-Atomen substituiert sein können. Dabei können auch mehrere benachbarte Substituenten ein Ringsystem miteinander bilden.

Wenn es sich bei dem Substituenten um eine Aralkylgruppe oder ein aromatisches Ringsystem handelt, so ist es bevorzugt, wenn diese keine kondensierten Arylgruppen mit mehr als 10 Kohlenstoffatomen aufweisen, in denen Arylgruppen über eine gemeinsame Kante direkt aneinander ankondensiert sind. Besonders bevorzugt weist dieses überhaupt keine kondensierten Arylgruppen auf, in denen Arylgruppen über eine gemeinsame Kante direkt aneinander ankondensiert sind. So ist es also bevorzugt, wenn das aromatische Ringsystem beispielsweise keine Anthracen- oder Pyrengruppen aufweist, und besonders bevorzugt, wenn das aromatische Ringsystem auch keine Naphthalingruppen aufweist. Dagegen kann es beispielsweise Biphenyl- oder Terphenylgruppen aufweisen, da diese keine kondensierten Arylgruppen aufweisen. Weiterhin kann es auch beispielsweise Fluoren- oder Spirobifluorengruppen aufweisen, da in diesen Gruppen keine Arylgruppen direkt über eine gemeinsame Kante aneinander ankondensiert sind.

Wenn der sterisch anspruchsvolle Substituent für eine Alkylgruppe steht, dann weist diese Alkylgruppe bevorzugt 4 bis 10 C-Atome auf. Bevorzugt handelt es sich um eine sekundäre, tertiäre oder cyclische Alkylgruppe, bei der das sekundäre oder tertiäre C-Atom entweder direkt an den fluoreszierenden Grundkörper gebunden ist oder über eine CH₂-Gruppe an den fluoreszierenden Grundkörper gebunden ist. Besonders bevorzugt ist diese Alkylgruppe ausgewählt aus den Strukturen der folgenden Formeln (R-1) bis (R-33): 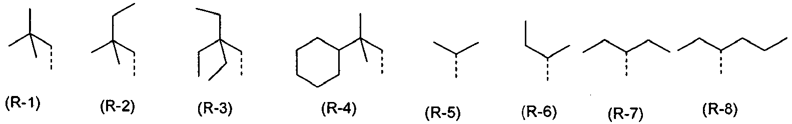 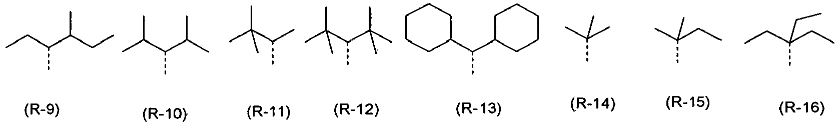 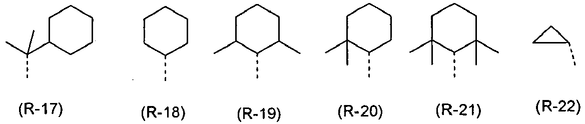 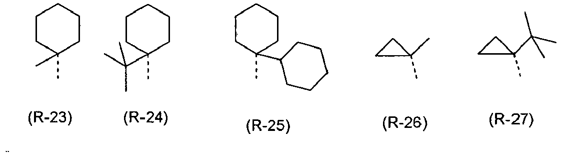 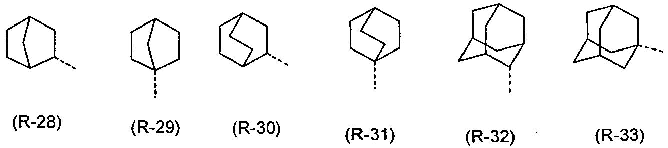 wobei die gestrichelte Bindung die Anknüpfung dieser Gruppen an den fluoreszierenden Grundkörper kennzeichnet.

Wenn der sterisch anspruchsvolle Substituent für eine Alkoxygruppe steht, dann weist diese Alkoxygruppe bevorzugt 3 bis 10 C-Atome auf und ist bevorzugt verzweigt oder cyclisch. Bevorzugt ist diese Alkoxygruppe ausgewählt aus den Strukturen der folgenden Formeln (R-34) bis (R-47): 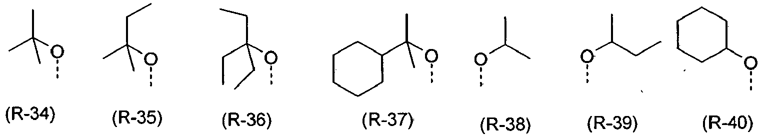 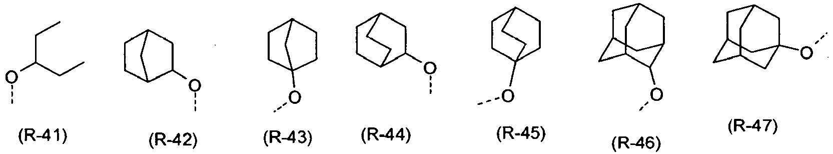 wobei die gestrichelte Bindung die Anknüpfung dieser Gruppen an den fluoreszierenden Grundkörper kennzeichnet.

Wenn der sterisch anspruchsvolle Substituent für eine Aralkylgruppe steht, dann ist diese Aralkylgruppe bevorzugt ausgewählt aus den Strukturen der folgenden Formeln (R-48) bis (R-61): 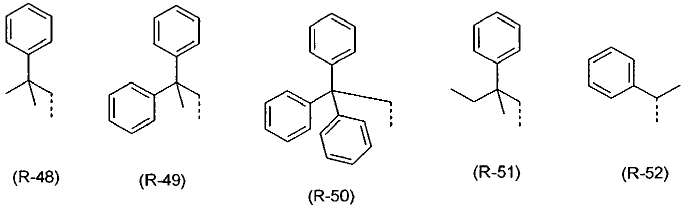 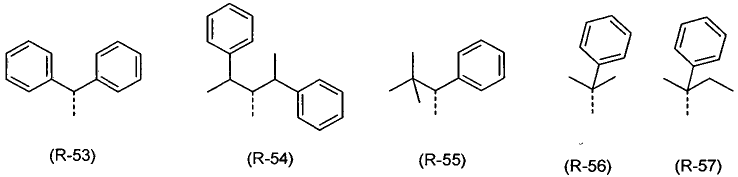 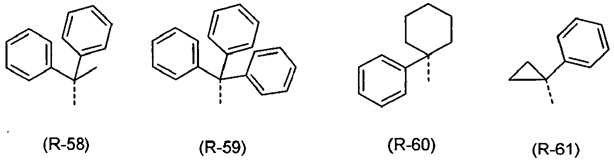 wobei die gestrichelte Bindung die Anknüpfung dieser Gruppen an den fluoreszierenden Grundkörper kennzeichnet und die Phenylgruppen jeweils durch einen oder mehrere Reste R^{a} substituiert sein können, wobei R^{a} wie unten für Ring (1) bis Ring (7) definiert ist.

Wenn der sterisch anspruchsvolle Substituent für ein aromatisches Ringsystem steht, dann weist dieses aromatische Ringsystem bevorzugt 6 bis 30 aromatische Ringatome auf, besonders bevorzugt 6 bis 24 aromatische Ringatome. Weiterhin enthält dieses aromatische Ringsystem bevorzugt nur Phenylgruppen. Dabei ist das aromatische Ringsystem bevorzugt ausgewählt aus den Strukturen der folgenden Formeln (R-62) bis (R-76): 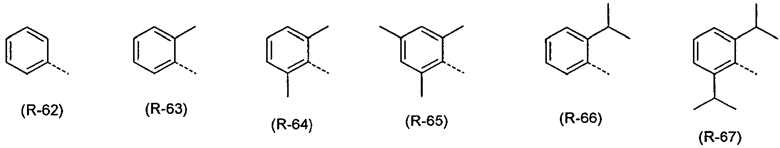 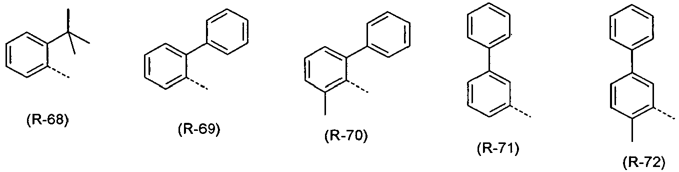 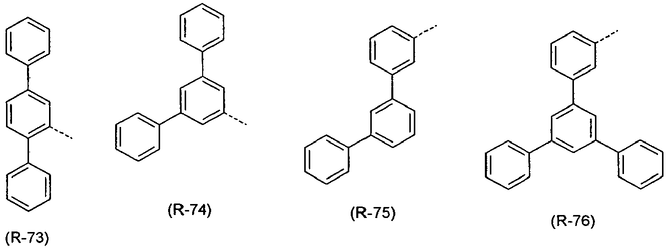 wobei die gestrichelte Bindung die Anknüpfung dieser Gruppen an den fluoreszierenden Grundkörper kennzeichnet und die Phenylgruppen jeweils durch einen oder mehrere Reste R^{a} substituiert sein können, wobei R^{a} wie unten für Ring (1) bis Ring (7) definiert ist. Geeignete sterisch anspruchsvolle Gruppen sind weiterhin ankondensierte aliphatische Gruppen, die bevorzugt in benzylischer Position keine aziden Protonen aufweisen. Bevorzugt sind die Strukturen der folgenden Formeln (Ring-1) bis (Ring-7), 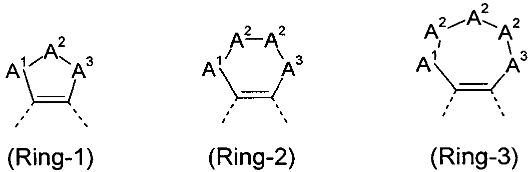 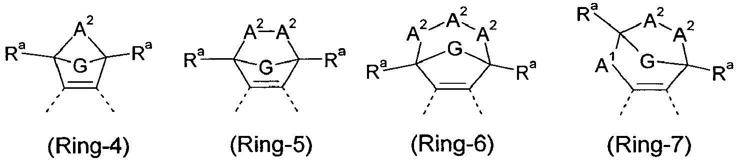 wobei die gestrichelten Bindungen die Verknüpfung der beiden Kohlenstoffatome im fluoreszierenden Grundkörper andeuten und weiterhin gilt:
- A¹, A², A³: ist gleich oder verschieden bei jedem Auftreten C(R^{a})₂, O oder S;
- G: ist eine Alkylengruppe mit 1, 2 oder 3 C-Atomen, welche mit einem oder mehreren Resten R^{b} substituiert sein kann oder eine orthoverknüpfte Arylengruppe mit 5 bis 14 aromatischen Ringatomen, welche durch einen oder mehrere Reste R^{b} substituiert sein kann;
- R^{a}: ist bei jedem Auftreten gleich oder verschieden ausgewählt aus der Gruppe bestehend aus H, D, F, einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R^{b} substituiert sein kann, einem aromatischen Ringsystem mit 5 bis 60 aromatischen Ringatomen, das jeweils mit einem oder mehreren Resten R^{b} substituiert sein kann, oder einer Aralkylgruppe mit 5 bis 60 aromatischen Ringatomen, die mit einem oder mehreren Resten R^{b} substituiert sein kann, wobei optional zwei oder mehr benachbarte Substituenten R^{a} ein Ringsystem bilden können, das mit einem oder mehreren Resten R^{b} substituiert sein kann;
- R^{b}: ist ausgewählt aus der Gruppe bestehend aus H, D, F, einem aliphatischem Kohlenwasserstoffrest mit 1 bis 20 C-Atomen, einem aromatischem Ringsystem mit 5 bis 30 aromatischen Ringatomen, wobei zwei oder mehr benachbarte Substituenten R^{b} miteinander ein Ringsystem bilden können;
mit der Maßgabe, dass in diesen Gruppen nicht zwei Heteroatome direkt aneinander gebunden sind.

In den Strukturen (Ring-1) bis (Ring-7) sowie den weiteren als bevorzugt genannten Ausführungsformen dieser Strukturen wird formal eine Doppelbindung zwischen den zwei Kohlenstoffatomen abgebildet. Dies stellt eine Vereinfachung der chemischen Struktur dar, da diese beiden Kohlenstoffatome in das aromatische oder heteroaromatische System des fluoreszierenden Kerns eingebunden sind und somit die Bindung zwischen diesen beiden Kohlenstoffatomen formal zwischen dem Bindungsgrad einer Einfachbindung und dem einer Doppelbindung liegt. Das Einzeichnen der formalen Doppelbindung ist somit nicht limitierend für die Struktur auszulegen, sondern es ist für den Fachmann offensichtlich, dass es sich hier um eine aromatische Bindung handelt.

Wenn es sich bei R^{a} bzw. R^{b} um eine Aralkylgruppe oder ein aromatisches Ringsystem handelt, so ist es bevorzugt, wenn diese keine kondensierten Arylgruppen mit mehr als 10 Kohlenstoffatomen aufweisen, in denen Arylgruppen über eine gemeinsame Kante direkt aneinander ankondensiert sind. Besonders bevorzugt weist dieses überhaupt keine kondensierten Arylgruppen auf, in denen Arylgruppen über eine gemeinsame Kante direkt aneinander ankondensiert sind.

Bevorzugt bei den Gruppen (Ring-1) bis (Ring-7) ist, wenn diese keine aziden benzylischen Protonen aufweisen. Unter benzylischen Protonen werden Protonen verstanden, die an ein Kohlenstoffatom binden, welches direkt an den Liganden gebunden ist. Die Abwesenheit von aziden benzylischen Protonen wird in (Ring-1) bis (Ring-3) und (Ring-7) dadurch erreicht, dass A¹ und A³, wenn diese für C(R^{a})₂ stehen, so definiert sind, dass R^{a} ungleich H oder D ist. Dies ist daher eine bevorzugte Ausführungsform. Die Abwesenheit von aziden benzylischen Protonen wird in (Ring-4) bis (Ring-7) dadurch erreicht, dass es sich dabei um eine bicyclische Struktur handelt. Aufgrund der starren räumlichen Anordnung ist R^{a}, wenn es für H steht, deutlich weniger azide als benzylische Protonen, da das korrespondierende Anion der bicyclischen Struktur nicht mesomeriestabilisiert ist. Auch wenn R^{a} in (Ring-4) bis (Ring-7) für H steht, handelt es sich dabei daher um ein nicht-azides Proton im Sinne der vorliegenden Anmeldung.

In einer bevorzugten Ausführungsform der Struktur (Ring-1) bis (Ring-7) steht maximal eine der Gruppen A¹, A² und A³ für ein Heteroatom, insbesondere für O, und die anderen Gruppen stehen für C(R^{a})₂ oder A¹ und A³ stehen gleich oder verschieden bei jedem Auftreten für O und A² steht für C(R^{a})₂. In einer besonders bevorzugten Ausführungsform der Erfindung stehen A¹, A² und A³ gleich oder verschieden bei jedem Auftreten für C(R^{a})₂.

Bevorzugte Ausführungsformen von (Ring-1) sind somit die Strukturen (Ring-1A), (Ring-1B), (Ring-1C) und (Ring-1D), 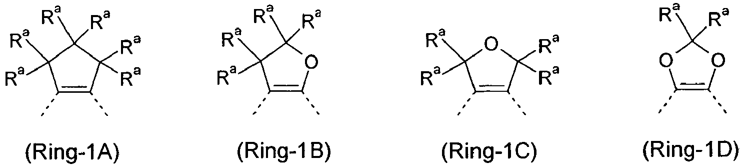 wobei R^{a} die oben genannten Bedeutungen aufweisen. Dabei steht R^{a} in den benzylischen Positionen bevorzugt nicht für H oder D.

Bevorzugte Ausführungsformen von (Ring-2) sind die Strukturen (Ring-2A) bis (Ring-2F), 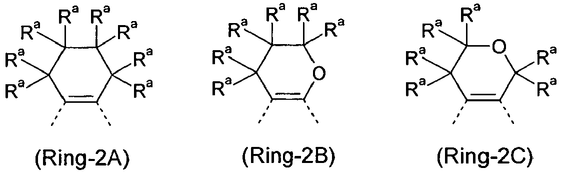 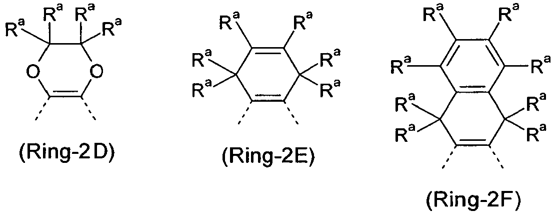 wobei R^{a} die oben genannten Bedeutungen aufweist. Dabei steht R^{a} in den benzylischen Positionen bevorzugt nicht für H oder D.

Bevorzugte Ausführungsformen von (Ring-3) sind die Strukturen (Ring-3A) bis (Ring-3E), 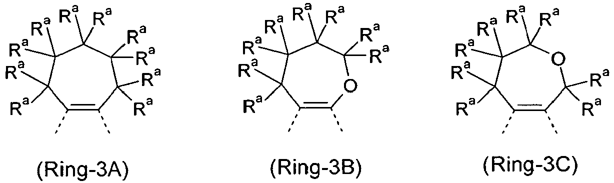 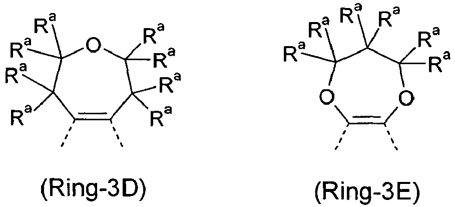 wobei R^{a} die oben genannten Bedeutungen aufweist. Dabei steht R^{a} in den benzylischen Positionen bevorzugt nicht für H oder D.

In einer bevorzugten Ausführungsform von (Ring-4) stehen die Reste R^{a}, die an den Brückenkopf gebunden sind, für H, D, F oder CH₃. Weiterhin bevorzugt steht A² für C(R^{a})₂ oder O, und besonders bevorzugt für C(R³)₂. Bevorzugte Ausführungsformen von (Ring-4) sind somit eine Strukturen (Ring-4A) und (Ring-4B), 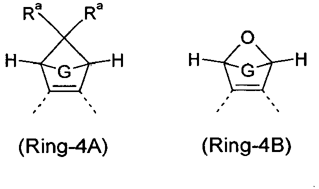 wobei die verwendeten Symbole die oben genannten Bedeutungen aufweisen.

In einer bevorzugten Ausführungsform von (Ring-5), (Ring-6) und (Ring-7) stehen die Reste R^{a}, die an den Brückenkopf gebunden sind, für H, D, F oder CH₃. Weiterhin bevorzugt steht A² für C(R^{a})₂. Bevorzugte Ausführungsformen von (Ring-5), (Ring-6) und (Ring-7) sind somit die Strukturen (Ring-5A), (Ring-6A) und (Ring-7A), 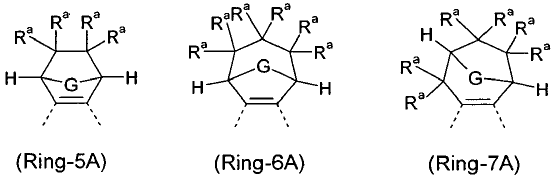 wobei die verwendeten Symbole die oben genannten Bedeutungen aufweisen. Dabei steht R^{a} in den benzylischen Positionen in (Ring-7A) bevorzugt nicht für H oder D.

Weiterhin bevorzugt steht die Gruppe G in den oben genannten Formeln für eine 1,2-Ethylengruppe, welche mit einem oder mehreren Resten R^{b} substituiert sein kann, wobei R^{b} bevorzugt gleich oder verschieden bei jedem Auftreten für H oder eine Alkylgruppe mit 1 bis 4 C-Atomen steht, oder eine ortho-Arylengruppe mit 6 bis 10 C-Atomen, welche mit einem oder mehreren Resten R^{b} substituiert sein kann, bevorzugt aber unsubstituiert ist, insbesondere eine ortho-Phenylengruppe, welche mit einem oder mehreren Resten R^{b} substituiert sein kann, bevorzugt aber unsubstituiert ist.

In einer weiteren bevorzugten Ausführungsform der Erfindung steht der Substituent R^{a}, insbesondere wenn er in einer benzylischen Position gebunden ist, in (Ring-1) bis (Ring-7) und in den bevorzugten Ausführungsformen gleich oder verschieden bei jedem Auftreten für F, eine geradkettige Alkylgruppe mit 1 bis 8 C-Atomen oder eine verzweigte oder cyclische Alkylgruppe mit 3 bis 10 C-Atomen, wobei jeweils ein oder mehrere H-Atome durch D oder F ersetzt sein können, oder ein aromatisches Ringsystem mit 6 bis 14 aromatischen Ringatomen, das jeweils durch einen oder mehrere Reste R^{b} substituiert sein kann; dabei können zwei Reste R^{a}, welche an dasselbe Kohlenstoffatom gebunden sind, miteinander ein Ringsystem bilden und so ein Spirosystem aufspannen; weiterhin kann R^{a} mit einem weiteren Rest R^{a} ein aliphatisches Ringsystem bilden. Dadurch kann beispielsweise ein Homoadamantan-Rest gebildet werden.

In einer besonders bevorzugten Ausführungsform der Erfindung steht R^{a}, insbesondere wenn er in einer benzylischen Position gebunden ist, in (Ring-1) bis (Ring-7) und in den bevorzugten Ausführungsformen gleich oder verschieden bei jedem Auftreten für F, eine geradkettige Alkylgruppe mit 1 bis 3 C-Atomen, insbesondere Methyl, oder ein aromatisches Ringsystem mit 6 bis 12 aromatischen Ringatomen, insbesondere Phenyl, das jeweils durch einen oder mehrere Reste R^{b} substituiert sein kann, bevorzugt aber unsubstituiert ist; dabei können zwei Reste R^{a}, welche an dasselbe Kohlenstoffatom gebunden sind, miteinander ein Ringsystem bilden und so ein Spirosystem aufspannen; weiterhin kann R^{a} mit einem weiteren Rest R^{a} ein aliphatisches Ringsystem bilden. Dadurch kann beispielsweise ein Homoadamantan-Rest gebildet werden.

Beispiele für besonders geeignete Gruppen (Ring-1) sind die im Folgenden aufgeführten Gruppen (Ring-1-1) bis (Ring-1-49), wobei (Ring-1-1), (Ring-1-2) und (Ring-1-27) besonders bevorzugt sind:

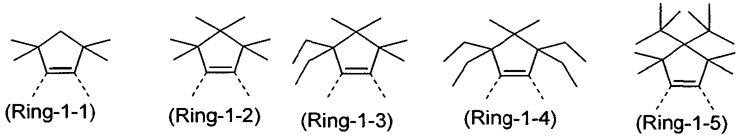

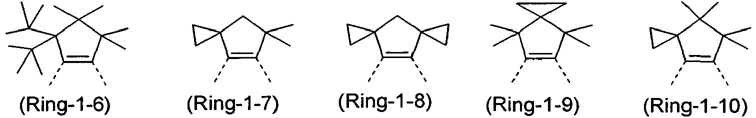

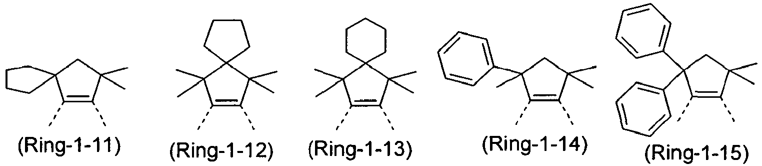

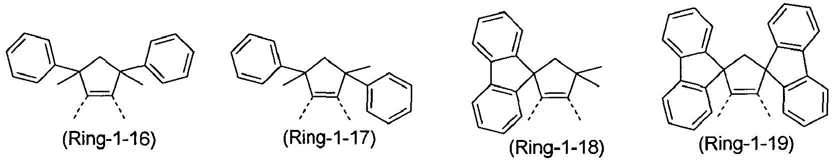

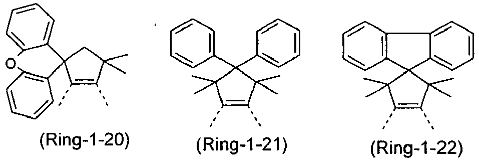

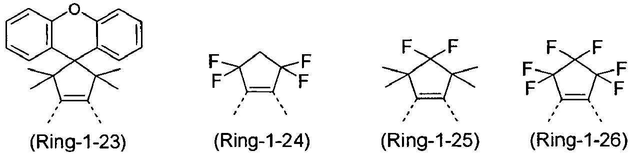

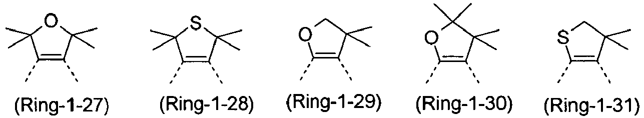

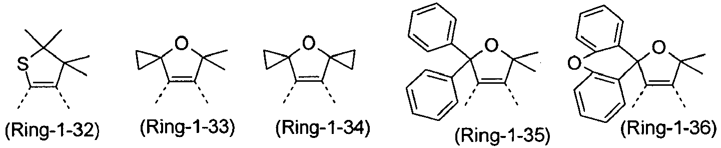

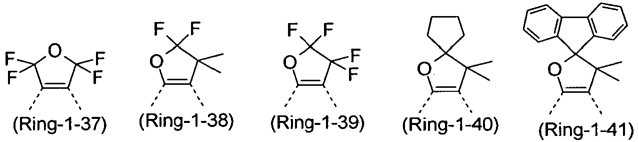

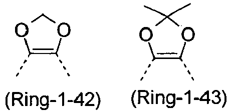

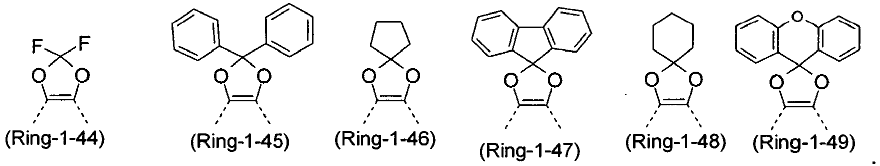

Beispiele für besonders geeignete Gruppen (Ring-2) sind die im Folgenden aufgeführten Gruppen (Ring-2-1) bis (Ring-2-12), wobei (Ring-2-1) besonders bevorzugt ist:

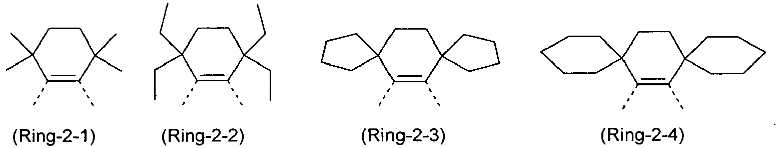

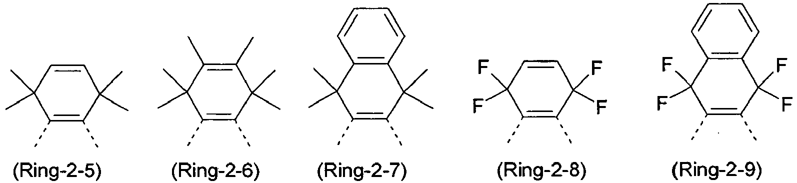

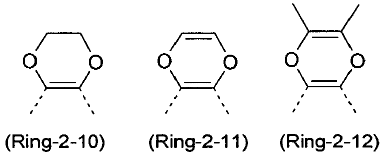

Beispiele für besonders geeignete Gruppen (Ring-3), (Ring-6) und (Ring-7) sind die im Folgenden aufgeführten Gruppen (Ring-3-1), (Ring-6-1), (Ring-7-1) und (Ring-7-2), wobei (Ring-7-1) und (Ring-7-2) besonders bevorzugt sind:

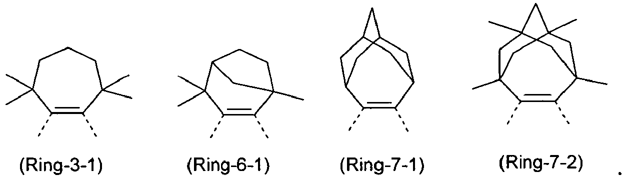

Beispiele für besonders geeignete Gruppen (Ring-4) sind die im Folgenden aufgeführten Gruppen (Ring-4-1) bis (Ring-4-22), wobei (Ring-4-4), (Ring-4-5) und (Ring-4-7) besonders bevorzugt sind:

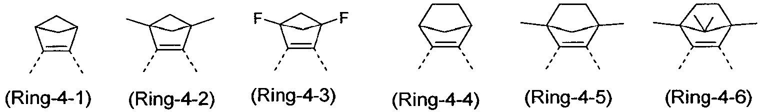

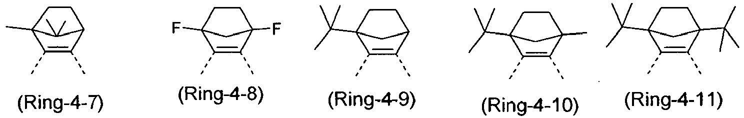

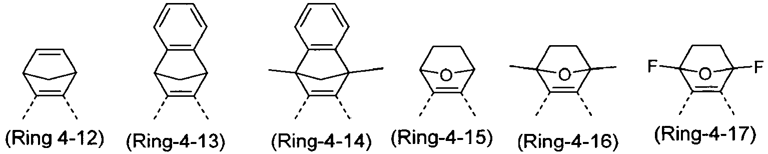

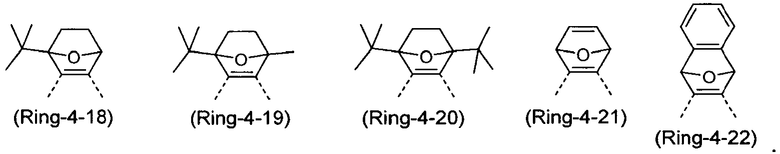

Beispiele für besonders geeignete Gruppen (Ring-5) sind die im Folgenden aufgeführten Gruppen (Ring-5-1) bis (Ring-5-5), wobei (Ring-5-1) und (Ring-5-3) besonders bevorzugt sind:

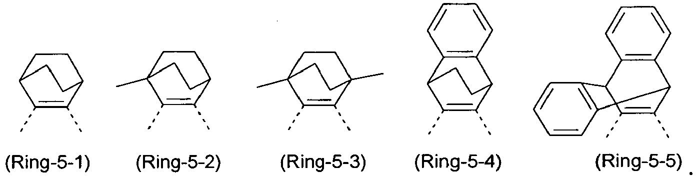

In den folgenden Schemata 1 bis 8 ist die Synthese derartiger Strukturen dargestellt. Dabei steht R jeweils für einen sterisch anspruchsvollen Substituenten, wie er oben beschrieben ist, und * kennzeichnet die Positionen, an denen ein aliphatischer Cyclus, wie oben beschrieben, ankondensiert ist. Alternativ ist in Schema 1 und Schema 5 durch eine gebogene Linie der ankondensierte aliphatische Cyclus angedeutet. Dabei kann die Synthese bevorzugt über eine Povarov-Cyclisierungsreaktion erfolgen, wie in Schema 1 gezeigt. Diese Reaktionstypen sind dem Fachmann im Allgemeinen bekannt.

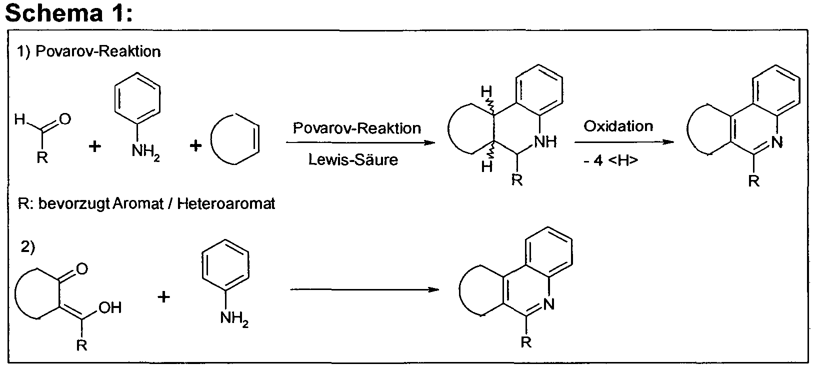

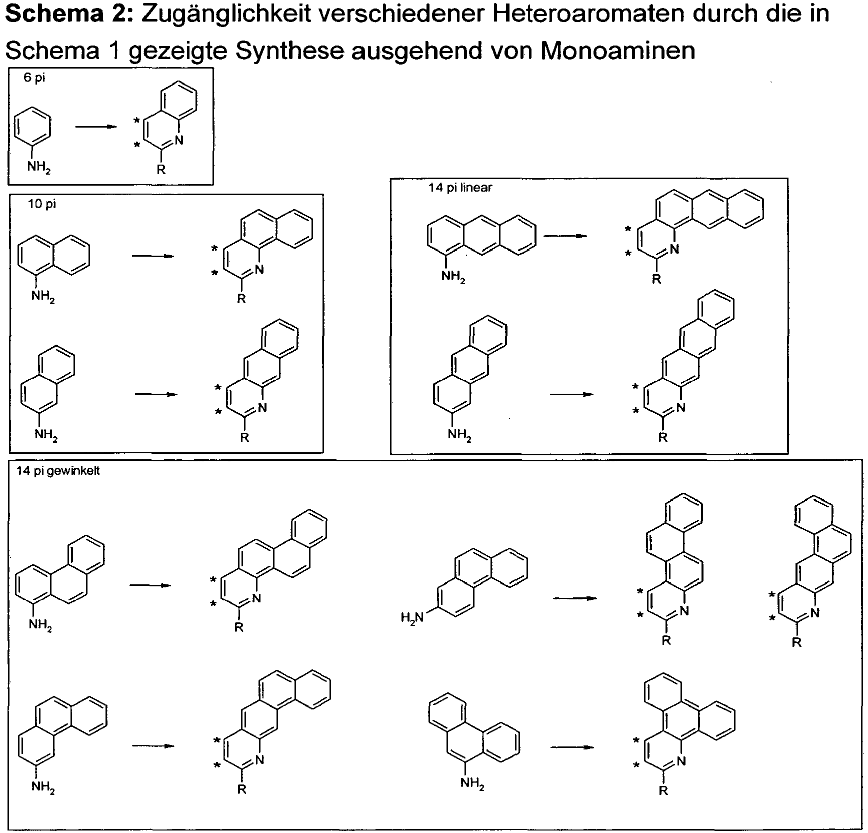

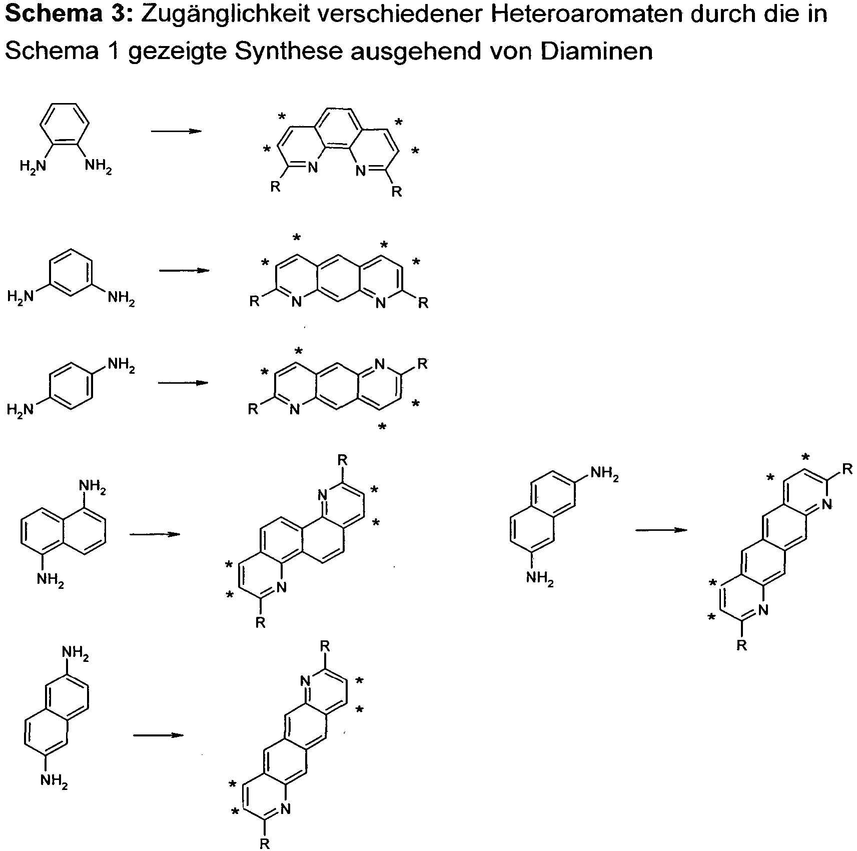

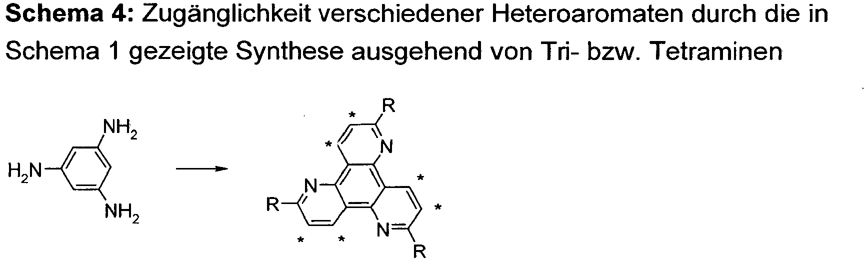

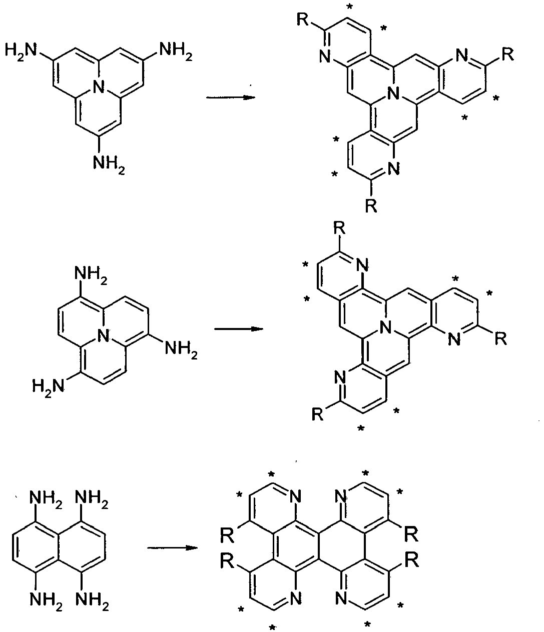

Alternativ können andere Substitutionsmuster durch die in Schema 5 gezeigte Reaktion synthetisiert werden.

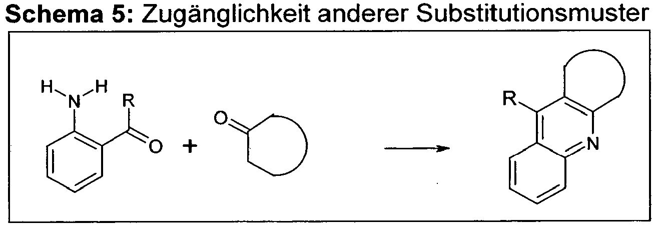

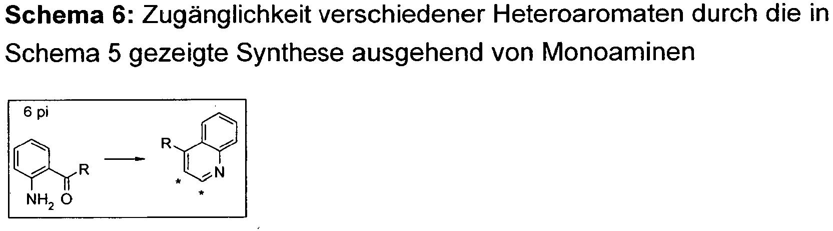

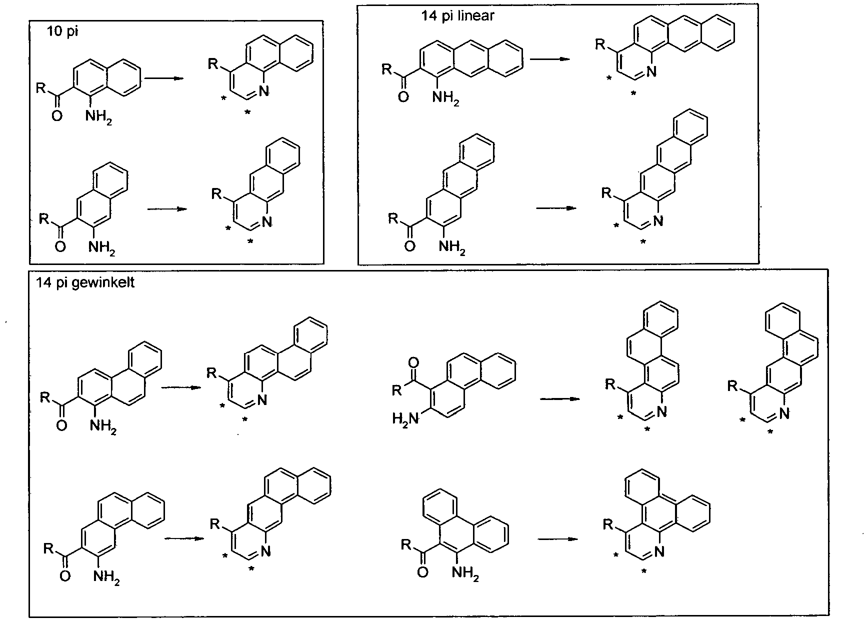

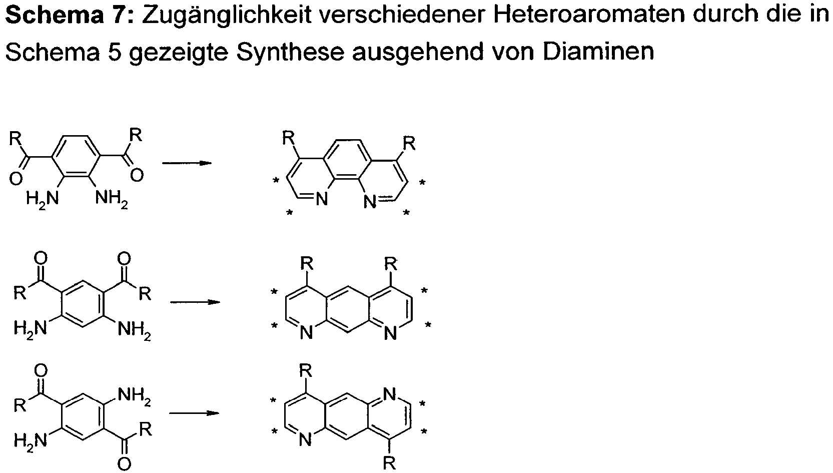

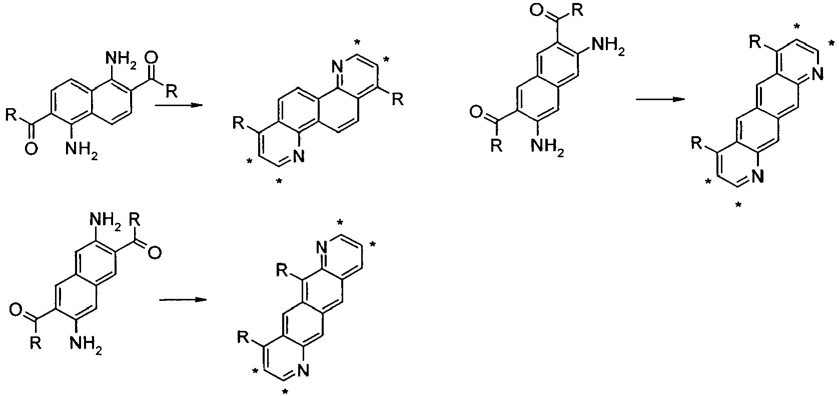

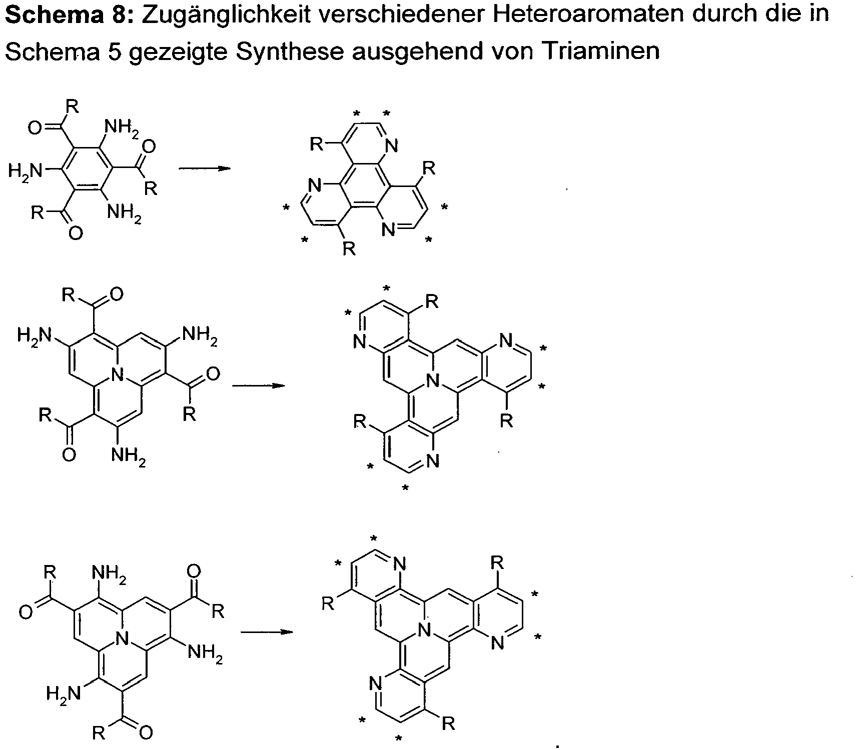

Geeignete aromatische fluroeszierende Grundkörper mit ankondensierten aliphatischen Cyclen sind die im Folgenden abgebildeten Strukturen, wobei * jeweils die Positionen andeutet, über die die aliphatischen Cyclen ankondensiert sind:

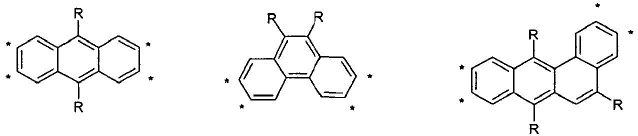

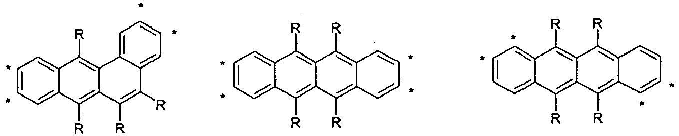

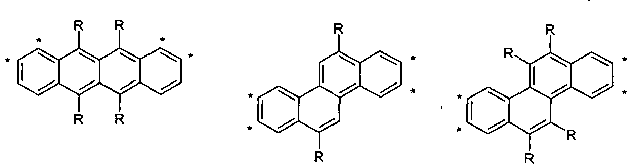

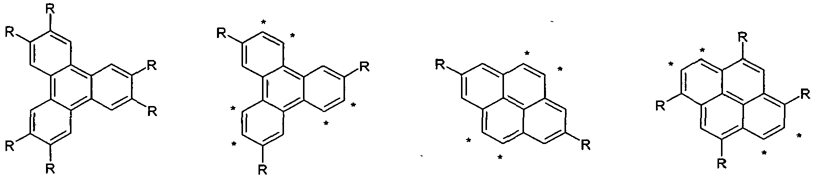

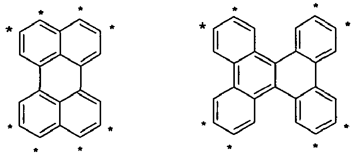

Dabei steht R jeweils bevorzugt für einen sterisch anspruchsvollen Substituenten, wie er vorne definiert ist.

Dabei ist es bevorzugt, wenn in den sterisch abgeschirmten fluoreszierenden Verbindungen nicht mehr als zwei unsubstituierte Kohlenstoff- bzw. Stickstoffatome zwischen zwei Substituenten liegen, besonders bevorzugt nicht mehr als ein unsubstituiertes Kohlenstoff- bzw. Stickstoffatom.

Im Folgenden sind Beispiele für sterisch abgeschirmte fluoreszierende Verbindungen mit hoher sterischer Abschirmung gezeigt. Der angegebene Abschirmparameter S wurde unter der Voraussetzung bestimmt, dass die fluoreszente Verbindung in Kombination mit der TADF-Verbindung D1 (siehe Beispielteil) eingesetzt wird. Für die Kombination mit anderen TADF-Verbindungen können sich andere Abschirmparameter ergeben.

| | | |
|---|---|---|
| 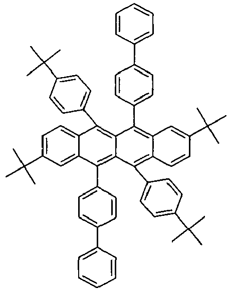 | 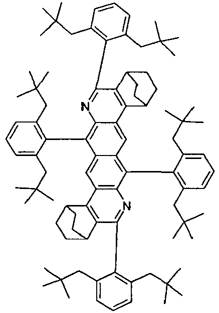 | 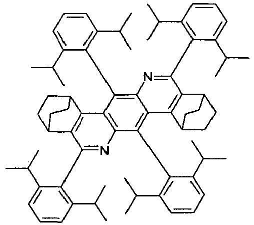 |
| SE1, S = 0.518 | S = 0.404 | S = 0.489 |
| 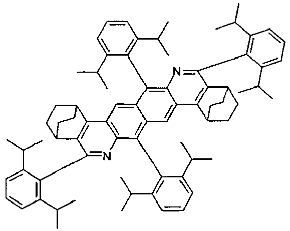 | 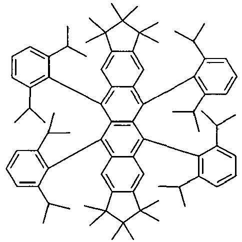 | 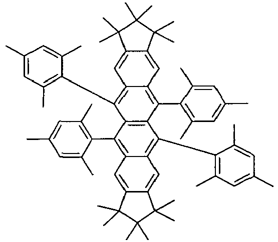 |
| S = 0.510 | S = 0.521 | S = 0.566 |
| 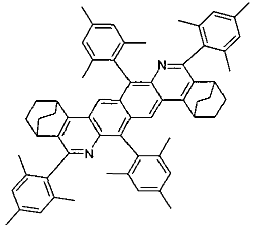 | | |
| S = 0.587 | | |

Im Folgenden wird die Matrixverbindung genauer beschrieben, falls die emittierende Schicht eine Matrixverbindung enthält.

In einer bevorzugten Ausführungsform der Erfindung trägt die Matrixverbindung nicht oder nicht wesentlich zur Emission der Mischung bei.

Es ist bevorzugt, dass die niedrigste Triplettenergie der Matrixverbindung maximal 0.1 eV niedriger ist als die Triplettenergie der TADF-Verbindung. Insbesondere bevozugt ist T₁(Matrix) ≥ T₁(TADF). Besonders bevorzugt gilt: T₁(Matrix) - T₁(TADF) ≥ 0.1 eV; ganz besonders bevorzugt: T₁(Matrix) - T₁(TADF) ≥ 0.2 eV. Dabei steht T₁(Matrix) für die niedrigste Triplettenergie der Matrixverbindung und T₁(TADF) für die niedrigste Triplettenergie der TADF-Verbindung. Dabei wird die Triplettenergie der Matrixverbindung T₁(Matrix) durch quantenchemische Rechnung bestimmt, wie hinten im Beispielteil allgemein beschrieben.

Beispiele für geeignete Matrixverbindungen, die in der erfindungsgemäßen emittierenden Schicht verwendet werden können, sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Dibenzofuranderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Pyrimidinderivate, Chinoxalinderivate, Zn-, AI- oder Be-Komplexe, z. B. gemäß EP 652273 oder WO 2009/062578, oder verbrückte Carbazolderivate, z. B. gemäß US 2009/0136779, WO 2010/050778, WO 2011/042107 oder WO 2011/088877. Es können auch Mischungen aus zwei oder mehr dieser Matrixmaterialien verwendet werden.

Bevorzugt weist die Matrixverbindung eine Glasübergangstemperatur T_{G} von größer als 70 °C auf, besonders bevorzugt größer als 90 °C, ganz besonders bevorzugt größer als 110 °C.

Die Matrixverbindungen sind bevorzugt ladungstransportierende, also elektronentransportierende oder lochtransportierende oder bipolare Verbindungen. Als Matrixverbindungen können weiterhin auch Verbindungen dienen, die weder loch- noch elektronentransportierend im Sinne der vorliegenden Anmeldung sind.

Eine elektronentransportierende Verbindung im Sinne der vorliegenden Erfindung ist eine Verbindung, die ein LUMO ≤ -2.50 eV aufweist. Bevorzugt ist das LUMO ≤ -2.60 eV, besonders bevorzugt ≤ -2.65 eV, ganz besonders bevorzugt ≤ -2.70 eV. Dabei ist das LUMO das niedrigste unbesetzte Molekülorbital (lowest unoccupied molecular orbital). Der Wert des LUMO der Verbindung wird durch quantenchemische Rechnung bestimmt, wie hinten im Beispielteil allgemein beschrieben.

Eine lochtransportierende Verbindung im Sinne der vorliegenden Erfindung ist eine Verbindung, die ein HOMO ≥ -5.5 eV aufweist. Bevorzugt ist das HOMO ≥ -5.4 eV, besonders bevorzugt ≥ -5.3 eV. Dabei ist das HOMO das höchste besetzte Molekülorbital (highest occupied molecular orbital). Der Wert des HOMO der Verbindung wird durch quantenchemische Rechnung bestimmt, wie hinten im Beispielteil allgemein beschrieben.

Eine bipolare Verbindung im Sinne der vorliegenden Erfindung ist eine Verbindung, die sowohl loch- wie auch elektronentransportierend ist.

Im Folgenden werden Verbindungsklassen beschrieben, die sich bevorzugt als elektronenleitende Matrixverbindung in der erfindungsgemäßen organischen Elektrolumineszenzvorrichtung eignen.

Geeignete elektronenleitende Matrixverbindungen sind ausgewählt aus den Stoffklassen der Triazine, der Pyrimidine, der Lactame, der Metallkomplexe, insbesondere der Be-, Zn- bzw. Al-Komplexe, der aromatischen Ketone, der aromatischen Phosphinoxide, der Azaphosphole, der Azaborole, welche mit mindestens einem elektronenleitenden Substituenten substituiert sind, und der Chinoxaline.

In einer bevorzugten Ausführungsform der Erfindung handelt es sich bei der elektronenleitenden Verbindung um eine rein organische Verbindung, d. h. um eine Verbindung, die keine Metalle enthält.

Wenn die elektronenleitende Matrixverbindung eine Triazin- oder Pyrimidinverbindung ist, dann ist diese Verbindung bevorzugt ausgewählt aus den Verbindungen der folgenden Formeln (M1) bzw. (M2), 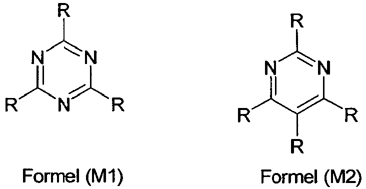 wobei für die verwendeten Symbole gilt:
- R: ist bei jedem Auftreten gleich oder verschieden ausgewählt aus der Gruppe bestehend aus H, D, F, Cl, Br, I, CN, NO₂, N(Ar)₂, N(R¹)₂, C(=O)Ar, C(=O)R¹, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 80, bevorzugt 5 bis 60, aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, oder einer Aralkyl- oder Heteroaralkylgruppe mit 5 bis 60 aromatischen Ringatomen, die mit einem oder mehreren Resten R¹ substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
- R¹: ist bei jedem Auftreten gleich oder verschieden ausgewählt aus der Gruppe bestehend aus H, D, F, CI, Br, I, CN, NO₂, N(Ar)₂, N(R²)₂, C(=O)Ar, C(=O)R², P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=S, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder eine Aralkyl- oder Heteroaralkylgruppe mit 5 bis 60 aromatischen Ringatomen, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann;
- Ar: ist bei jedem Auftreten gleich oder verschieden ein aromatisches oder heteroaromatisches Ringsystem mit 5-60 aromatischen Ringatomen, das mit einem oder mehreren nicht-aromatischen Resten R² substituiert sein kann; dabei können zwei Reste Ar, welche an dasselbe N-Atom oder P-Atom binden, auch durch eine Einfachbindung oder eine Brücke, ausgewählt aus N(R²), C(R²)₂, O oder S, miteinander verbrückt sein;
- R²: ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches, aromatisches oder heteroaromatisches Ringsystem bilden können.

Benachbarte Substituenten im Sinne der vorliegenden Anmeldung sind Substituenten, die entweder an dasselbe Kohlenstoffatom gebunden sind oder die an Kohlenstoffatome gebunden sind, die wiederum direkt aneinander gebunden sind.

Die im Folgenden aufgeführten Definitionen beziehen sich nicht nur auf die Matrixverbindung, sondern auf die gesamte vorliegende Erfindung, also auch auf die sterisch abgeschirmte fluoreszierende Verbindung.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 30 bzw. 5 - 60 aromatischen Ringatomen, welches noch jeweils mit den oben genannten Resten R, R¹ oder R² substituiert sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer bevorzugten Ausführungsform der Verbindungen gemäß Formel (M1) bzw. Formel (M2) steht mindestens einer der Substituenten R für ein aromatisches oder heteroaromatisches Ringsystem. In Formel (M1) stehen besonders bevorzugt alle drei Substituenten R für ein aromatisches oder heteroaromatisches Ringsystem, das jeweils durch einen oder mehrere Reste R¹ substituiert sein kann. In Formel (M2) stehen besonders bevorzugt ein, zwei oder drei Substituenten R für ein aromatisches oder heteroaromatisches Ringsystem, das jeweils durch einen oder mehrere Reste R¹ substituiert sein kann, und die anderen Substituenten R stehen für H. Bevorzugte Ausführungsformen sind somit die Verbindungen der folgenden Formeln (M1a) bzw. (M2a) bis (M2d), 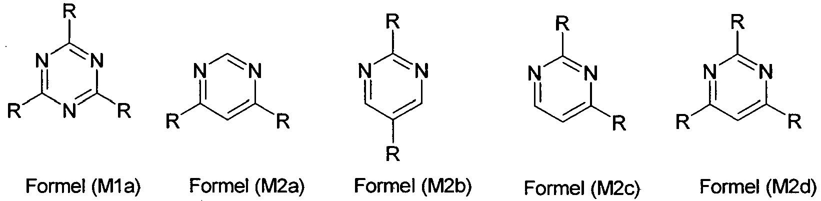 wobei R gleich oder verschieden für ein aromatisches oder heteroaromatisches Ringsystem mit 5 bis 60 aromatischen Ringatomen steht, das jeweils mit einem oder mehreren Resten R¹ substituiert sein kann, und R¹ die oben genannte Bedeutung aufweist.

Dabei sind bei den Pyrimidinverbindungen die Verbindungen der Formeln (M2a) und (M2d) bevorzugt, insbesondere Verbindungen der Formel (M2d).

Bevorzugte aromatische oder heteroaromatische Ringsysteme enthalten 5 bis 30 aromatische Ringatome, insbesondere 6 bis 24 aromatische Ringatome, und können durch einen oder mehrere Reste R¹ substituiert sein. Dabei enthalten die aromatischen bzw. heteroaromatischen Ringsysteme bevorzugt keine kondensierten Aryl- bzw. Heteroarylgruppen, in denen mehr als zwei aromatische Sechsringe direkt aneinander ankondensiert sind. Besonders bevorzugt enthalten sie überhaupt keine Aryl- bzw. Heteroarylgruppen, in denen aromatische Sechsringe direkt aneinander ankondensiert sind. Diese Bevorzugung ist mit der höheren Triplettenergie derartiger Substituenten zu begründen. So ist es besonders bevorzugt, wenn R beispielsweise keine Naphthylgruppen oder höhere kondensierte Arylgruppen aufweist und ebenso keine Chinolingruppen, Acridingruppen, etc.. Dagegen ist es möglich, dass R beispielsweise Carbazolgruppen, Dibenzofurangruppen, Fluorengruppen, etc. aufweist, weil in diesen Strukturen keine 6-Ring-Aromaten bzw. -Heteroaromaten direkt aneinander ankondensiert sind.

Bevorzugte Substituenten R sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Kombinationen aus zwei oder drei dieser Gruppen, welche jeweils mit einem oder mehreren Resten R¹ substituiert sein können. Dabei können in den oben genannten aromatischen Ringsystemen auch ein oder mehrere C-Atome durch N ersetzt sein.

Bevorzugte Biphenyl-, Terphenyl- und Quaterphenylgruppen sind die Gruppen der folgenden Formeln (Bi-1) bis (Bi-3), (Ter-1) bis (Ter-3) und (Quater-1) bis (Quater-4), 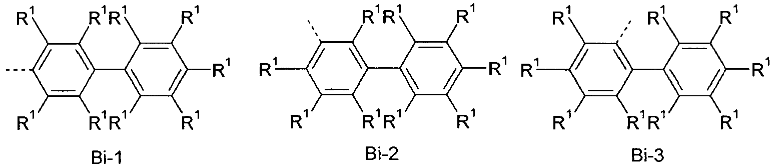 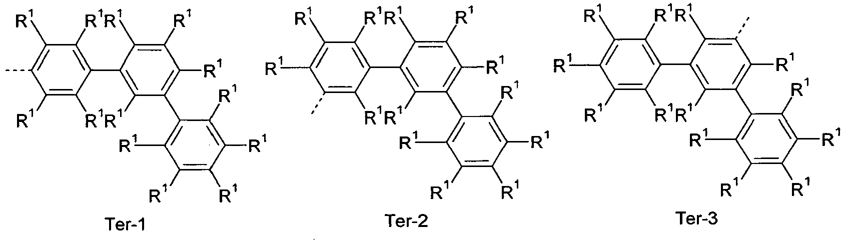 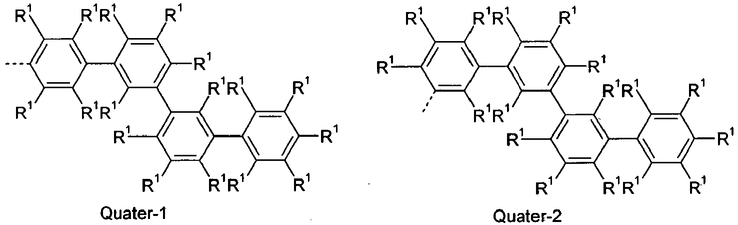 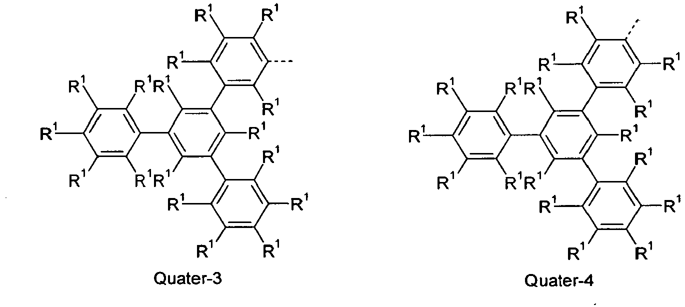 wobei R¹ die oben genannten Bedeutungen aufweist und die gestrichelte Bindung die Bindung an das Triazin bzw. Pyrimidin andeutet.

Insbesondere bevorzugt ist es, wenn mindestens eine Gruppe R ausgewählt ist aus den Strukturen der folgenden Formeln (M3) bis (M44), 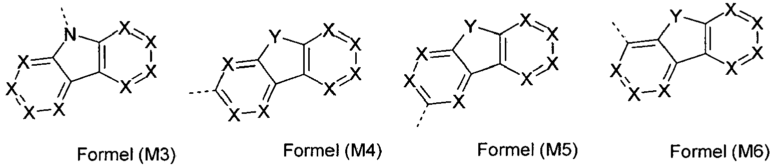 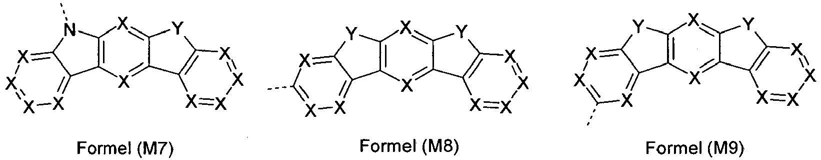 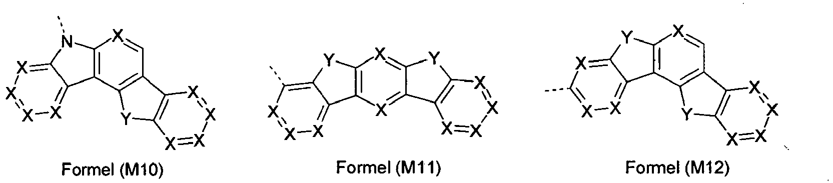 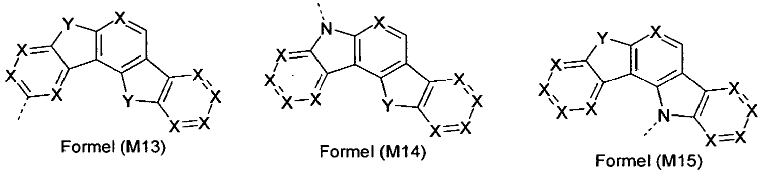 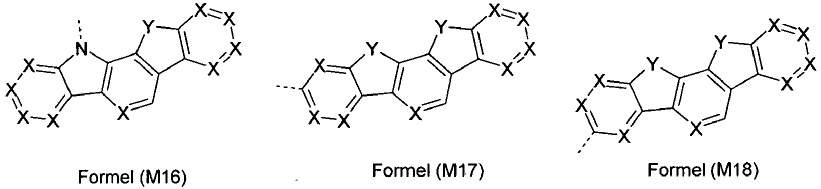 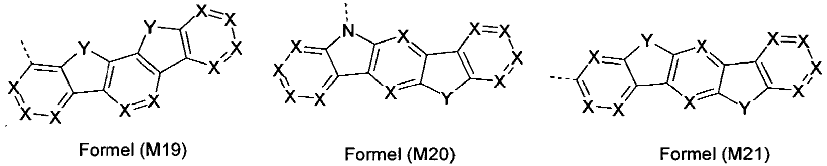 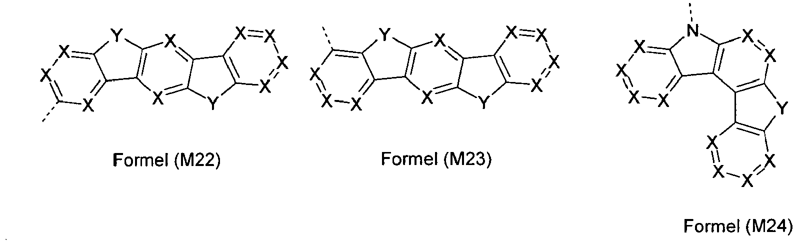 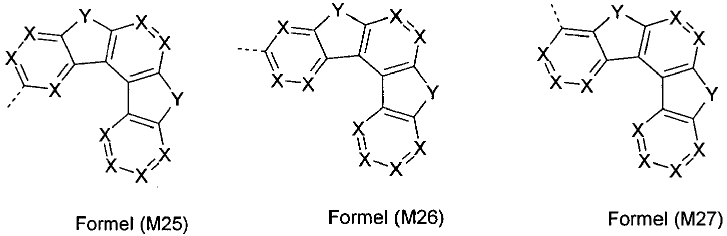 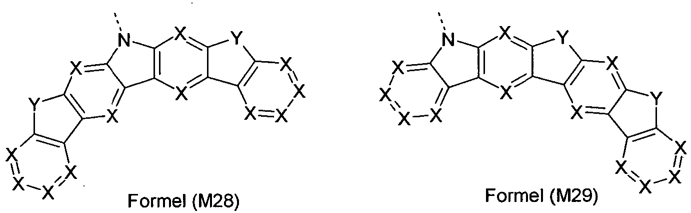 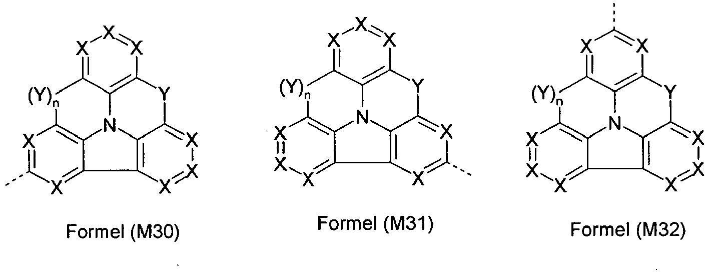 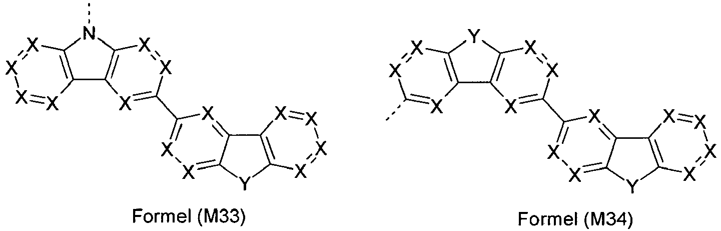 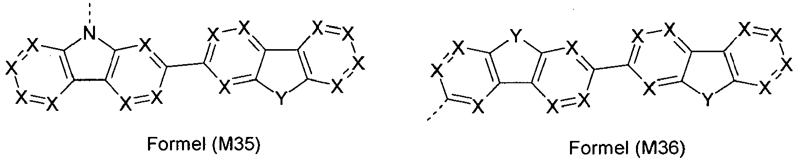 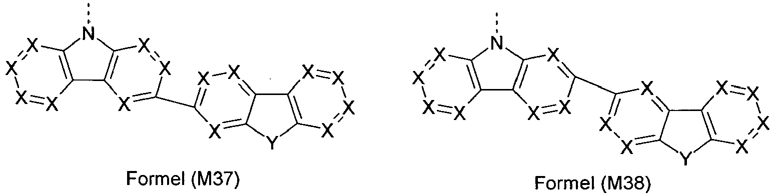 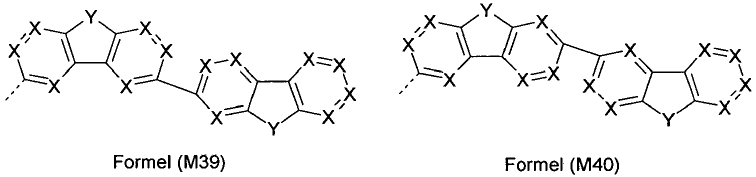 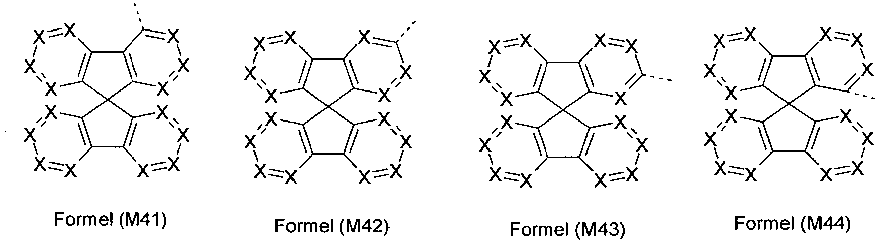 wobei R¹ und R² die oben genannten Bedeutungen aufweisen, die gestrichelte Bindung die Bindung an die Gruppe der Formel (M1) bzw. (M2) darstellt und weiterhin gilt:
- X: ist bei jedem Auftreten gleich oder verschieden CR¹ oder N, wobei bevorzugt maximal 2 Symbole X pro Cyclus für N stehen;
- Y: ist bei jedem Auftreten gleich oder verschieden C(R¹)₂, NR¹, O oder S;
- n: ist 0 oder 1, wobei n gleich 0 bedeutet, dass an dieser Position keine Gruppe Y gebunden ist und an den entsprechenden Kohlenstoffatomen statt dessen Reste R¹ gebunden sind.

Der oben genannte und auch im Folgenden verwendete Begriff "pro Cyclus" bezieht sich im Sinne der vorliegenden Anmeldung auf jeden einzelnen in der Verbindung enthaltenen Ring, also auf jeden einzelnen 5-bzw. 6-Ring.

In bevorzugten Gruppen der oben genannten Formeln (M3) bis (M44) steht maximal ein Symbol X pro Cyclus für N. Besonders bevorzugt steht das Symbol X gleich oder verschieden bei jedem Auftreten für CR¹, insbesondere für CH.

Wenn die Gruppen der Formeln (M3) bis (M44) mehrere Gruppen Y aufweisen, so kommen hierfür alle Kombinationen aus der Definition von Y in Frage. Bevorzugt sind Gruppen der Formeln (M3) bis (M44), in denen eine Gruppe Y für NR¹ und die andere Gruppe Y für C(R¹)₂ steht oder in denen beide Gruppen Y für NR¹ stehen oder in denen beide Gruppen Y für O stehen.

In einer weiteren bevorzugten Ausführungsform der Erfindung steht mindestens eine Gruppe Y in den Formeln (M3) bis (M44) gleich oder verschieden bei jedem Auftreten für C(R¹)₂ oder für NR¹.

Wenn Y für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in welchen zwei oder mehr aromatische bzw. heteroaromatische 6-Ring-Gruppen direkt aneinander ankondensiert sind, aufweist und welches jeweils auch durch einen oder mehrere Reste R² substituiert sein kann. Besonders bevorzugt sind die vorne aufgeführten Strukturen Bi-1 bis Bi-3, Ter-1 bis Ter-3 und Quater-1 bis Quater-4.

Wenn Y für C(R¹)₂ steht, steht R¹ bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Weiterhin kann es bevorzugt sein, wenn die Gruppe der oben genannten Formeln (M3) bis (M44) nicht direkt an das Triazin in Formel (M1) bzw. das Pyrimidin in Formel (M2) binden, sondern über eine verbrückende Gruppe. Diese verbrückende Gruppe ist dann bevorzugt ausgewählt aus einem aromatischen oder heteroaromatischen Ringsystem mit 5 bis 24 aromatischen Ringatomen, insbesondere mit 6 bis 12 aromatischen Ringatomen, welches jeweils mit einem oder mehreren Resten R¹ substituiert sein kann. Bevorzugte aromatische Ringsysteme sind ortho-, meta- oder para-Phenylen oder eine Biphenylgruppe, die jeweils durch einen oder mehrere Reste R¹ substituiert sein können, bevorzugt aber unsubstituiert sind.

Beispiele für bevorzugte Verbindungen gemäß Formel (M1) bzw. (M2) sind die folgenden Verbindungen.

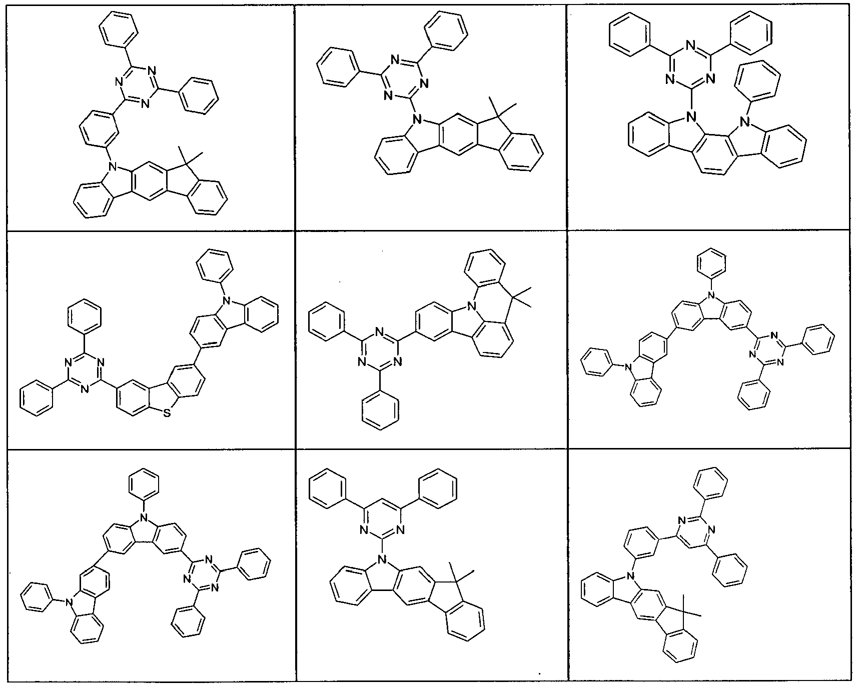

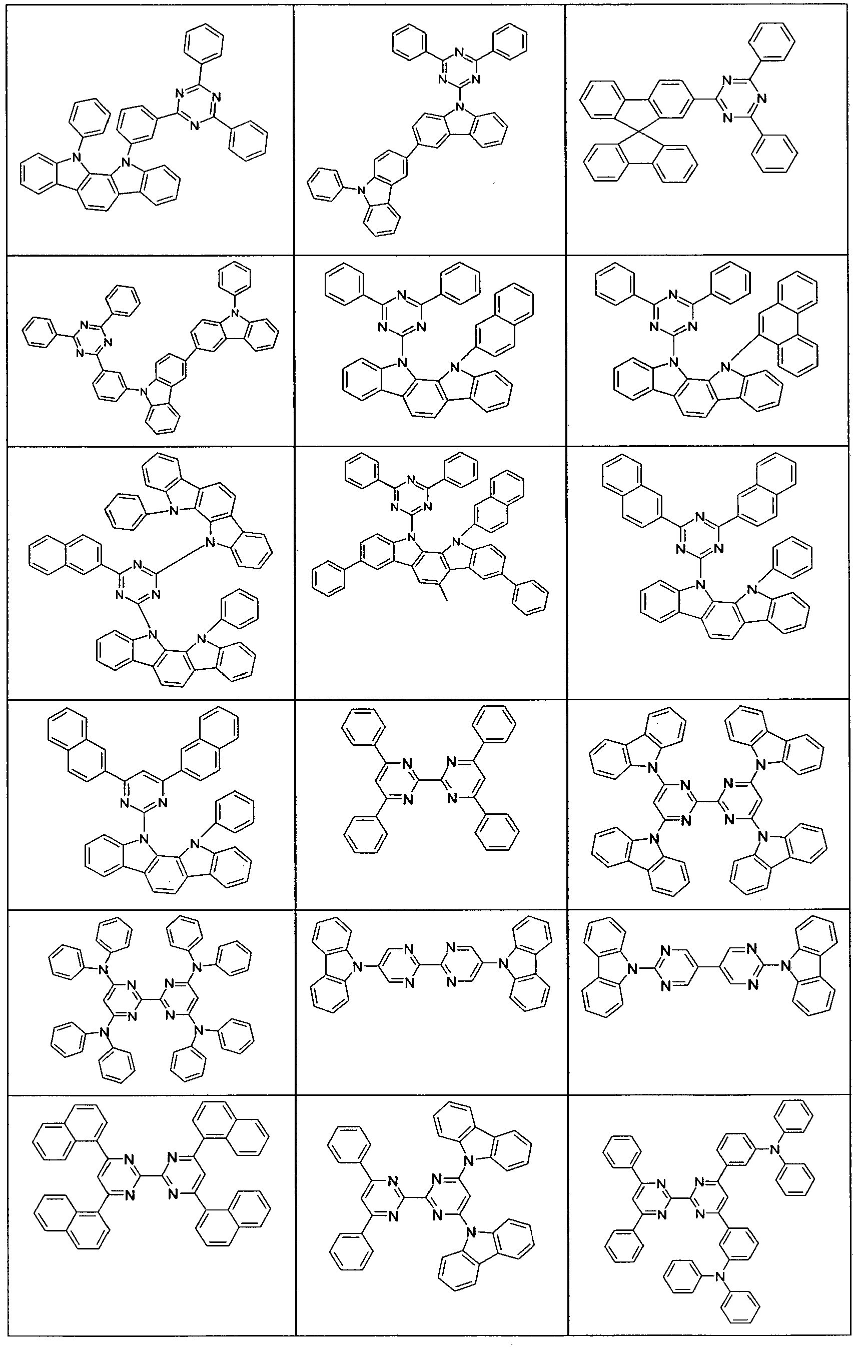

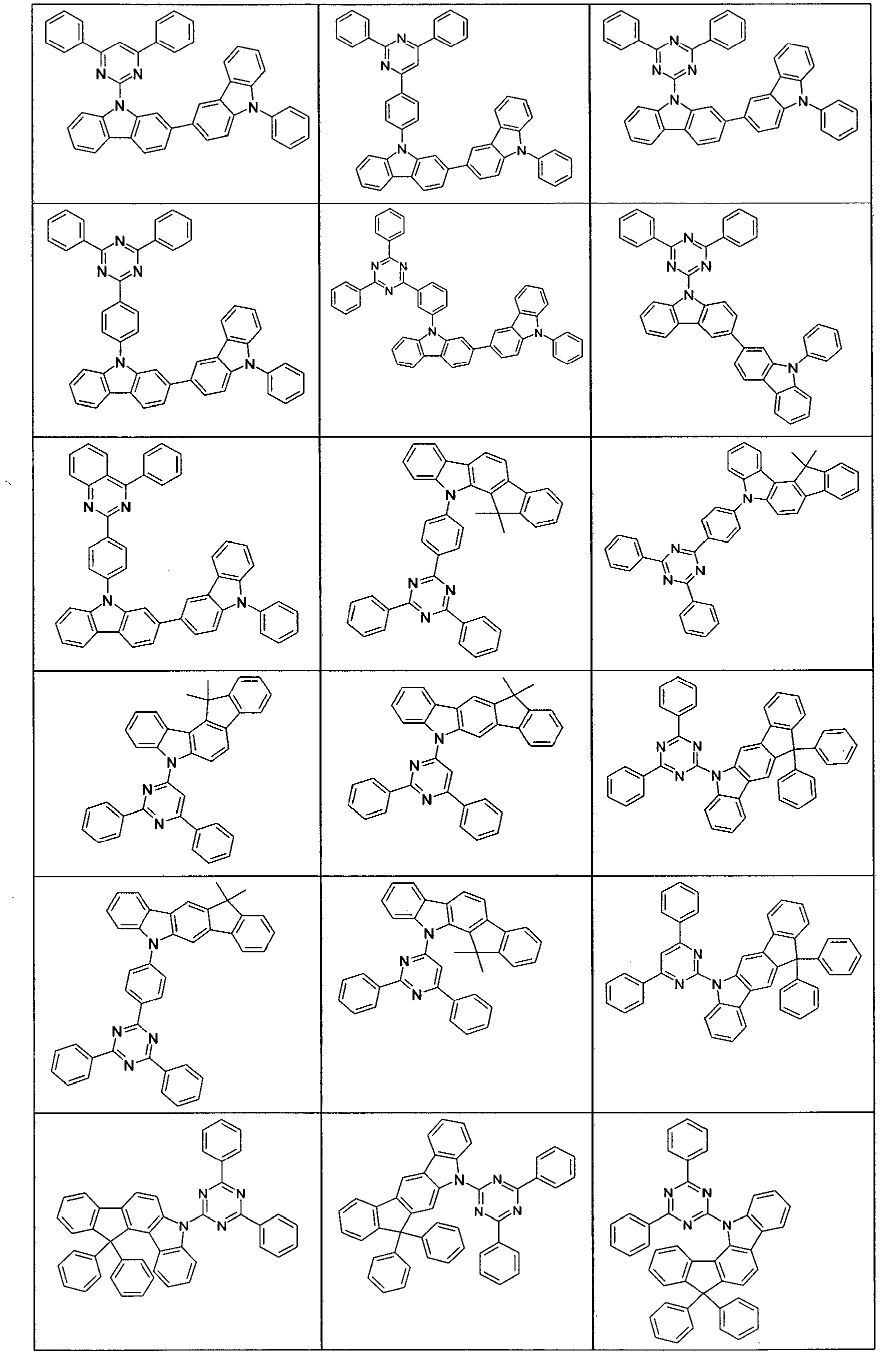

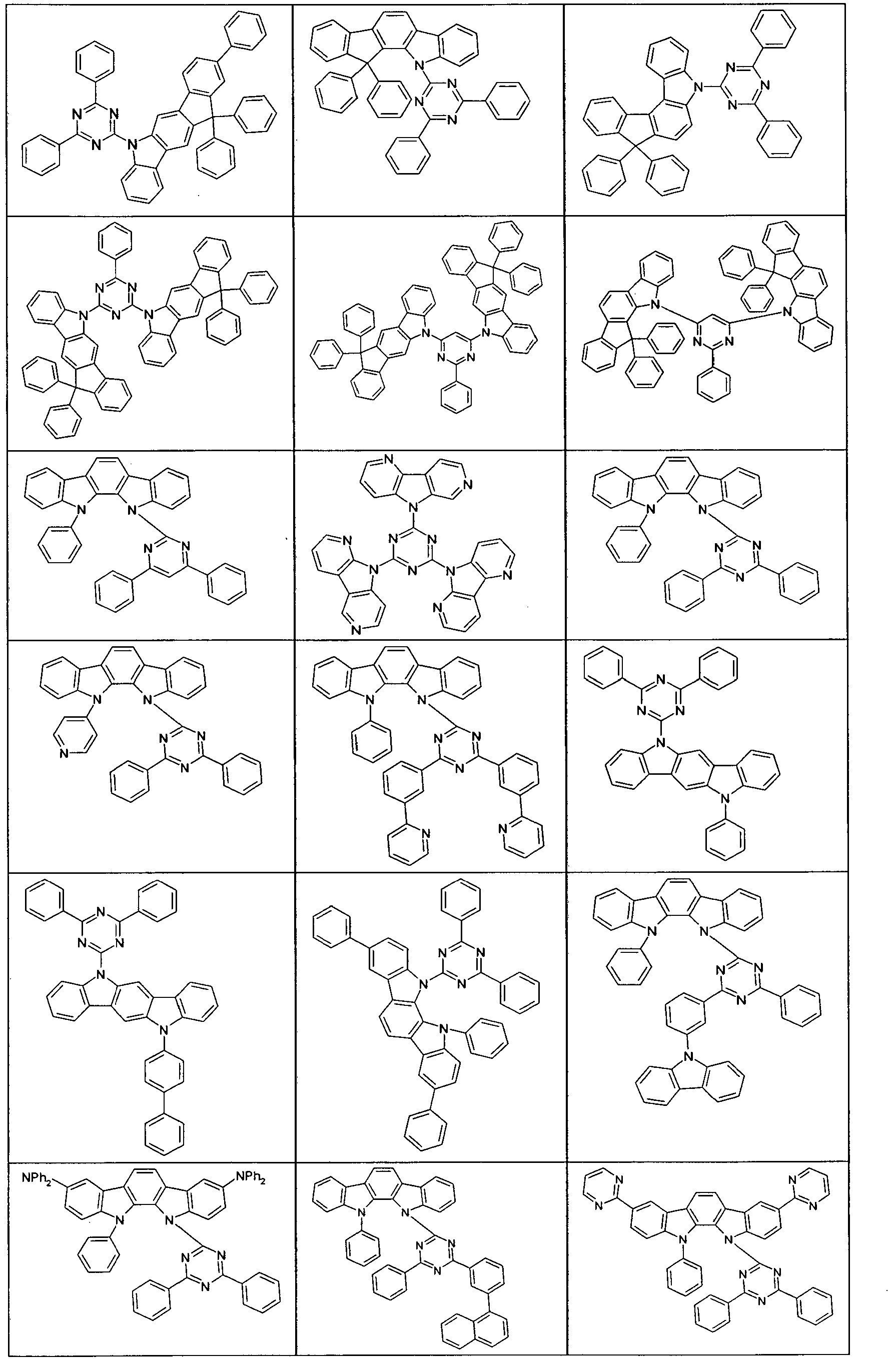

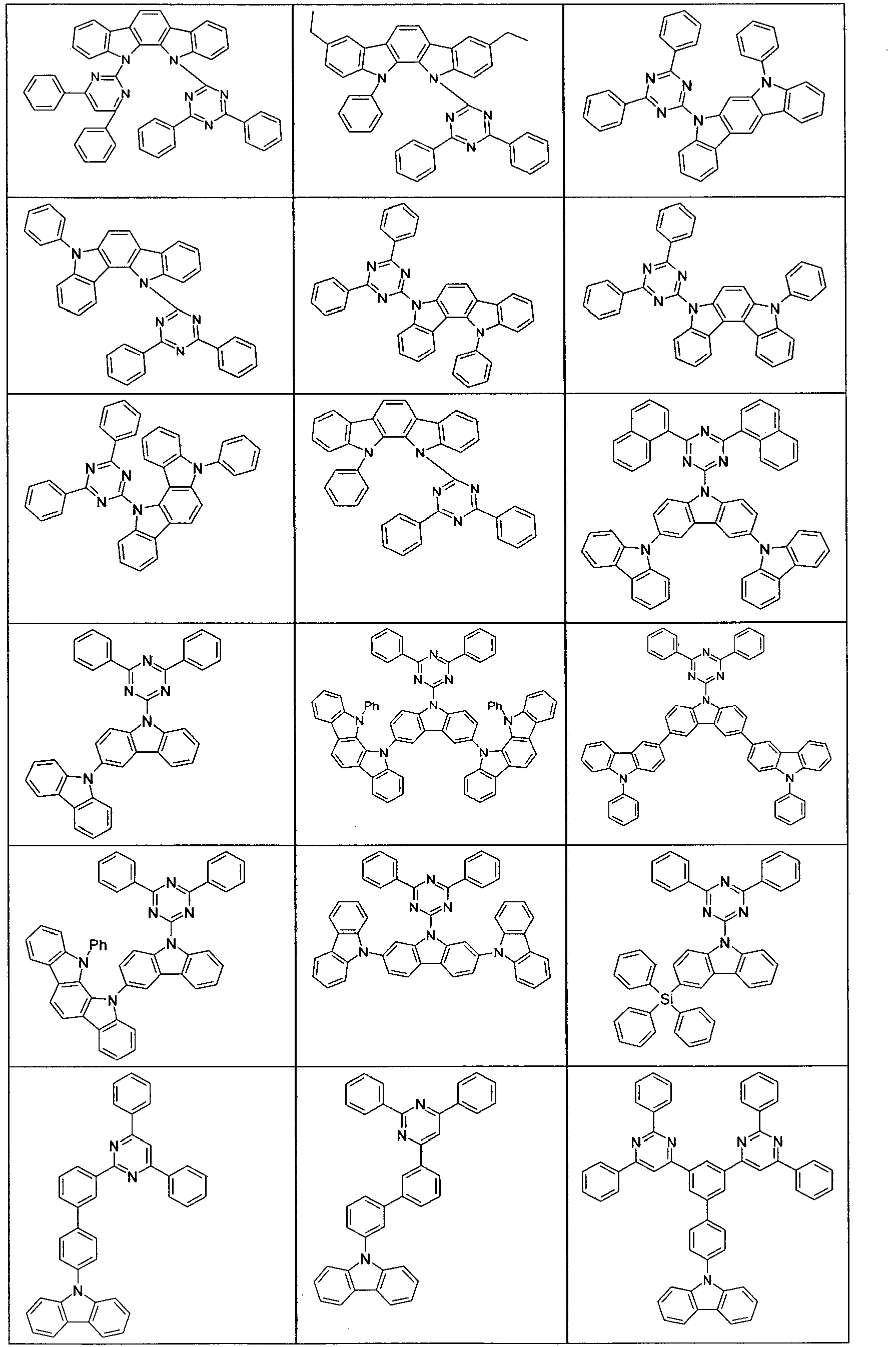

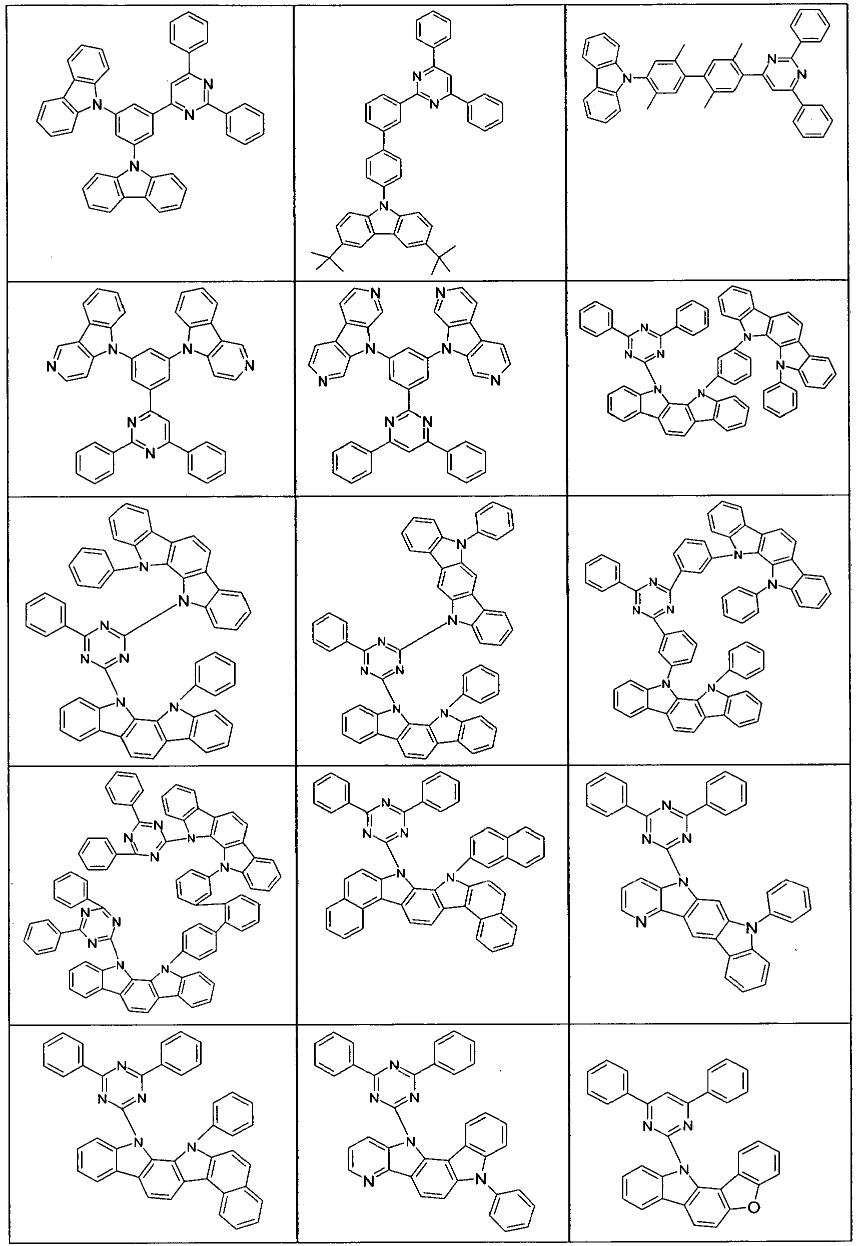

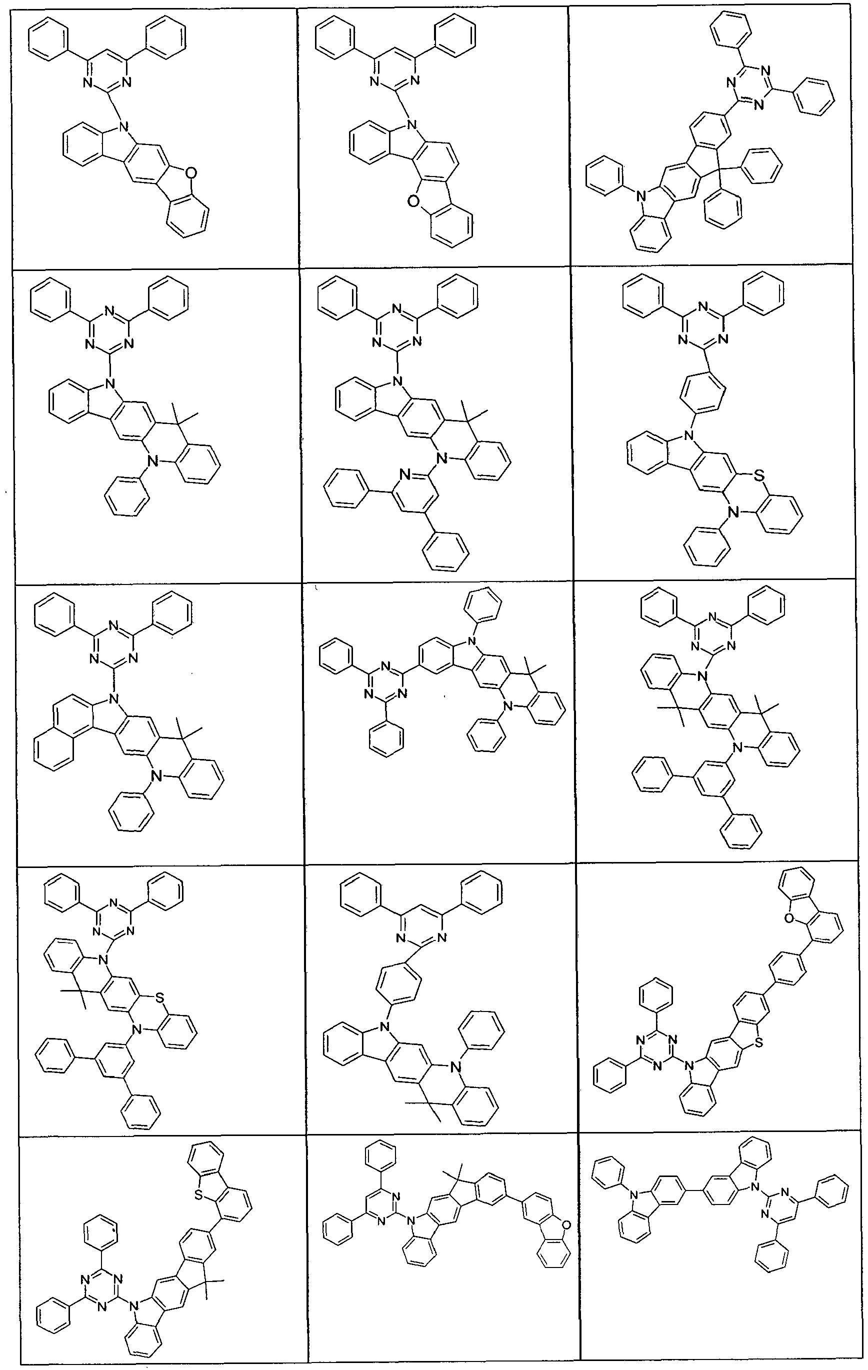

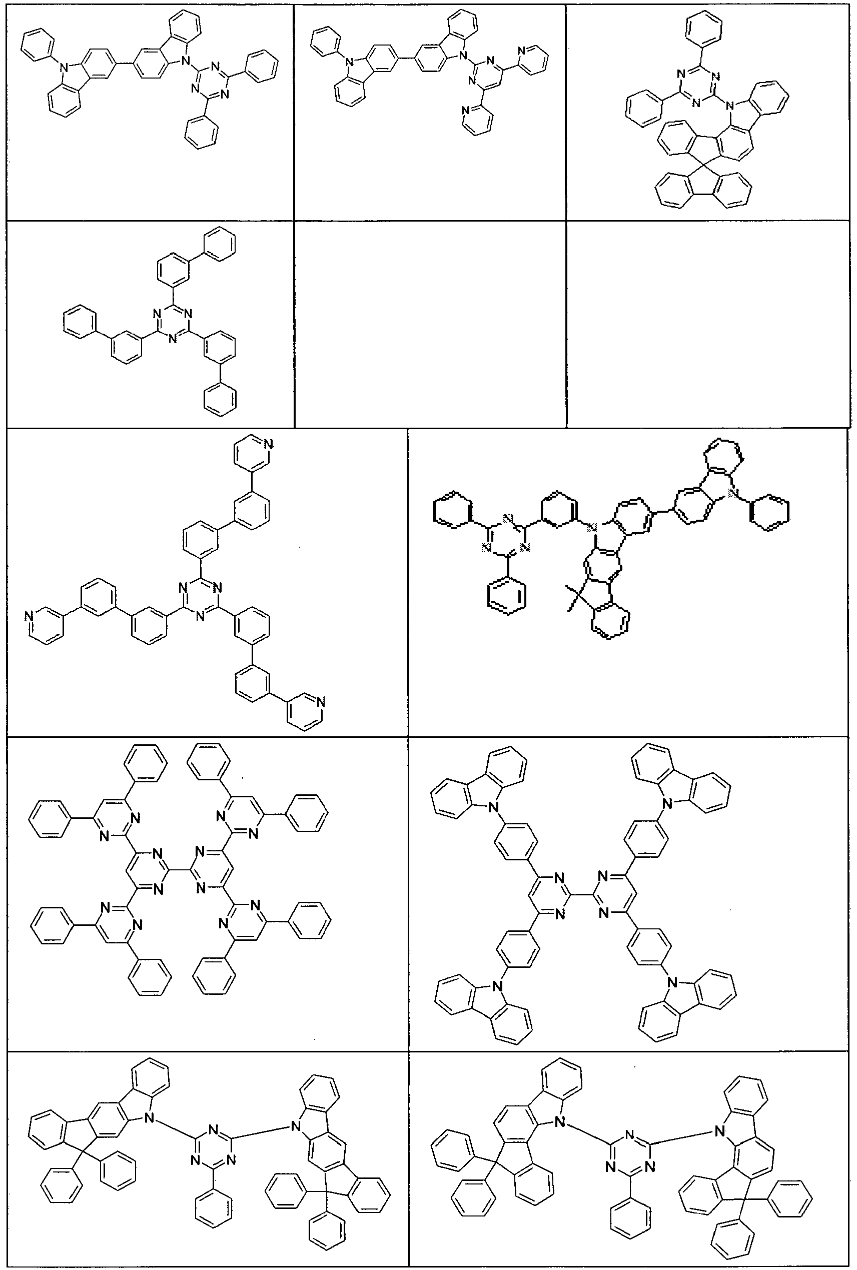

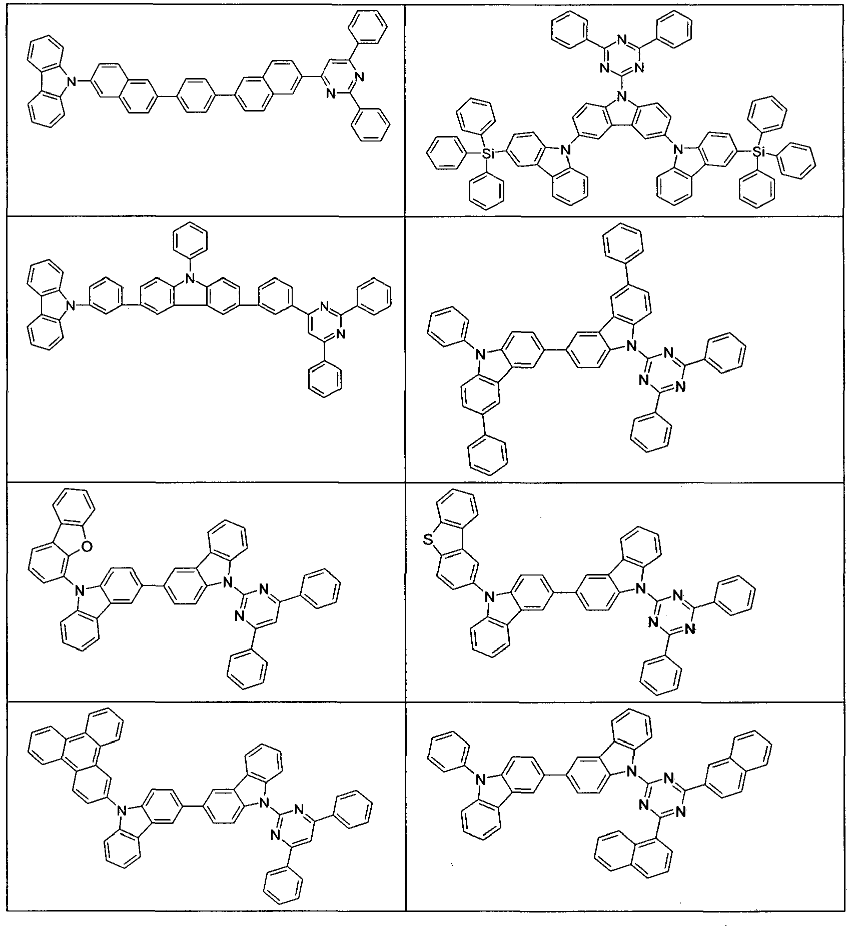

Wenn die elektronenleitende Verbindung ein Lactam ist, dann ist diese Verbindung bevorzugt ausgewählt aus den Verbindungen der folgenden Formeln (M45) bzw. (M46), 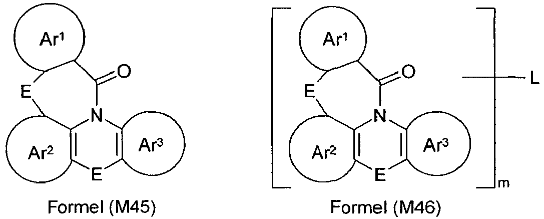 wobei R, R¹, R² und Ar die oben genannten Bedeutungen aufweisen und für die weiteren verwendeten Symbole und Indizes gilt:
- E: ist gleich oder verschieden bei jedem Auftreten eine Einfachbindung, NR, CR₂, O, S, SiR₂, BR, PR und P(=O)R;
- Ar¹, Ar², Ar³: ist gleich oder verschieden bei jedem Auftreten zusammen mit den explizit dargestellten Kohlenstoffatomen ein aromatisches oder heteroaromatisches Ringsystem mit 5 bis 30 aromatischen Ringatomen, welches durch einen oder mehrere Reste R substituiert sein kann;
- L: ist für m = 2 eine Einfachbindung oder eine bivalente Gruppe, bzw. für m = 3 eine trivalente Gruppe bzw. für m = 4 eine tetravalente Gruppe, welche jeweils an eine beliebige Position an Ar¹, Ar² oder Ar³ oder statt einem Rest R an E gebunden ist;
- m: ist 2, 3 oder 4.

In einer bevorzugten Ausführungsform der Verbindung gemäß Formel (M45) bzw. (M46) steht die Gruppe Ar¹ für eine Gruppe der folgenden Formel (M47), (M48), (M49) oder (M50), 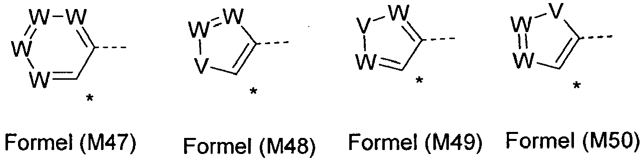 wobei die gestrichelte Bindung die Verknüpfung mit der Carbonylgruppe andeutet, * die Position der Verknüpfung mit E bzw. Ar² andeutet und weiterhin gilt:
- W: ist gleich oder verschieden bei jedem Auftreten CR oder N; oder zwei benachbarte Gruppen W stehen für eine Gruppe der folgenden Formel (M51) oder (M52), 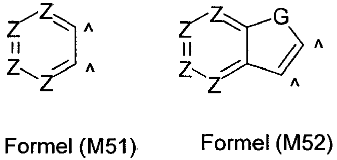 wobei G für CR₂, NR, O oder S steht, Z gleich oder verschieden bei jedem Auftreten für CR oder N steht und ^ die entsprechenden benachbarten Gruppen W in der Formel (M47) bis (M50) andeuten;
- V: ist NR, O oder S.

In einer weiteren bevorzugten Ausführungsform der Erfindung steht die Gruppe Ar² für eine Gruppe gemäß einer der folgenden Formeln (M53), (M54) oder (M55), 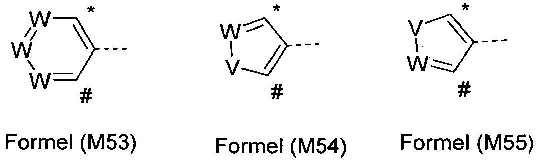 wobei die gestrichelte Bindung die Verknüpfung mit N andeutet, # die Position der Verknüpfung mit E bzw. Ar³ andeutet, * die Verknüpfung mit E bzw. Ar¹ andeutet und W und V die oben genannten Bedeutungen aufweisen.

In einer weiteren bevorzugten Ausführungsform der Erfindung steht die Gruppe Ar³ für eine Gruppe gemäß einer der folgenden Formeln (M56), (M57), (M58) oder (M59), 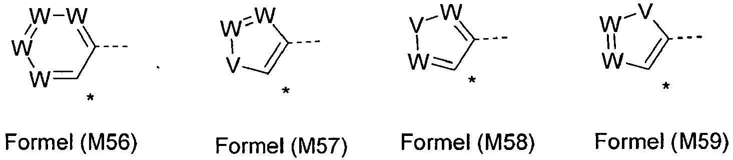 wobei die gestrichelte Bindung die Verknüpfung mit N andeutet, * die Verknüpfung mit E bzw. Ar² andeutet und W und V die oben genannten Bedeutungen aufweisen.

Dabei können die oben genannten bevorzugten Gruppen Ar¹, Ar² und Ar³ beliebig miteinander kombiniert werden.

In einer weiteren bevorzugten Ausführungsform der Erfindung steht mindestens eine Gruppe E für eine Einfachbindung.

In einer bevorzugten Ausführungsform der Erfindung treten die oben genannten Bevorzugungen gleichzeitig auf. Besonders bevorzugt sind daher Verbindungen gemäß Formel (M45) bzw. (M46), für die gilt:
- Ar¹: ist ausgewählt aus den Gruppen der oben genannten Formeln (M47), (M48), (M49) oder (M50);
- Ar²: ist ausgewählt aus den Gruppen der oben genannten Formeln (M53), (M54) oder (M55);
- Ar³: ist ausgewählt aus den Gruppen der oben genannten Formeln (M56), (M57), (M58) oder (M59).

Besonders bevorzugt stehen mindestens zwei der Gruppen Ar¹, Ar² und Ar³ für eine 6-Ring-Aryl- oder eine 6-Ring-Heteroarylgruppe. Besonders bevorzugt steht also Ar¹ für eine Gruppe der Formel (M47) und gleichzeitig steht Ar² für eine Gruppe der Formel (M53), oder Ar¹ steht für eine Gruppe der Formel (M47) und gleichzeitig steht Ar³ für eine Gruppe der Formel (M56), oder Ar² steht für eine Gruppe der Formel (M53) und gleichzeitig steht Ar³ für eine Gruppe der Formel (M59).

Besonders bevorzugte Ausführungsformen der Formel (M45) sind daher die Verbindungen der folgenden Formeln (M60) bis (M69), 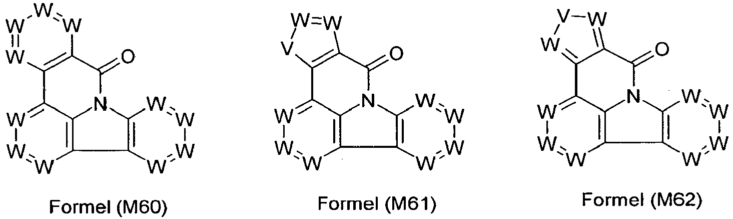 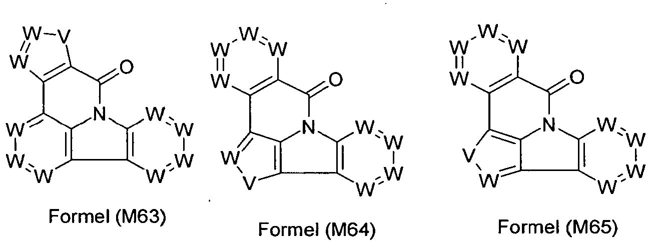 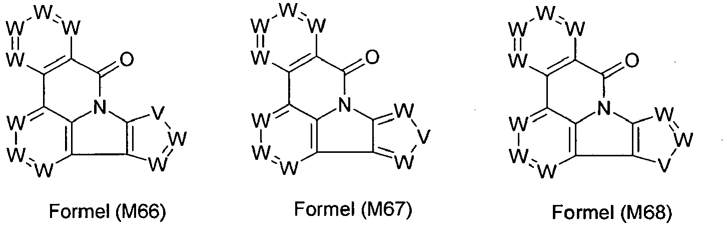 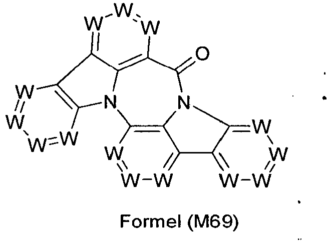 wobei die verwendeten Symbole die oben genannten Bedeutungen aufweisen.

Es ist weiterhin bevorzugt, wenn W für CR oder N steht und nicht für eine Gruppe der Formel (M51) oder (M52). In einer bevorzugten Ausführungsform der Verbindungen gemäß Formel (M60) bis (M69) steht pro Cyclus insgesamt maximal ein Symbol W für N, und die verbleibenden Symbole W stehen für CR. In einer besonders bevorzugten Ausführungsform der Erfindung stehen alle Symbole W für CR. Besonders bevorzugt sind daher die Verbindungen gemäß den folgenden Formeln (M60a) bis (M69a), 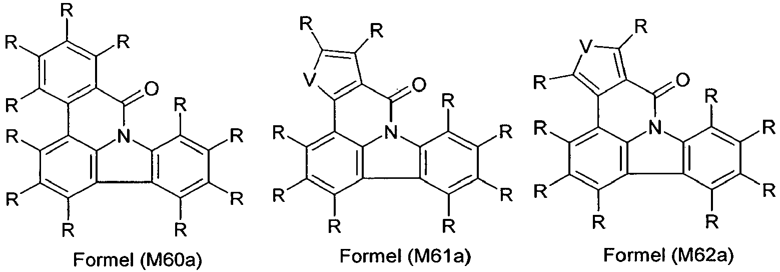 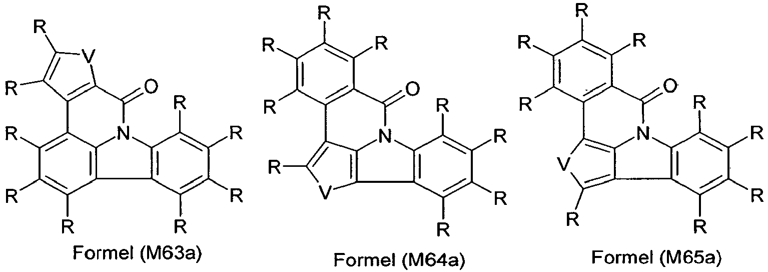 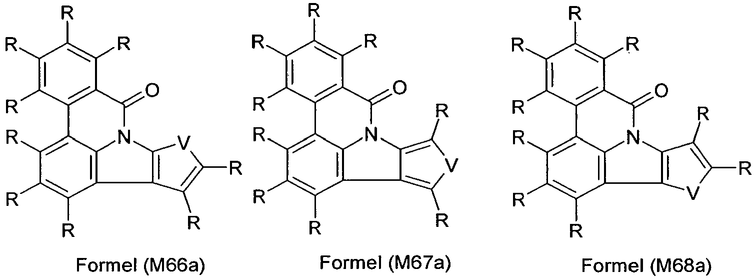 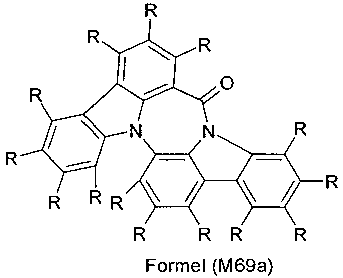 wobei die verwendeten Symbole die oben genannten Bedeutungen aufweisen.

Ganz besonders bevorzugt sind die Strukturen der Formeln (M60b) bis (M69b), 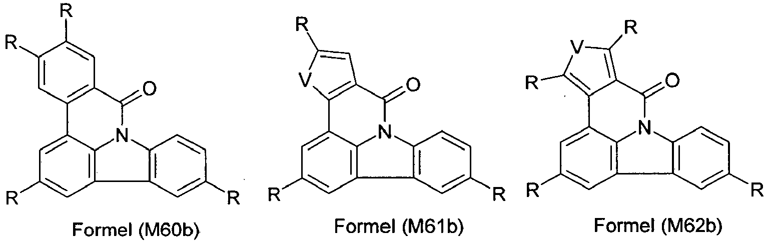 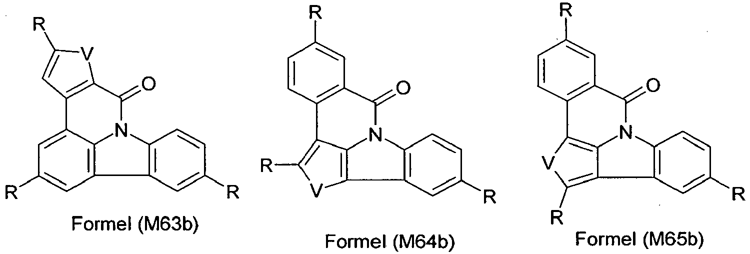 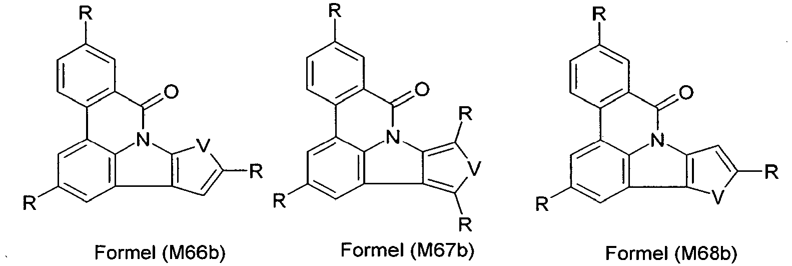 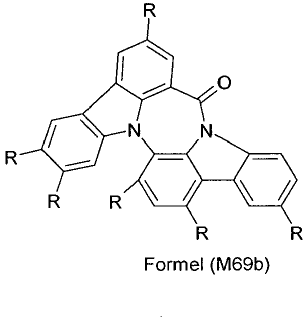 wobei die verwendeten Symbole die oben genannten Bedeutungen aufweisen.

Ganz besonders bevorzugt sind die Verbindungen der Formel (M60) bzw. (M60a) bzw. (M60b).

Die verbrückende Gruppe L in den Verbindungen der Formeln (M46a) ist bevorzugt ausgewählt aus einer Einfachbindung oder einem aromatischen oder heteroaromatischen Ringsystem mit 5 bis 30 aromatischen Ringatomen, welches durch einen oder mehrere Reste R substituiert sein kann. Dabei enthalten die aromatischen bzw. heteroaromatischen Ringsysteme bevorzugt keine kondensierten Aryl- bzw. Heteroarylgruppen, in denen mehr als zwei aromatische Sechsringe direkt aneinander ankondensiert sind. Besonders bevorzugt enthalten sie überhaupt keine Aryl- bzw. Heteroarylgruppen, in denen aromatische Sechsringe direkt aneinander ankondensiert sind.

In einer weiteren bevorzugten Ausführungsform der Erfindung ist der Index m in Verbindungen der Formel (M46) = 2 oder 3, insbesondere gleich 2.

In einer bevorzugten Ausführungsform der Erfindung ist R in den Formeln (M45) und (M46) sowie den bevorzugten Ausführungsformen gleich oder verschieden bei jedem Auftreten ausgewählt aus der Gruppe bestehend aus H, D, F, Cl, Br, CN, N(Ar)₂, C(=O)Ar, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R¹ substituiert sein kann, einer Aryloxy- oder Heteroaryloxygruppe mit 5 bis 30 aromatischen Ringatomen, die mit einem oder mehreren Resten R¹ substituiert sein kann, oder einer Kombination dieser Systeme.

In einer besonders bevorzugten Ausführungsform der Erfindung ist R in den oben genannten Formeln gleich oder verschieden bei jedem Auftreten ausgewählt aus der Gruppe bestehend aus H, D, F, Cl, Br, CN, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R¹ substituiert sein kann, wobei ein oder mehrere H-Atome durch D oder F ersetzt sein können, einem aromatischen oder heteroaromatischen Ringsystem mit 5 bis 18 aromatischen Ringatomen, das jeweils mit einem oder mehreren Resten R¹ substituiert sein kann, oder einer Kombination dieser Systeme.

Dabei enthalten die Reste R, wenn diese aromatische oder heteroaromatischen Ringsysteme enthalten, bevorzugt keine kondensierten Aryl- bzw. Heteroarylgruppen, in denen mehr als zwei aromatische Sechsringe direkt aneinander ankondensiert sind. Besonders bevorzugt enthalten sie überhaupt keine Aryl- bzw. Heteroarylgruppen, in denen aromatische Sechsringe direkt aneinander ankondensiert sind. Insbesondere bevorzugt sind hier Phenyl, Biphenyl, Terphenyl, Quaterphenyl, Carbazol, Dibenzothiophen, Dibenzofuran, Indenocarbazol, Indolocarbazol, Triazin oder Pyrimidin, welches jeweils auch durch einen oder mehrere Reste R¹ substituiert sein kann.

Dabei haben für Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom.

Die Verbindungen der Formeln (M45) und (M46) sind prinzipiell bekannt. Die Synthese dieser Verbindungen kann gemäß den in der WO 2011/116865 und WO 2011/137951 beschriebenen Verfahren erfolgen.

Beispiele für bevorzugte Verbindungen gemäß den oben aufgeführten Ausführungsformen sind die in der folgenden Tabelle aufgeführten Verbindungen.

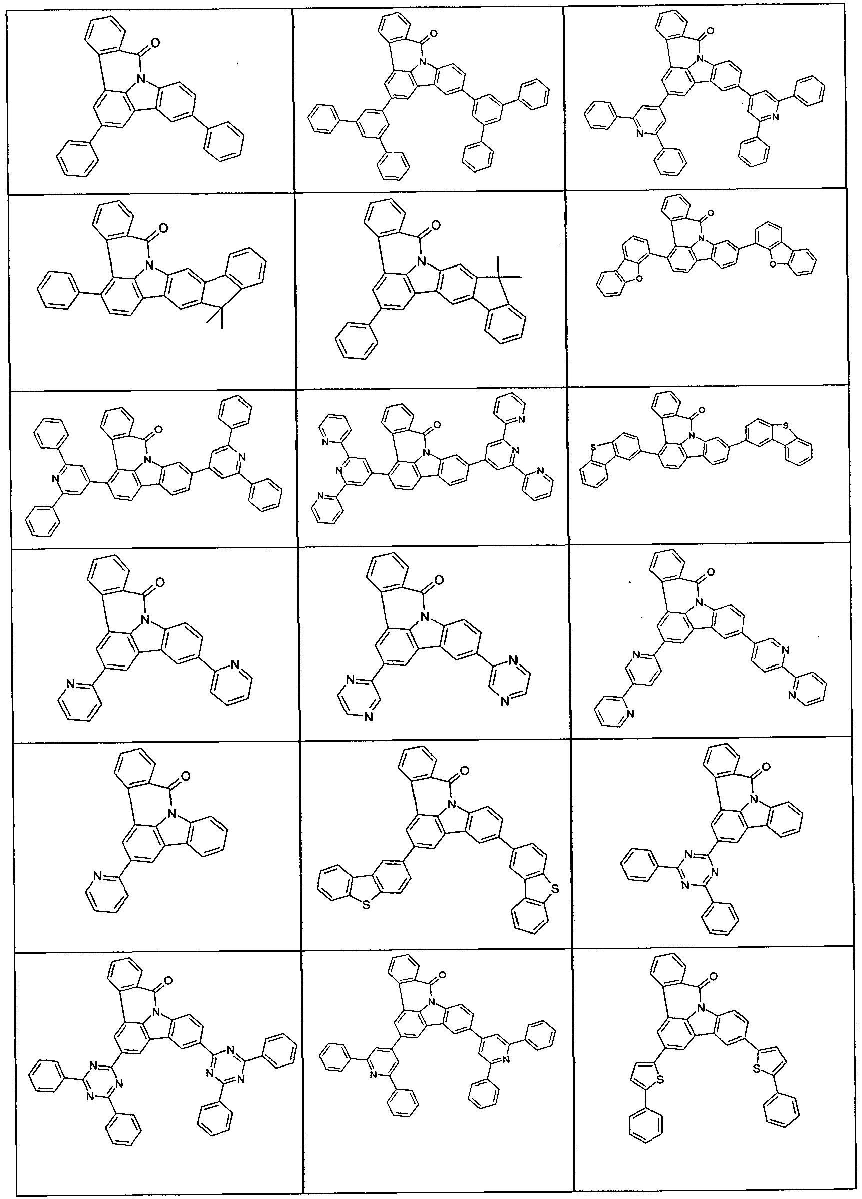

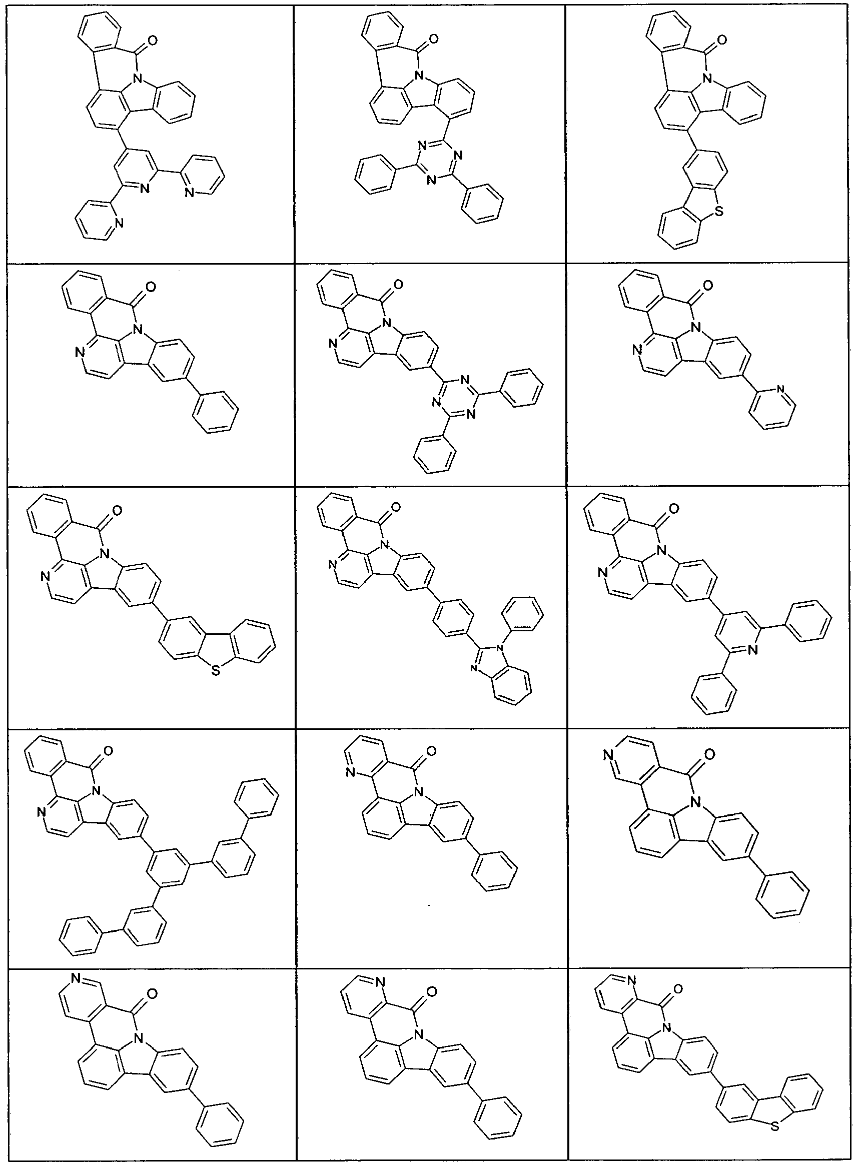

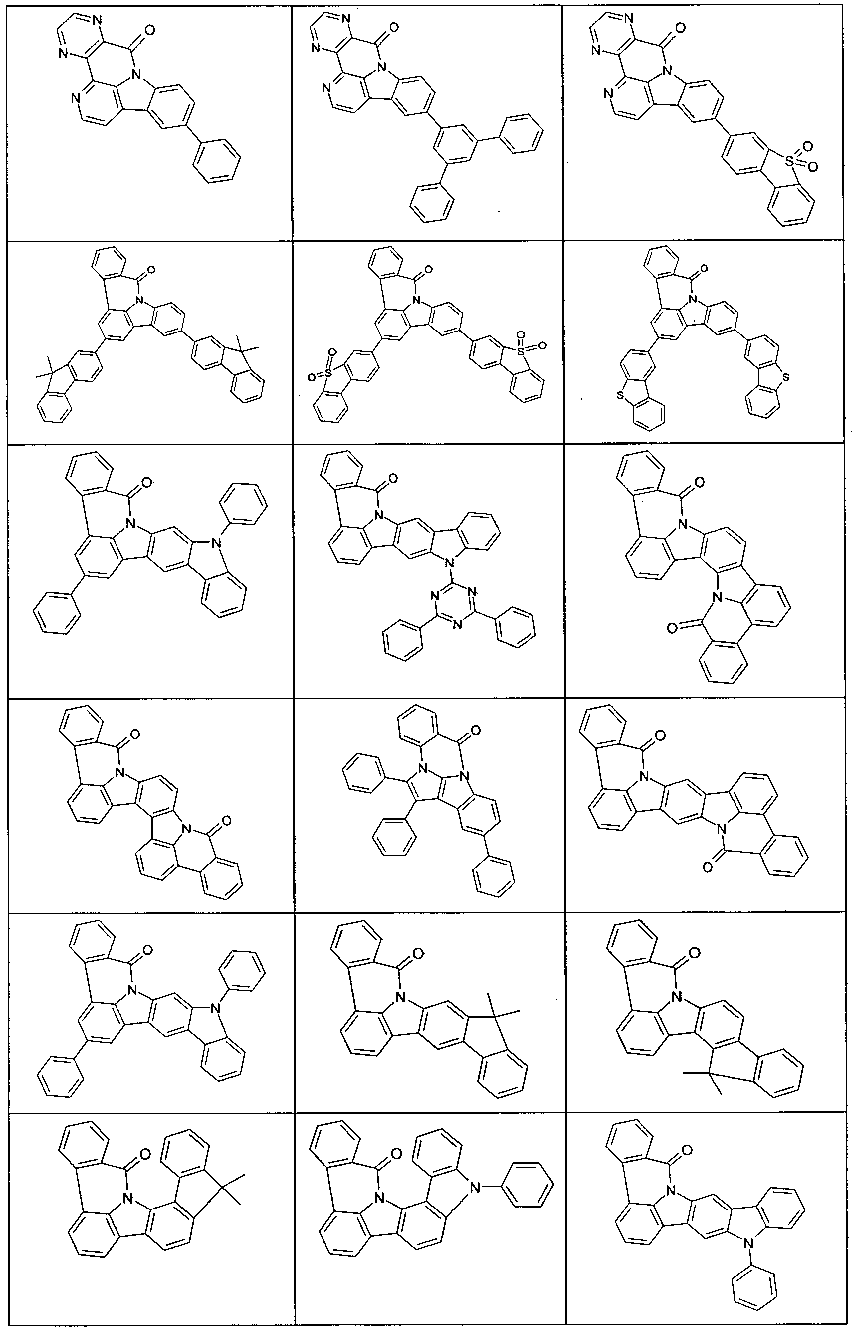

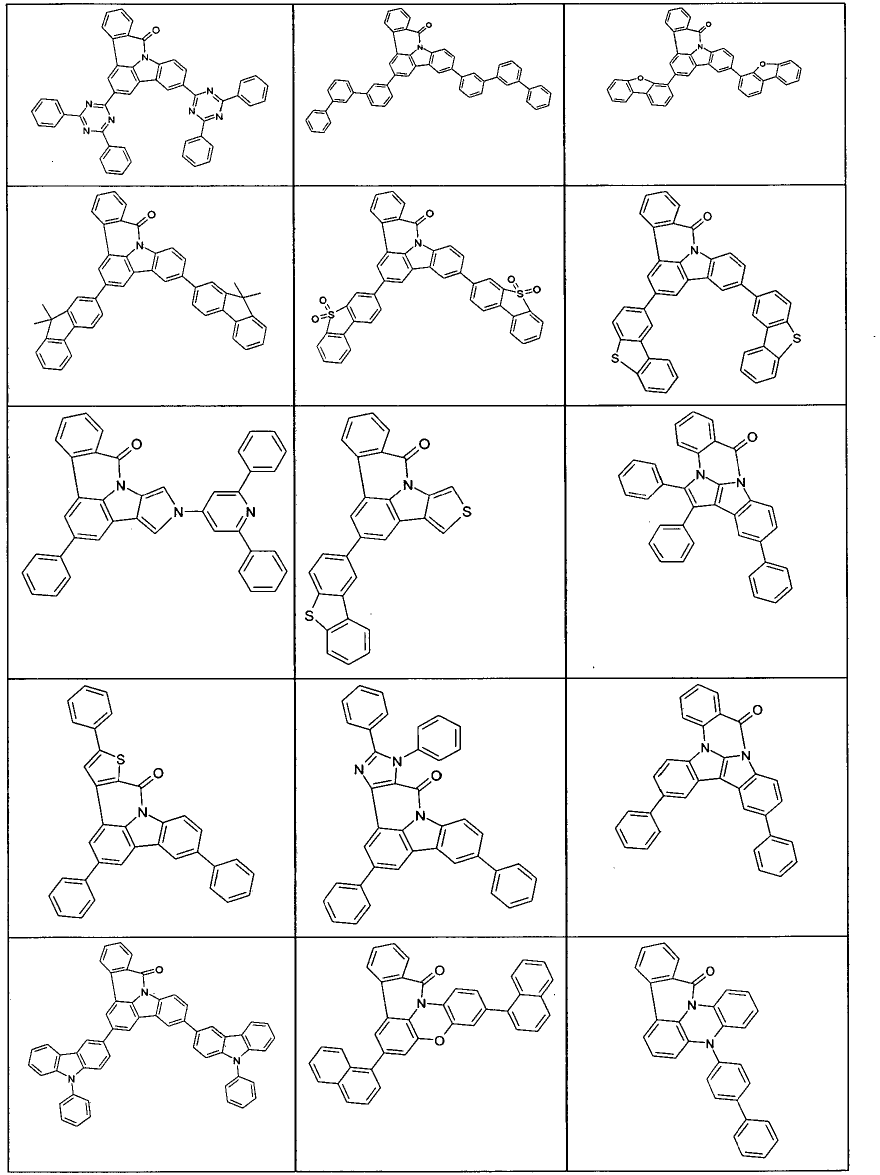

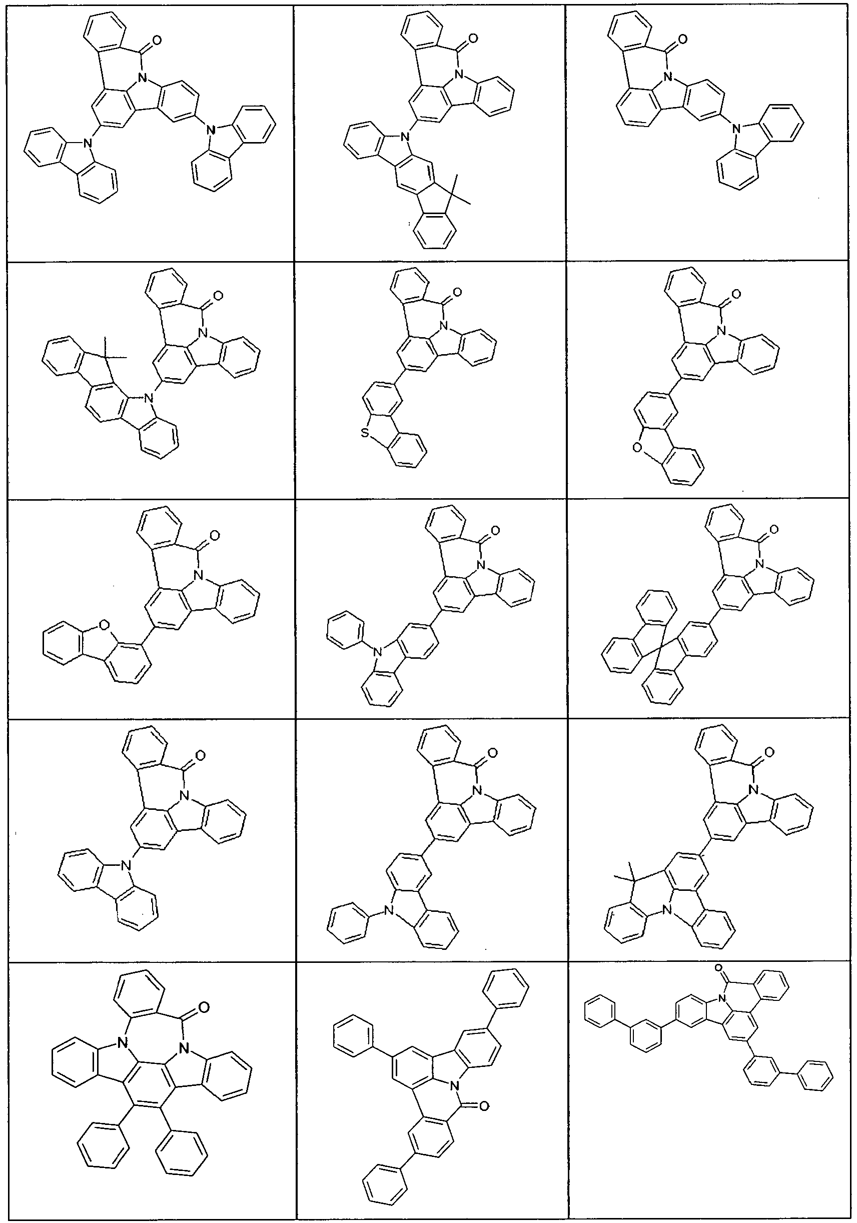

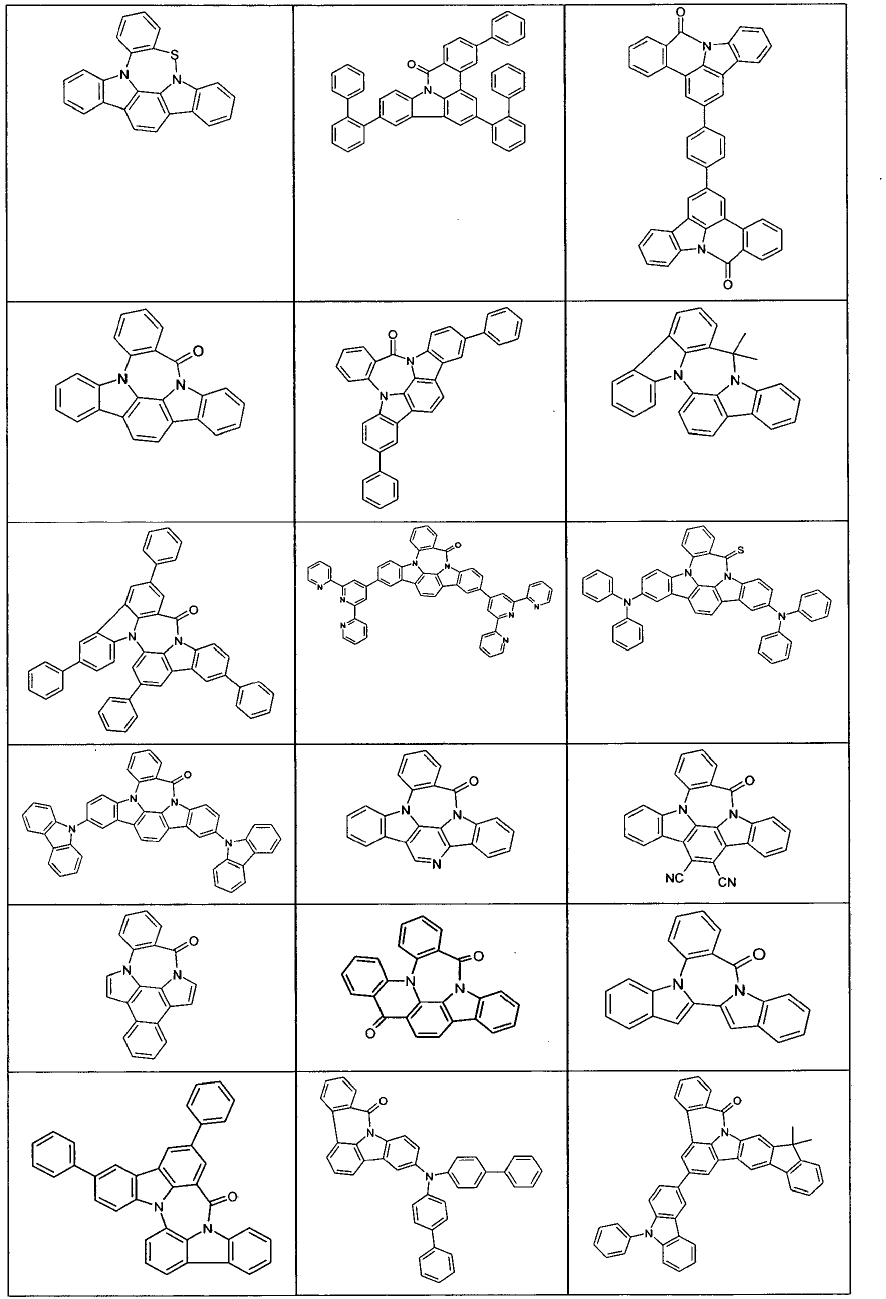

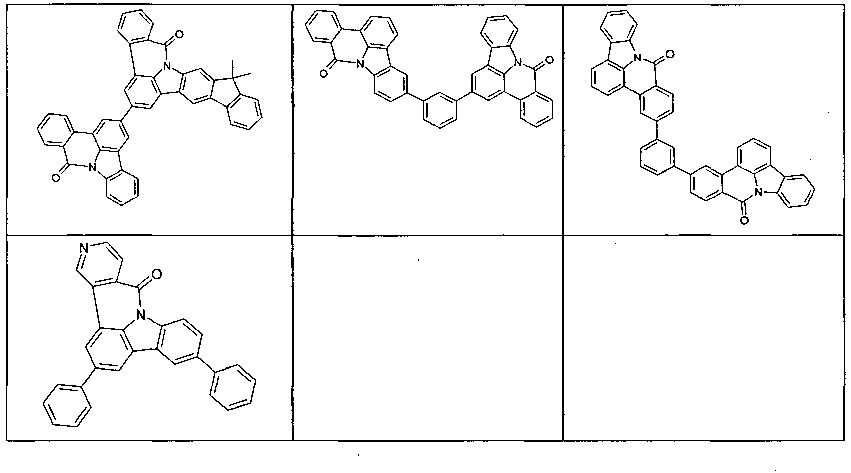

Weiterhin eignen sich aromatische Ketone oder aromatische Phosphinoxide als elektronenleitende Verbindung. Unter einem aromatischen Keton im Sinne dieser Anmeldung wird eine Carbonylgruppe verstanden, an die zwei aromatische oder heteroaromatische Gruppen bzw. aromatische oder heteroaromatische Ringsysteme direkt gebunden sind. Unter einem aromatischen Phosphinoxid im Sinne dieser Anmeldung wird eine P=O Gruppe verstanden, an die drei aromatische oder heteroaromatische Gruppen bzw. aromatische oder heteroaromatische Ringsysteme direkt gebunden sind

Wenn die elektronenleitende Verbindung ein aromatisches Keton oder ein aromatisches Phosphinoxid ist, dann ist diese Verbindung bevorzugt ausgewählt aus den Verbindungen der folgenden Formeln (M70) bzw. (M71), 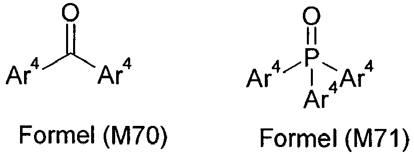 wobei R, R¹, R² und Ar die oben aufgeführten Bedeutungen hat und für die weiteren verwendeten Symbole gilt:
- Ar⁴: ist bei jedem Auftreten gleich oder verschieden ein aromatisches oder heteroaromatisches Ringsystem mit 5 bis 80 aromatischen Ringatomen, bevorzugt bis 60 aromatischen Ringatomen, welches jeweils mit einer oder mehreren Gruppen R substituiert sein kann.

Geeignete Verbindungen gemäß Formel (M70) und (M71) sind insbesondere die in WO 2004/093207 und WO 2010/006680 offenbarten Ketone und die in WO 2005/003253 offenbarten Phosphinoxide.

Aus der Definition der Verbindung gemäß Formel (M70) und (M70) geht hervor, dass diese nicht nur eine Carbonylgruppe bzw. Phosphinoxidgruppe enthalten muss, sondern auch mehrere dieser Gruppen enthalten kann.

Bevorzugt ist die Gruppe Ar⁴ in Verbindungen gemäß Formel (M70) bzw. (M71) ein aromatisches Ringsystem mit 6 bis 40 aromatischen Ringatomen, d. h. sie enthält keine Heteroarylgruppen. Wie oben definiert, muss das aromatische Ringsystem nicht notwendigerweise nur aromatische Gruppen aufweisen, sondern es können auch zwei Arylgruppen durch eine nicht-aromatische Gruppe, beispielsweise durch eine weitere Carbonylgruppe bzw. Phosphinoxidgruppe unterbrochen sein.

In einer weiteren bevorzugten Ausführungsform der Erfindung weist die Gruppe Ar⁴ nicht mehr als zwei kondensierte Ringe auf. Sie ist also bevorzugt nur aus Phenyl- und/oder Naphthylgruppen, besonders bevorzugt nur aus Phenylgruppen, aufgebaut, enthält aber keine größeren kondensierten Aromaten, wie beispielsweise Anthracen.

Bevorzugte Gruppen Ar⁴, die an die Carbonylgruppe gebunden sind, sind gleich oder verschieden bei jedem Auftreten Phenyl, 2-, 3- oder 4-Tolyl, 3- oder 4-o-Xylyl, 2- oder 4-m-Xylyl, 2-p-Xylyl, o-, m- oder p-tert-Butylphenyl, o-, m- oder p-Fluorphenyl, Benzophenon, 1-, 2- oder 3-Phenylmethanon, 2-, 3- oder 4-Biphenyl, 2-, 3- oder 4-o-Terphenyl, 2-, 3- oder 4-m-Terphenyl, 2-, 3- oder 4-p-Terphenyl, 2'-p-Terphenyl, 2'-, 4'- oder 5'-m-Terphenyl, 3'- oder 4'-o-Terphenyl, p-, m,p-, o,p-, m,m-, o,m- oder o,o-Quaterphenyl, Quinquephenyl, Sexiphenyl, 1-, 2-, 3- oder 4-Fluorenyl, 2-, 3- oder 4-Spiro-9,9'-bifluorenyl, 1-, 2-, 3- oder 4-(9,10-Dihydro)-phenanthrenyl, 1- oder 2-Naphthyl, 2-, 3-, 4-, 5-, 6-, 7- oder 8-Chinolinyl, 1-, 3-, 4-, 5-, 6-, 7- oder 8-iso-Chinolinyl, 1- oder 2-(4-Methylnaphthyl), 1- oder 2-(4-Phenylnaphthyl), 1- oder 2-(4-Naphthyl-naphthyl), 1-, 2- oder 3-(4-naphthyl-phenyl), 2-, 3- oder 4-Pyridyl, 2-, 4- oder 5-Pyrimidinyl, 2- oder 3-Pyrazinyl, 3- oder 4-Pyridanzinyl, 2-(1,3,5-Triazin)yl-, 2-, 3- oder 4-(Phenylpyridyl), 3-, 4-, 5- oder 6-(2,2'-Bipyridyl), 2-, 4-, 5- oder 6-(3,3'-Bipyridyl), 2- oder 3-(4,4'-Bipyridyl) und Kombinationen eines oder mehrerer dieser Reste.

Die Gruppen Ar⁴ können durch einen oder mehrere Reste R substituiert sein. Diese Reste R sind bevorzugt gleich oder verschieden bei jedem Auftreten gewählt aus der Gruppe bestehend aus H, D, F, C(=O)Ar, P(=O)(Ar)₂, S(=O)Ar, S(=O)₂Ar, einer geradkettigen Alkylgruppe mit 1 bis 4 C-Atomen oder einer verzweigten oder cyclischen Alkylgruppe mit 3 bis 5 C-Atomen, die jeweils mit einem oder mehreren Resten R¹ substituiert sein kann, wobei ein oder mehrere H-Atome durch F ersetzt sein können, oder einem aromatischen Ringsystem mit 6 bis 24 aromatischen Ringatomen, das durch einen oder mehrere Reste R¹ substituiert sein kann, oder eine Kombination dieser Systeme; dabei können zwei oder mehrere benachbarte Substituenten R auch miteinander ein mono- oder polycyclisches, aliphatisches oder aromatisches Ringsystem bilden. Wenn die organische Elektrolumineszenzvorrichtung aus Lösung aufgebracht wird, sind auch geradkettige, verzweigte oder cyclische Alkylgruppen mit bis zu 10 C-Atomen als Substituenten R bevorzugt. Die Reste R sind besonders bevorzugt gleich oder verschieden bei jedem Auftreten gewählt aus der Gruppe bestehend aus H, C(=O)Ar oder einem aromatischen Ringsystem mit 6 bis 24 aromatischen Ringatomen, das durch einen oder mehrere Reste R¹ substituiert sein kann, bevorzugt aber unsubstituiert ist.

In nochmals einer bevorzugten Ausführungsform der Erfindung ist die Gruppe Ar gleich oder verschieden bei jedem Auftreten ein aromatisches Ringsystem mit 6 bis 24 aromatischen Ringatomen, das mit einem oder mehreren Resten R¹ substituiert sein kann. Besonders bevorzugt ist Ar gleich oder verschieden bei jedem Auftreten ein aromatisches Ringsystem mit 6 bis 12 aromatischen Ringatomen.

Bevorzugt sind weiterhin Benzophenon-Derivate, die jeweils an den 3,5,3',5'-Positionen durch ein aromatisches oder heteroaromatisches Ringsystem mit 5 bis 30 aromatischen Ringatomen substituiert sind, welche wiederum durch einen oder mehrere Reste R gemäß der obigen Definition substituiert sein kann. Weiterhin bevorzugt sind Ketone, welche mit mindestens einer Spirobifluorengruppe substituiert sind.

Bevorzugte aromatische Ketone und Phosphinoxide sind daher die Verbindungen der folgenden Formel (M72) bis (M75), 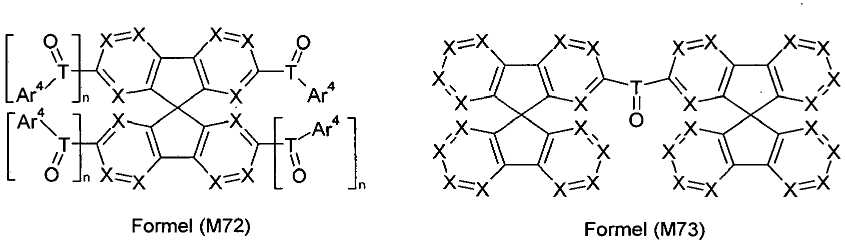 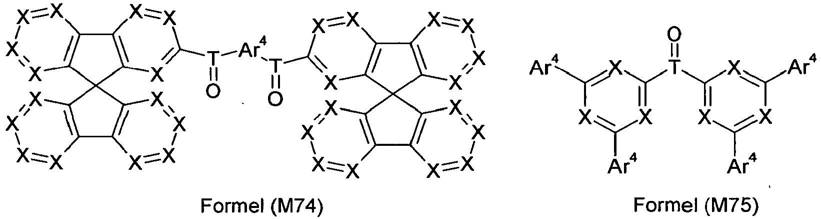 wobei X, Ar⁴, R, R¹ und R² dieselbe Bedeutung haben, wie oben beschrieben, und weiterhin gilt:
- T: ist gleich oder verschieden bei jedem Auftreten C oder P(Ar⁴);
- n: ist gleich oder verschieden bei jedem Auftreten 0 oder 1.

Bevorzugt steht Ar⁴ in der oben genannten Formel (M72) und (M75) für ein aromatisches oder heteroaromatisches Ringsystem mit 5 bis 30 aromatischen Ringatomen, welches mit einem oder mehreren Resten R¹ substituiert sein kann. Besonders bevorzugt sind die oben genannten Gruppen Ar⁴.

Beispiele für geeignete Verbindungen gemäß Formel (M70) und (M71) sind die in der folgenden Tabelle abgebildeten Verbindungen.

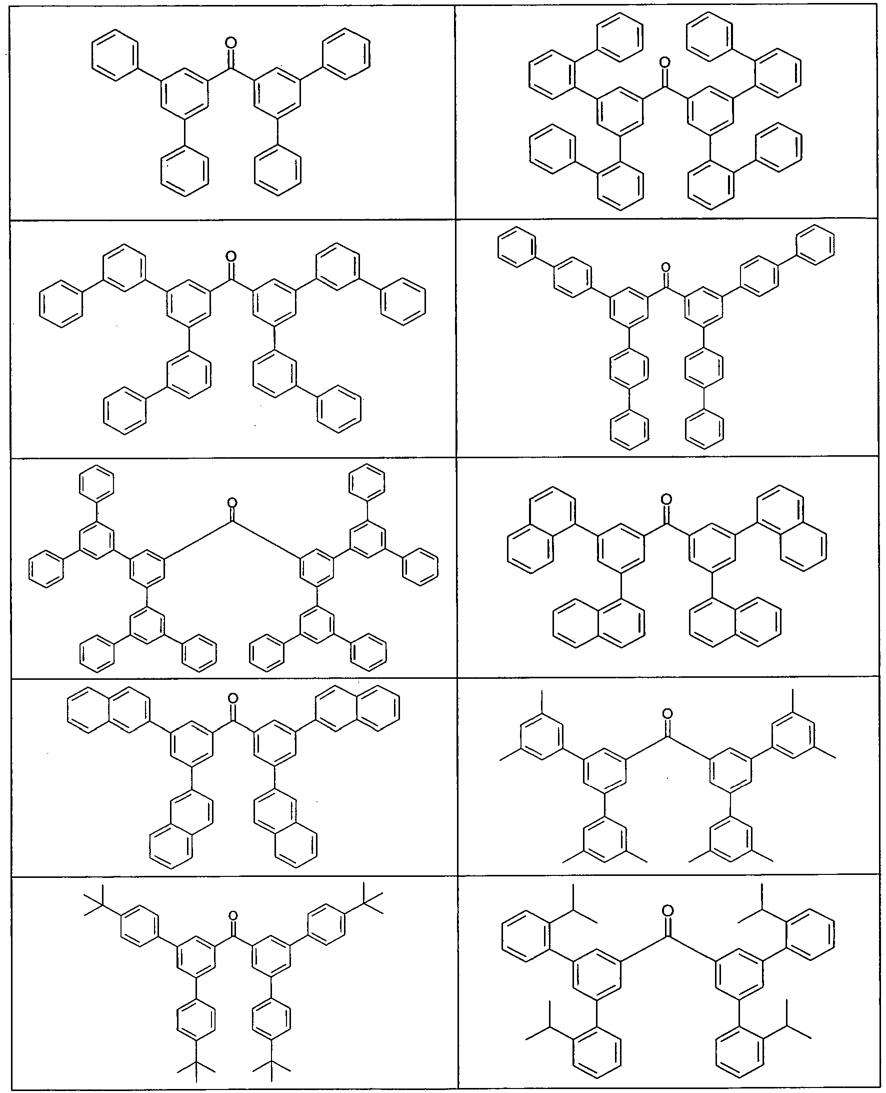

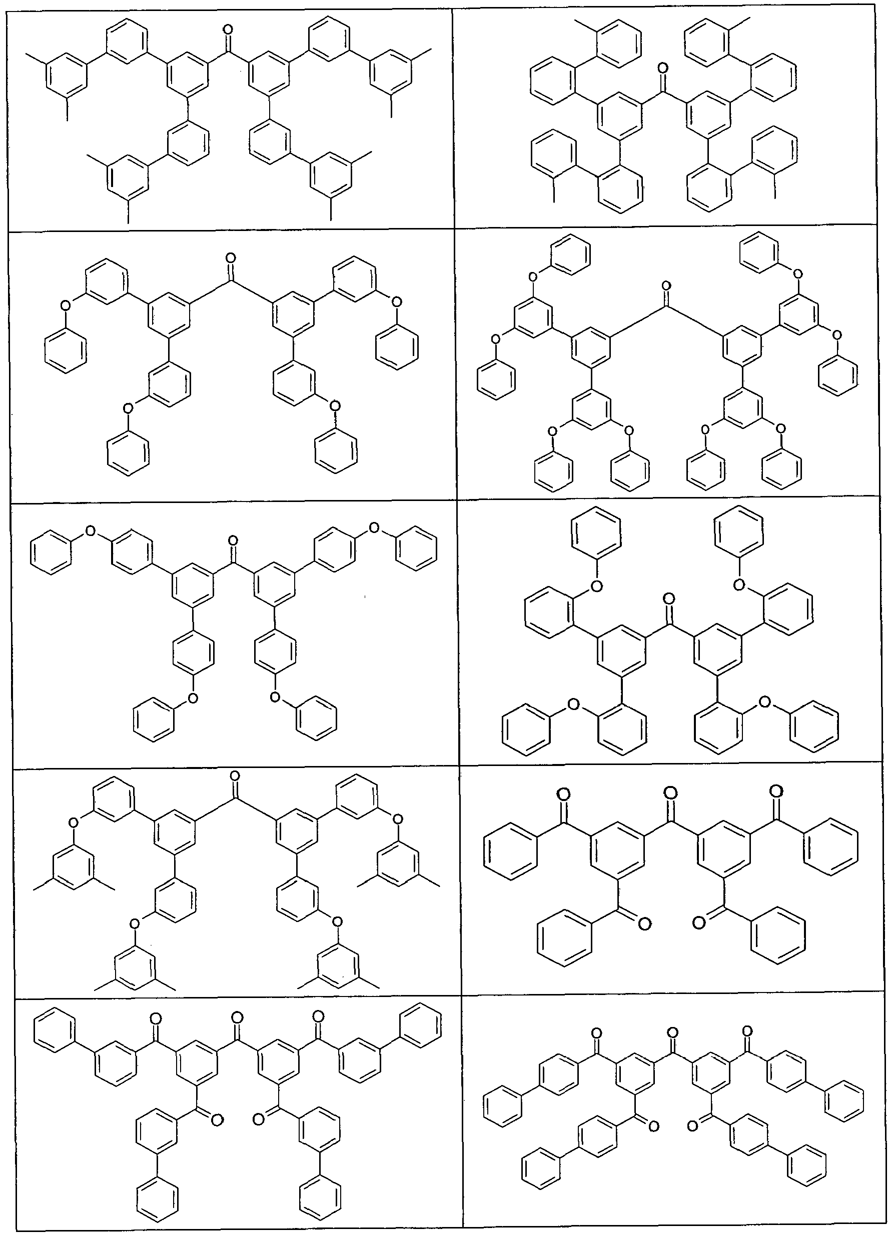

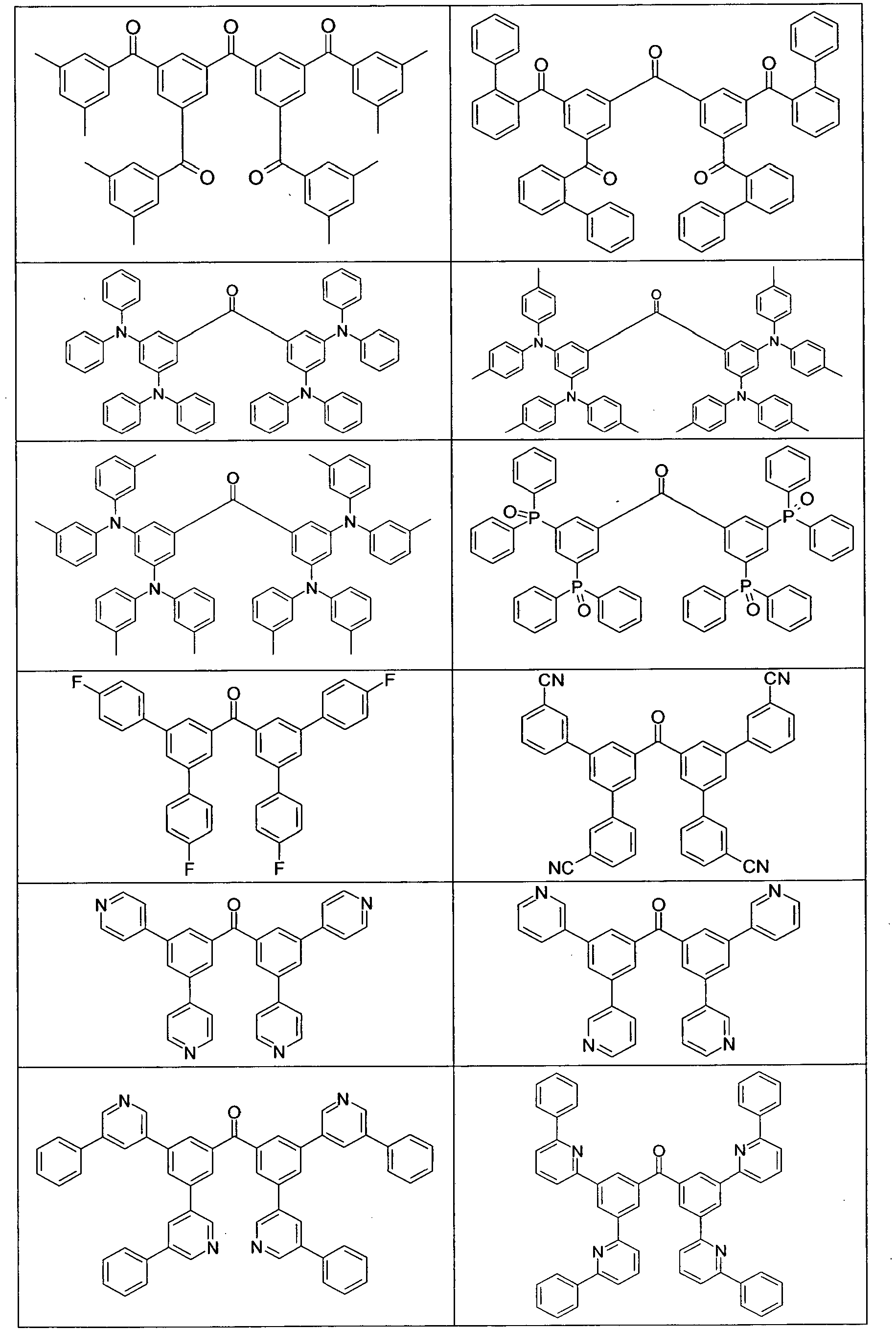

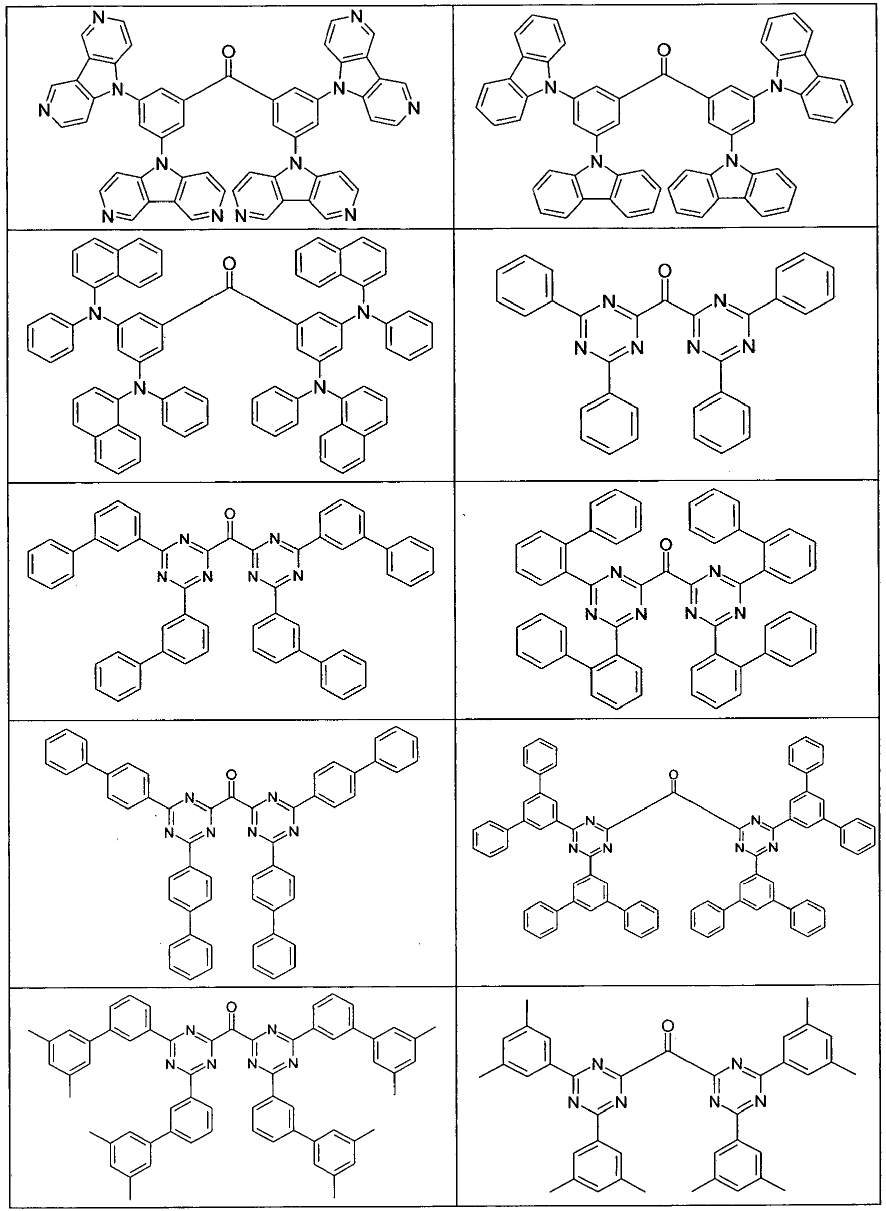

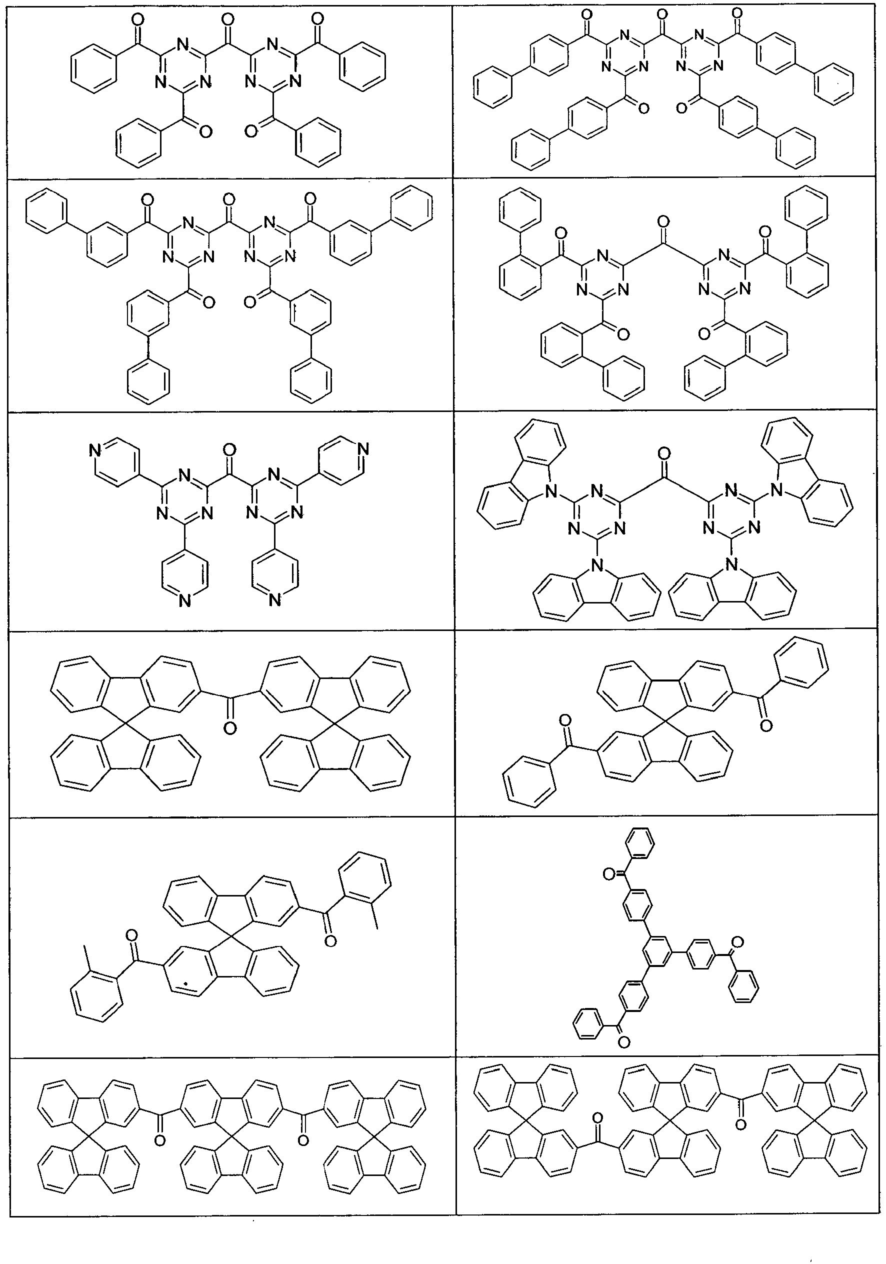

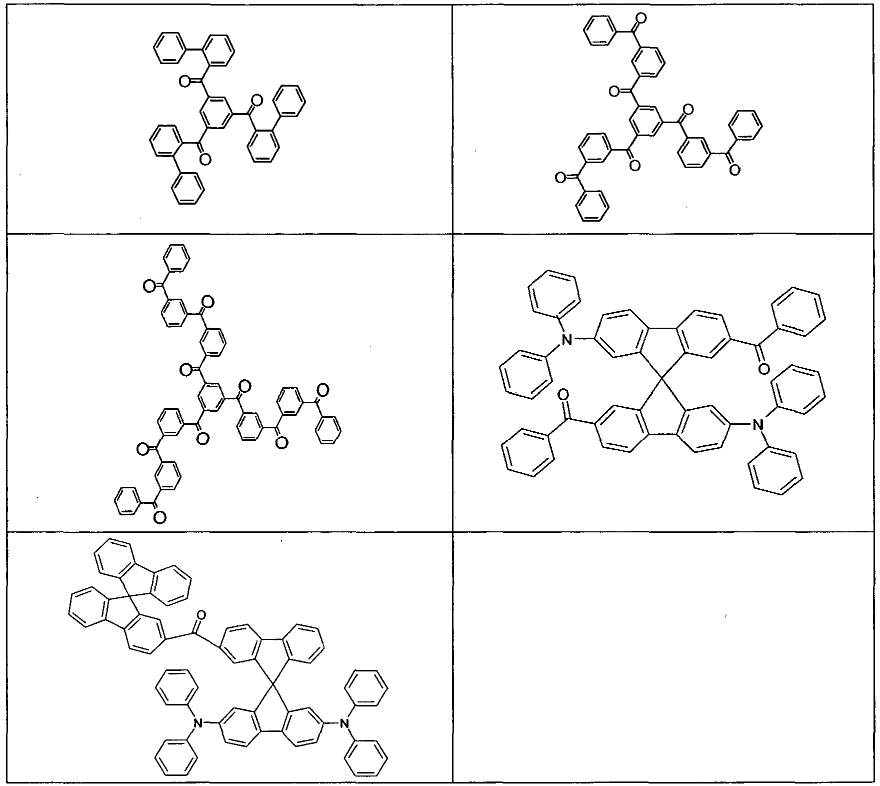

Beispiele für geeignete aromatische Phosphinoxidderivate sind die im Folgenden abgebildeten Verbindungen.

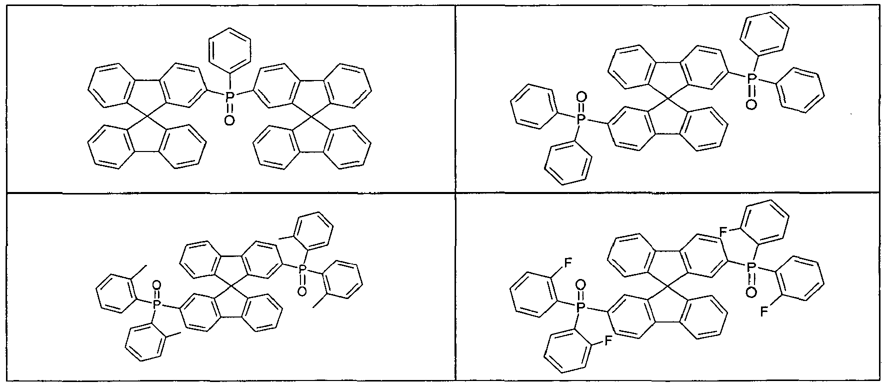

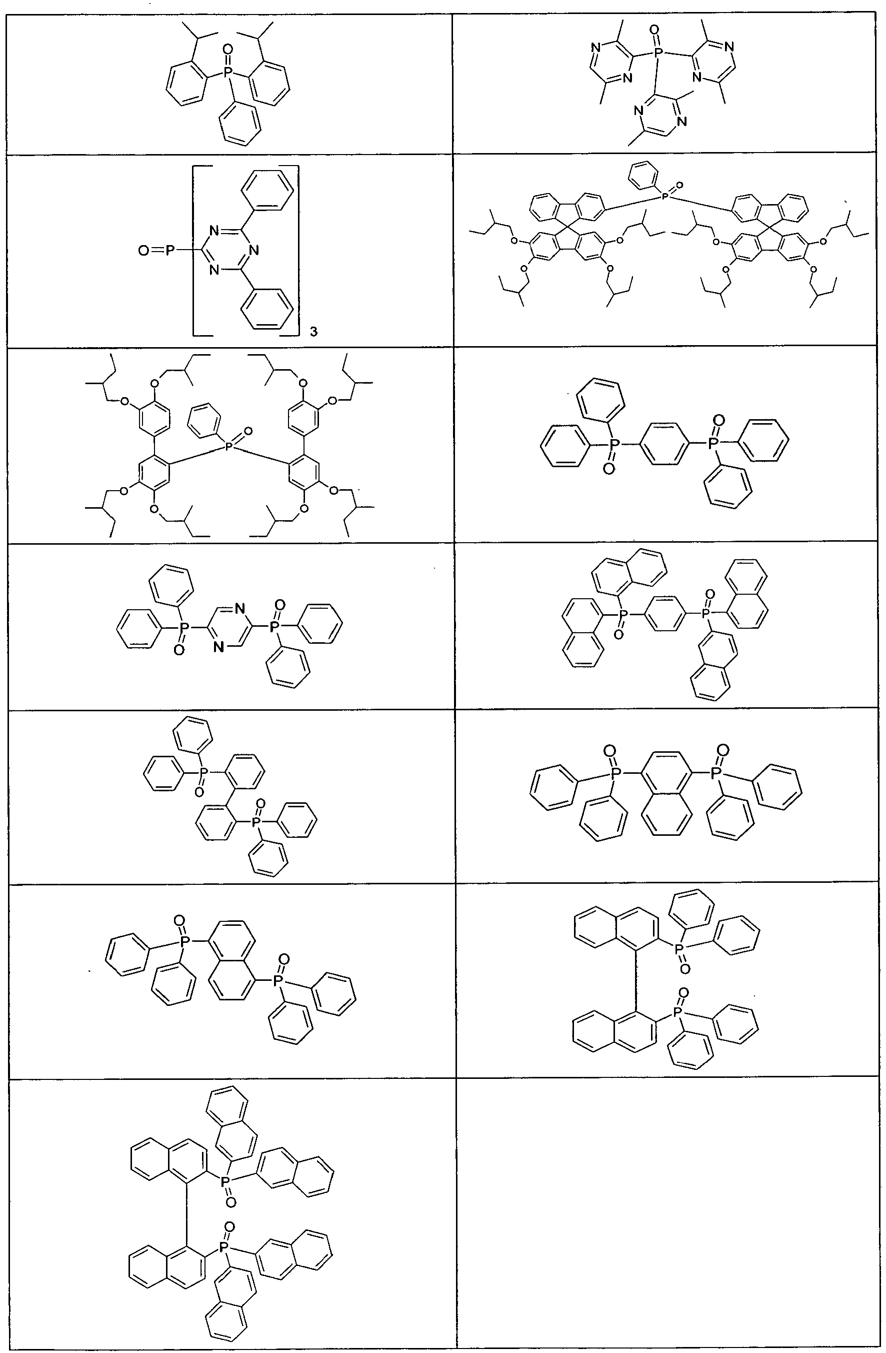

Geeignete Metallkomplexe, die als Matrixmaterial in der erfindungsgemäßen organischen Elektrolumineszenzvorrichtung eingesetzt werden können, sind Be-, Zn- bzw. Al-Komplexe. Geeignet sind beispielsweise die in WO 2009/062578 offenbarten Zn-Komplexe.

Beispiele für geeignete Metallkomplexe sind die in der folgenden Tabelle aufgeführten Komplexe.

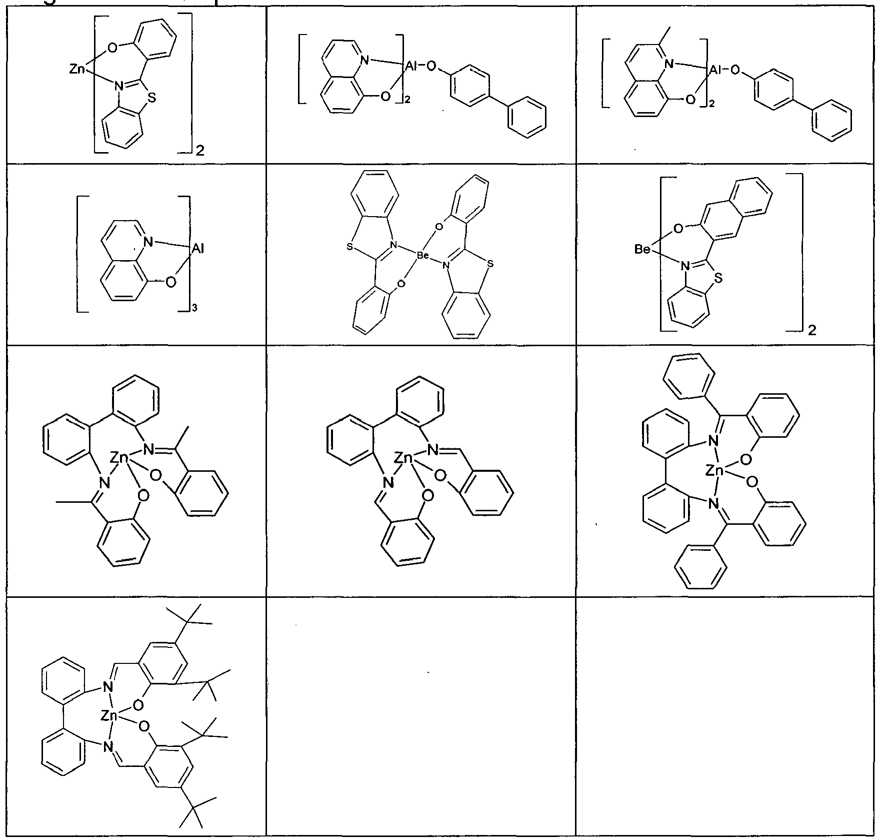

Geeignete Azaphosphole, die als Matrixmaterial in der erfindungsgemäßen organischen Elektrolumineszenzvorrichtung eingesetzt werden können, sind solche Verbindungen, wie sie in WO 2010/054730 offenbart sind.

### Diese Anmeldung ist via Zitat Bestandteil der vorliegenden Erfindung.

Geeignete Azaborole, die als Matrixmaterial in der erfindungsgemäßen organischen Elektrolumineszenzvorrichtung eingesetzt werden können, sind insbesondere Azaborolderivate, welche mit mindestens einem elektronenleitenden Substituenten substituiert sind. Solche Verbindungen sind in der noch nicht offen gelegten Anmeldung EP 11010103.7 offenbart. Geeignete Matrixverbindungen sind weiterhin die Verbindungen der folgenden Formel (M76), 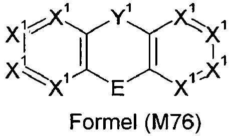 wobei R, Z und E wie oben definiert sind und für die weiteren verwendeten Symbole gilt:
- X¹: ist bei jedem Auftreten gleich oder verschieden CR oder N oder eine Gruppe X¹-X¹ steht für eine Gruppe der folgenden Formel (M77), mit der Maßgabe, dass in der Verbindung der Formel (M76) mindestens eine Gruppe X¹-X¹ für eine Gruppe der Formel (M77) steht und dass pro Cyclus maximal eine Gruppe X¹-X¹ für eine Gruppe der Formel (M77) steht, 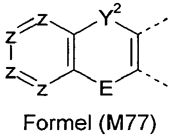 wobei die C-Atome mit den gestrichelten Bindungen die Bindung der Gruppe andeuten;
- Y¹, Y²: ist bei jedem Auftreten gleich oder verschieden ausgewählt aus der Gruppe bestehend aus CR₂, NR, O, S, SiR₂, BR, PR und P(=O)R.

Diese Verbindungen können, je nach genauer Struktur und Substitution, lochtransportierend, elektronentransportierend, bipolar oder weder lochnoch elektronentransportierend sein.

Die Gruppe X¹-X¹ ist hier mit einer Einfachbindung angedeutet. Da die Gruppe X¹-X¹ in der Verbindung der Formel (M77) jedoch in einer aromatischen Gruppe gebunden ist, ist offensichtlich, dass hiermit eine aromatische Bindung gemeint ist, d. h. der Bindungsgrad der Bindung zwischen den beiden Atomen X¹ liegt zwischen 1 und 2. Dabei ist die Gruppe der Formel (M77) in beliebigen Positionen gebunden, und die Gruppen Y¹ und Y² können entweder in cis- oder in trans-Konfiguration zueinander stehen. Die Gruppen X¹, die nicht für eine Gruppe der Formel (M77) stehen, stehen gleich oder verschieden bei jedem Auftreten für CR oder N.

In einer bevorzugten Ausführungsform der Erfindung ist E bei jedem Auftreten gleich oder verschieden ausgewählt aus der Gruppe bestehend aus einer Einfachbindung, CR₂, NR, O oder S. Besonders bevorzugt steht E für eine Einfachbindung.

Bevorzugte Verbindungen der Formel (M76) sind die Verbindungen der folgenden Formeln (M78) bis (M84), 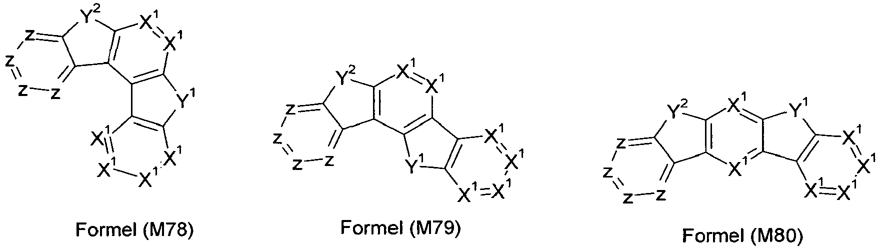 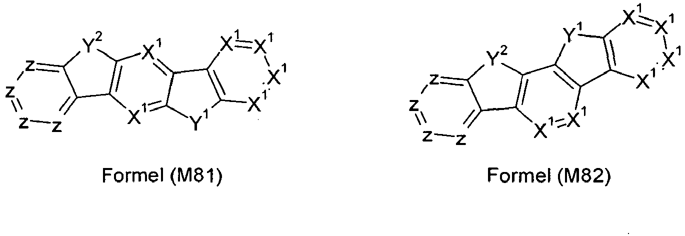 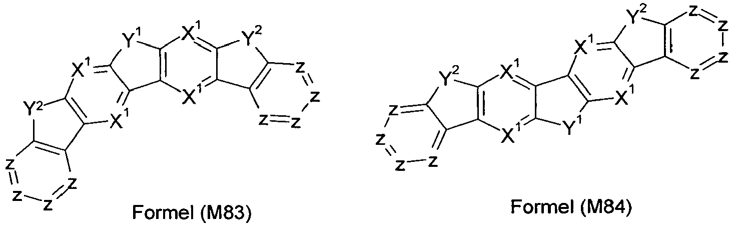 wobei Z, Y¹, Y², R, R¹ und R² die oben genannten Bedeutungen aufweisen und weiterhin gilt:
- X¹: ist bei jedem Auftreten gleich oder verschieden CR oder N.

In bevorzugten Gruppen der oben genannten Formeln (M76) und (M78) bis (M84) stehen maximal zwei Symbole X¹ pro Cyclus für N, besonders bevorzugt steht maximal ein Symbol X¹ pro Cyclus für N. Ganz besonders bevorzugt steht das Symbol X¹ gleich oder verschieden bei jedem Auftreten für CR.

In bevorzugten Gruppen der oben genannten Formeln (M77) bis (M84) stehen maximal zwei Symbole Z pro Cyclus für N, besonders bevorzugt steht maximal ein Symbol Z pro Cyclus für N. Ganz besonders bevorzugt steht das Symbol Z gleich oder verschieden bei jedem Auftreten für CR.

Insbesondere bevorzugt stehen in den Formeln (M78) bis (M84) alle Symbole X¹ und alle Symbole Z gleich oder verschieden für CR.

Bevorzugte Ausführungsformen der Formeln (M78) bis (M84) sind die Verbindungen der folgenden Formeln (M78a) bis (M84a), 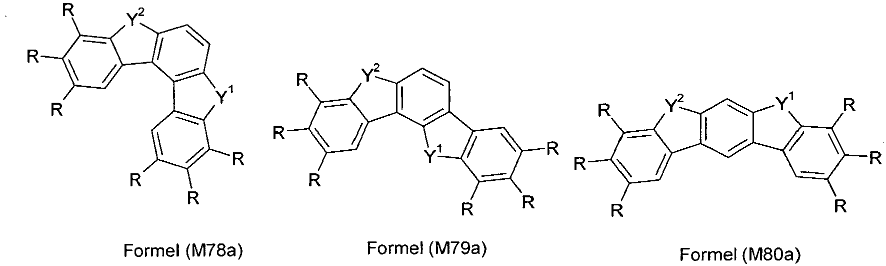 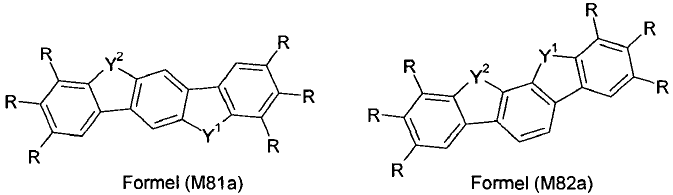 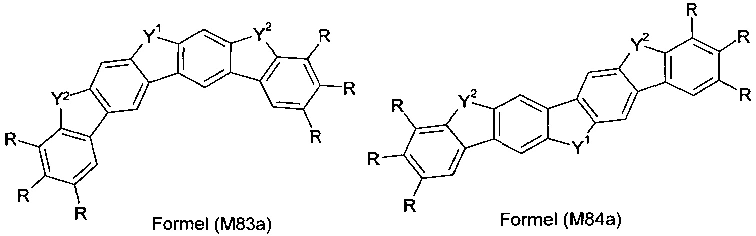 wobei die verwendeten Symbole die oben genannten Bedeutungen aufweisen.

In einer weiteren bevorzugten Ausführungsform der Erfindung sind Y¹ und Y² gleich oder verschieden bei jedem Auftreten ausgewählt aus der Gruppe bestehend aus CR₂, NR, O oder S.

In den Gruppen der Formel (M76) bzw. (M78) bis (M84) bzw. (M78a) bis (M84a) kommen für die Gruppen Y¹ und Y² alle Kombinationen in Frage. Bevorzugt steht mindestens eine Gruppe Y¹ und/oder Y² für ein Heteroatom, d. h. bevorzugt ist mindestens eine der Gruppen Y¹ und/oder Y² von CR₂ verschieden.

Geeignete Kombinationen aus Y¹ und Y² sind die in der folgenden Tabelle aufgeführten Kombinationen.

| Y¹ | Y² |
|---|---|
| CR₂ | CR₂ |
| CR₂ | NR |
| CR₂ | O |
| CR₂ | S |
| NR | CR₂ |
| NR | NR |
| NR | O |
| NR | S |
| O | CR₂ |
| O | NR |
| O | O |
| O | S |
| S | CR₂ |
| S | NR |
| S | O |
| S | S |

Bevorzugt sind Verbindungen der Formel (M76) bzw. (M78) bis (M84) bzw. (M78a) bis (M84a), in denen eine der Gruppen Y¹ bzw. Y² für CR₂ steht und die andere der Gruppen Y¹ bzw. Y² für NR steht oder in denen beide Gruppen Y¹ und Y² für NR stehen oder in denen beide Gruppen Y¹ und Y² für O stehen.

Wenn Y¹ bzw. Y² für NR steht, steht der Substituent R, der an dieses Stickstoffatom gebunden ist, bevorzugt für ein aromatisches oder heteroaromatisches Ringsystem mit 5 bis 24 aromatischen Ringatomen, welches auch durch einen oder mehrere Reste R¹ substituiert sein kann. Dabei enthalten die aromatischen bzw. heteroaromatischen Ringsysteme bevorzugt keine kondensierten Aryl- bzw. Heteroarylgruppen, in denen mehr als zwei aromatische Sechsringe direkt aneinander ankondensiert sind. In einer besonders bevorzugten Ausführungsform steht dieser Substituent R gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in welchen zwei oder mehr aromatische bzw. heteroaromatische 6-Ring-Gruppen direkt aneinander ankondensiert sind, aufweist und welches jeweils auch durch einen oder mehrere Reste R¹ substituiert sein kann. So ist es bevorzugt, wenn R beispielsweise keine Naphthylgruppen oder höhere kondensierte Arylgruppen aufweist und ebenso keine Chinolingruppen, Acridingruppen, etc.. Dagegen ist es möglich, dass R beispielsweise Carbazolgruppen, Dibenzofurangruppen, Fluorengruppen, etc. aufweist, weil in diesen Strukturen keine 6-Ring-Aromaten bzw. -Heteroaromaten direkt aneinander ankondensiert sind. Bevorzugte Substituenten R sind ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Kombinationen aus zwei oder drei dieser Gruppen, welche jeweils mit einem oder mehreren Resten R¹ substituiert sein können. Bevorzugte Biphenyl-, Terphenyl- und Quaterphenylgruppen sind die Strukturen der oben abgebildeten Formeln (Bi-1) bis (Bi-3), (Ter-1) bis (Ter-3) und (Quater-1) bis (Quater-4).

Wenn Y¹ bzw. Y² für CR₂ steht, steht R bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹ substituiert sein kann. Dabei können die Reste R auch ein Ringsystem miteinander bilden und so ein Spirosystem aufspannen. Ganz besonders bevorzugt steht R für eine Methylgruppe oder für eine Phenylgruppe.

In einer bevorzugten Ausführungsform der Erfindung steht mindestens eine der Gruppen Y¹ und Y² für NR, und die entsprechende Gruppe R steht für ein aromatisches oder heteroaromatisches Ringsystem, wie oben beschrieben. In diesem Fall kann es auch bevorzugt sein, wenn alle Substituenten R, die am Grundgerüst der Verbindung gemäß Formel (M76) bzw. den bevorzugten Ausführungsformen gebunden sind, für H stehen.

In einer weiteren bevorzugten Ausführungsform der Verbindung gemäß Formel (M76) oder den oben ausgeführten bevorzugten Ausführungsformen steht mindestens ein Substituent R, der am Grundgerüst der Verbindung gemäß Formel (M76) gebunden ist, für einen Rest ungleich H oder D. Insbesondere ist mindestens einer der in den Formeln (M78a) bis (M84a) explizit eingezeichneten Reste R ungleich H oder D.

Dieser Substituent R ungleich H oder D steht bevorzugt für ein aromatisches oder heteroaromatisches Ringsystem mit 5 bis 24 aromatischen Ringatomen, welches auch durch einen oder mehrere Reste R¹ substituiert sein kann. Dabei enthalten die aromatischen bzw. heteroaromatischen Ringsysteme bevorzugt keine kondensierten Aryl- bzw. Heteroarylgruppen, in denen mehr als zwei aromatische Sechsringe direkt aneinander ankondensiert sind. In einer besonders bevorzugten Ausführungsform steht dieser Substituent R gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in welchen zwei oder mehr aromatische bzw. heteroaromatische 6-Ring-Gruppen direkt aneinander ankondensiert sind, aufweist und welches jeweils auch durch einen oder mehrere Reste R¹ substituiert sein kann. So ist es bevorzugt, wenn R beispielsweise keine Naphthylgruppen oder höhere kondensierte Arylgruppen aufweist und ebenso keine Chinolingruppen, Acridingruppen, etc.. Dagegen ist es möglich, dass R beispielsweise Carbazolgruppen, Dibenzofurangruppen, Fluorengruppen, etc. aufweist, weil in diesen Strukturen keine 6-Ring-Aromaten bzw. -Heteroaromaten direkt aneinander ankondensiert sind. Bevorzugte Substituenten R sind ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Kombinationen aus zwei oder drei dieser Gruppen, welche jeweils mit einem oder mehreren Resten R¹ substituiert sein können. Bevorzugte Biphenyl-, Terphenyl- und Quaterphenylgruppen sind die Strukturen der oben abgebildeten Formeln (Bi-1) bis (Bi-3), (Ter-1) bis (Ter-3) und (Quater-1) bis (Quater-4).

In einer bevorzugten Ausführungsform der Erfindung treten die oben genannten Bevorzugungen gleichzeitig auf. Bevorzugt sind also die Verbindungen der Formeln (M78a) bis (M84a), in denen Y¹ und Y² gleich oder verschieden bei jedem Auftreten ausgewählt sind aus CR₂, NR, O oder S, insbesondere in den oben genannten Kombinationen, und in denen für R die oben genannten Bevorzugungen gelten.

Beispiele für bevorzugte Verbindungen gemäß Formel (M76) sind die in der folgenden Tabelle aufgeführten Verbindungen. Weitere geeignete Verbindungen sind die oben zu den Verbindungen der Formeln (M1) und (M2) aufgeführten Strukturen, die gleichzeitig auch Strukturen der Formel (M76) enthalten.

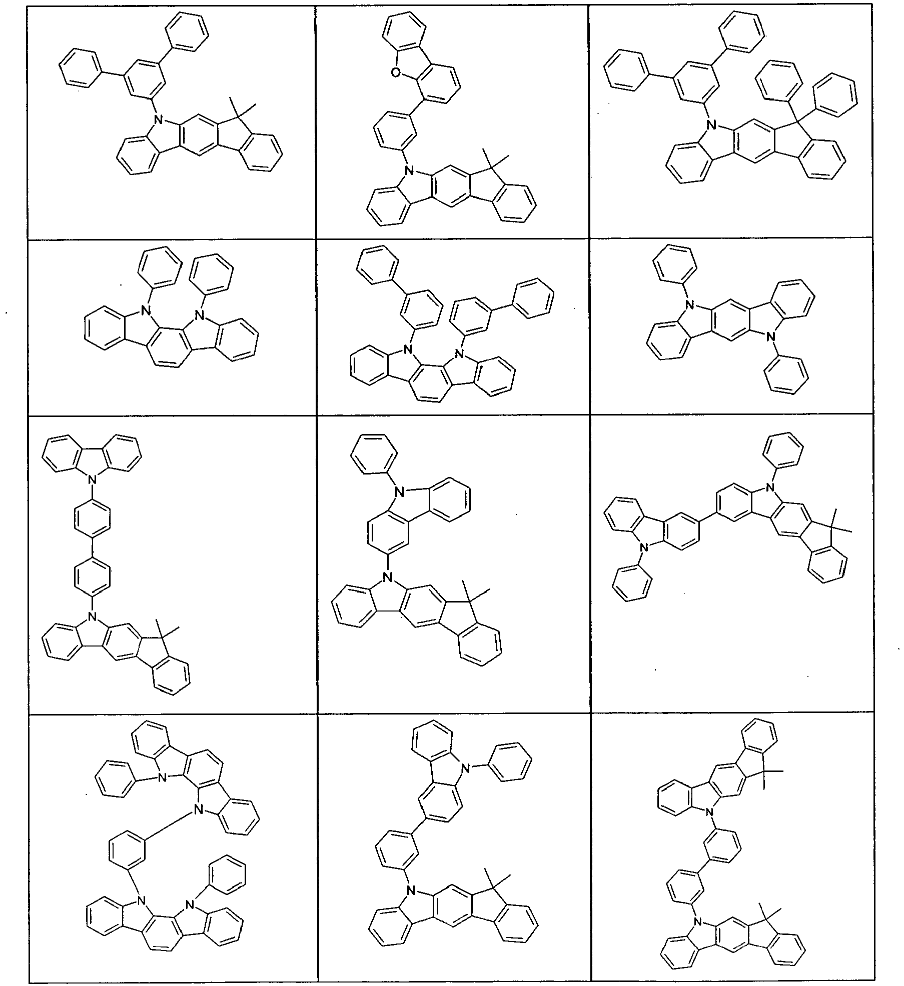

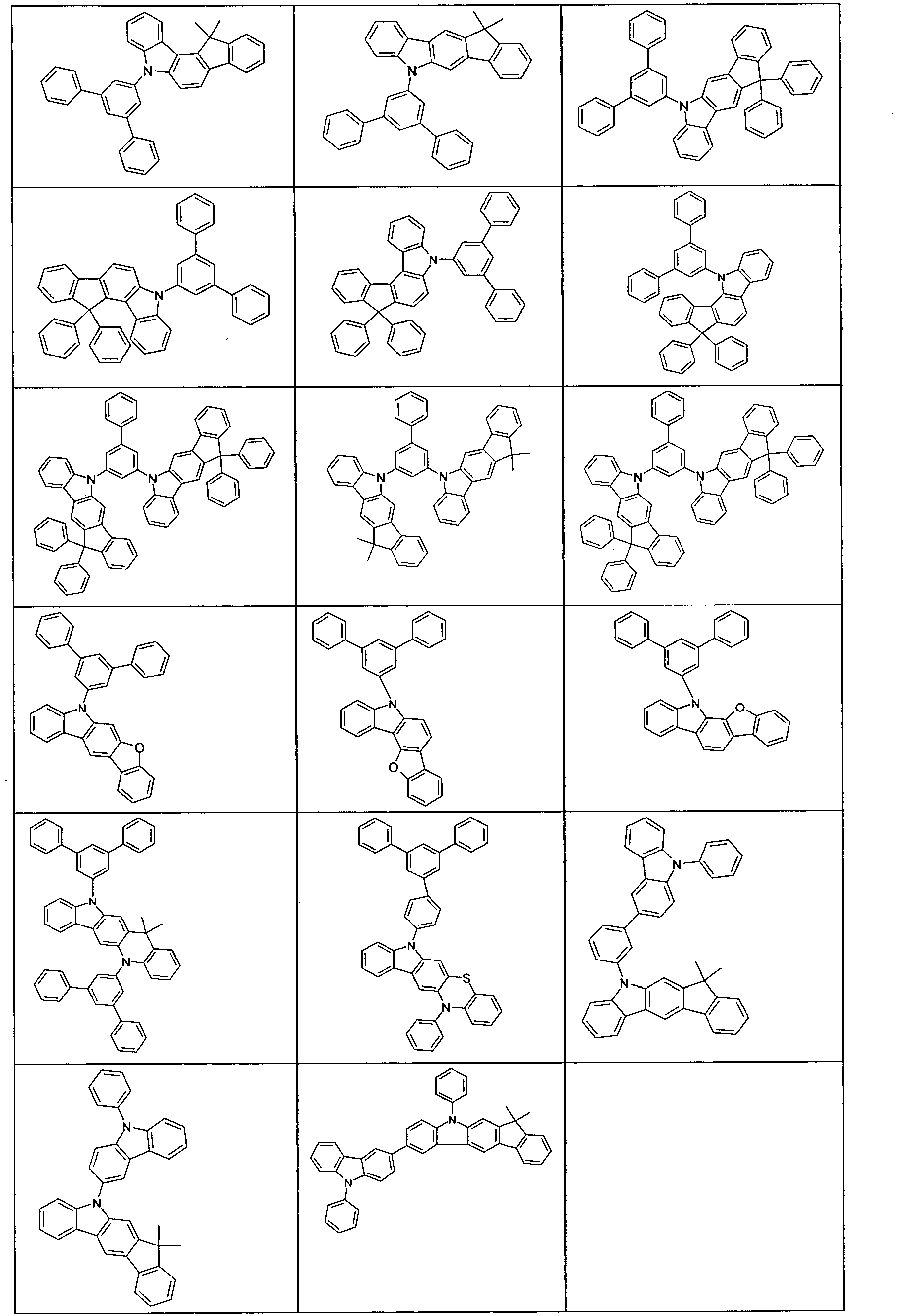

Nochmal weiterhin bevorzugt sind die Matrixverbindungen der folgenden Formeln (M85) bis (M91), 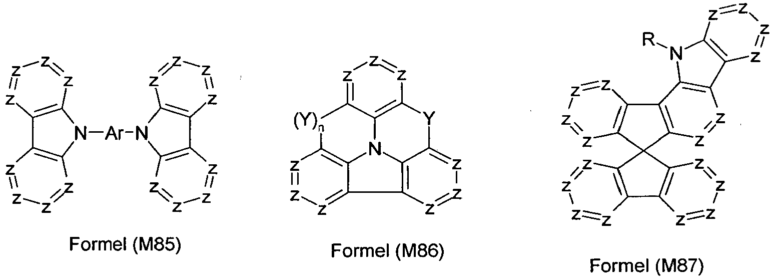 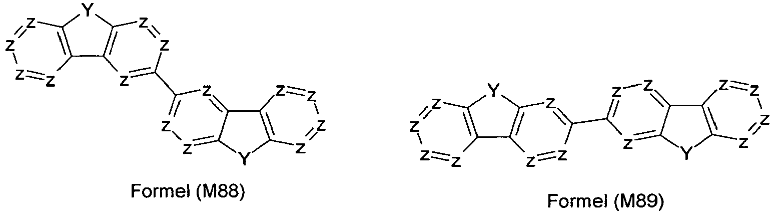 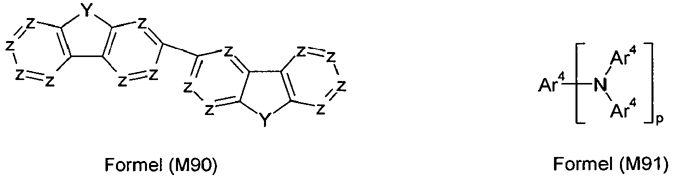 wobei die verwendeten Symbole und Indizes dieselben Bedeutungen aufweisen, wie oben beschrieben, und p für 1, 2, 3, 4, 5 oder 6 steht, insbesondere für 1, 2 oder 3. Weiterhin können in der Struktur der Formel (M91) zwei Gruppen Ar⁴, die an dasselbe Stickstoffatom binden, durch eine Gruppe, ausgewählt aus NR, O, CR₂ oder S, miteinander verbrückt sein.

Dabei sind insbesondere die Verbindungen der Formel (M91) lochtransportierende Verbindungen.

Weiterhin sind bevorzugte Ausführungsformen der Verbindungen gemäß den Formeln (M85) bis (M91) solche Verbindungen, in denen die verwendeten Symbole und Indizes die oben aufgeführten bevorzugten Ausführungsformen aufweisen.

Bevorzugten Ausführungsformen der Verbindungen der Formel (M85) sind die in der folgenden Tabelle aufgeführten Strukturen.

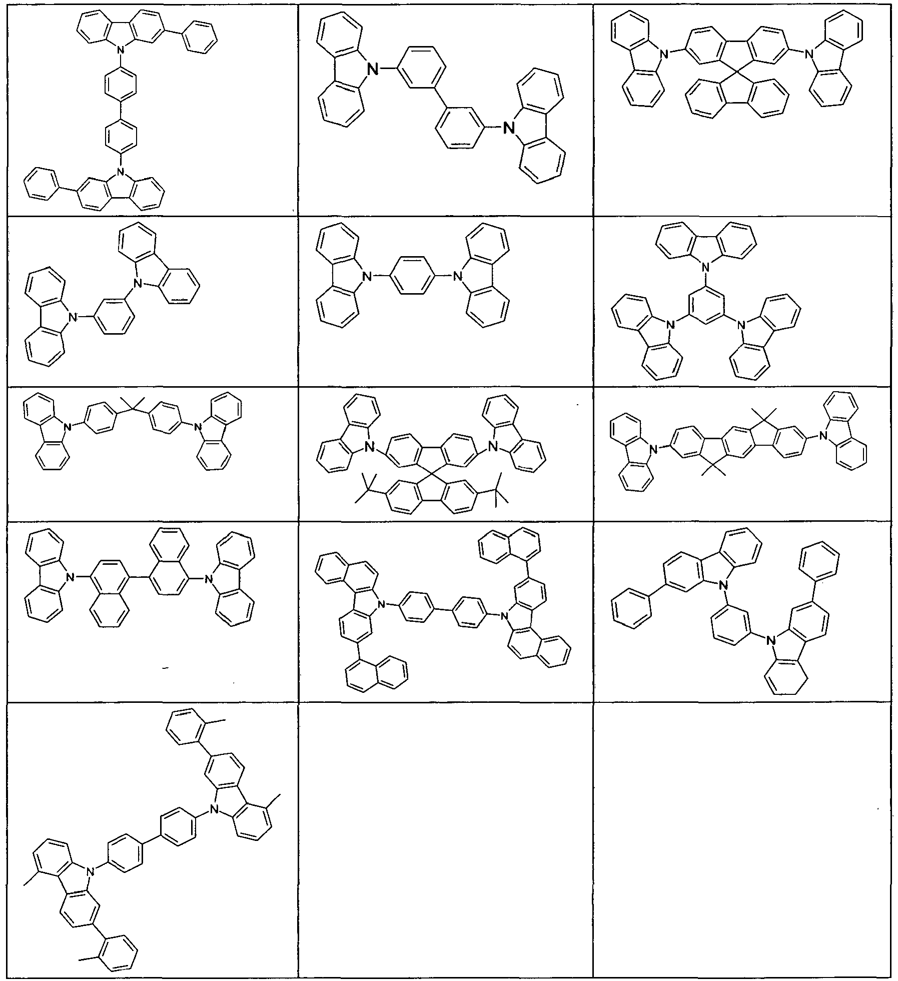

Bevorzugten Ausführungsformen der Verbindungen der Formel (M86) sind die in der folgenden Tabelle aufgeführten Strukturen.

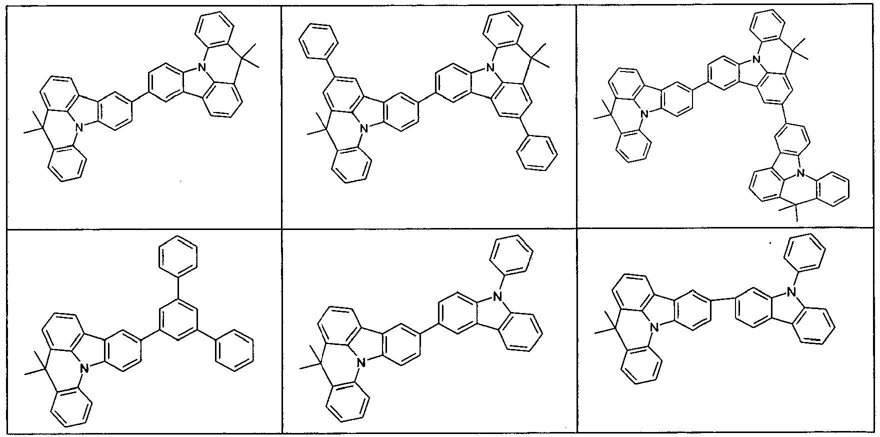

Bevorzugten Ausführungsformen der Verbindungen der Formel (M87) sind die in der folgenden Tabelle aufgeführten Strukturen.

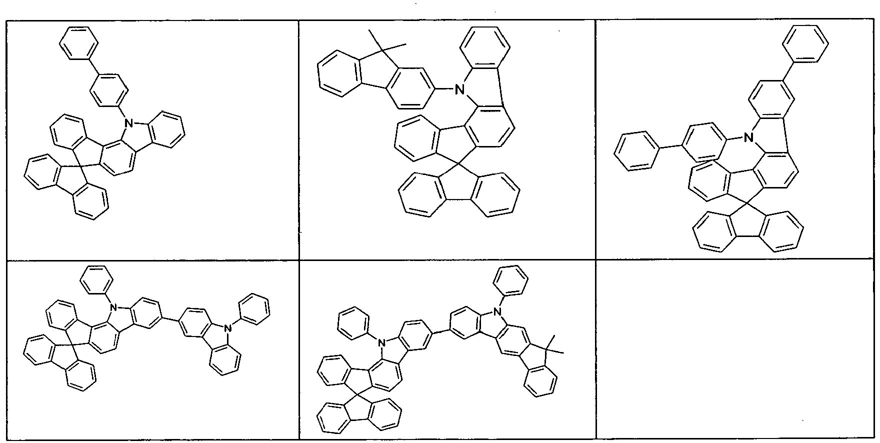

Bevorzugten Ausführungsformen der Verbindungen der Formel (M88) bis (M90) sind die in der folgenden Tabelle aufgeführten Strukturen.

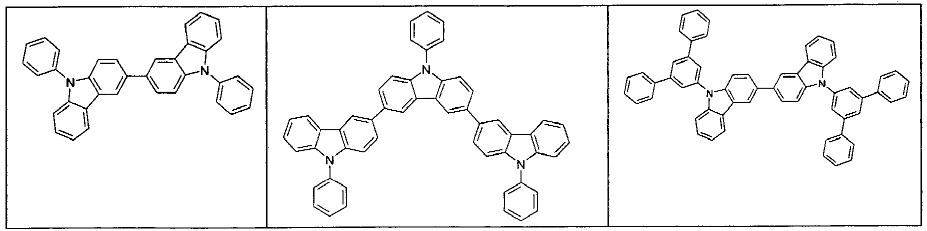

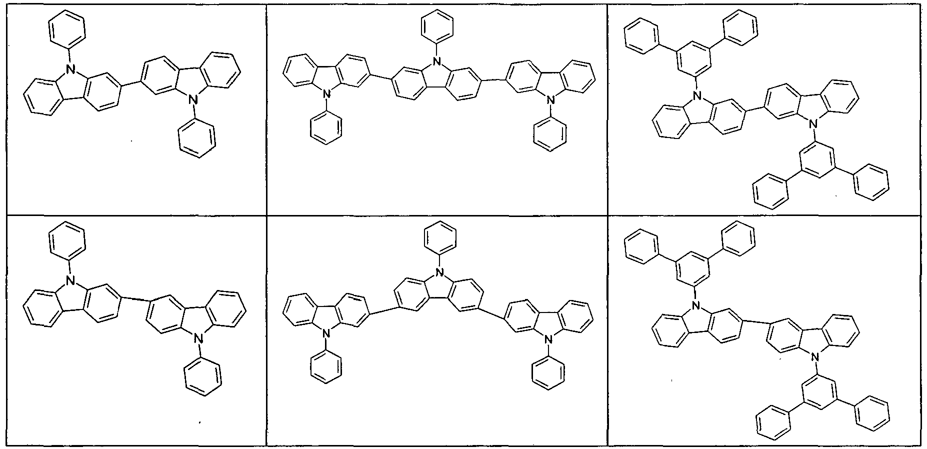

Im Folgenden wird die organische Elektrolumineszenzvorrichtung näher beschrieben.

Die organische Elektrolumineszenzvorrichtung enthält Kathode, Anode und emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Es sei aber darauf hingewiesen, dass nicht notwendigerweise jede dieser Schichten vorhanden sein muss.

In den weiteren Schichten der erfindungsgemäßen organischen Elektrolumineszenzvorrichtung, insbesondere in den Lochinjektions- und -transportschichten und in den Elektroneninjektions- und -transportschichten, können alle Materialien verwendet werden, wie sie üblicherweise gemäß dem Stand der Technik eingesetzt werden. Dabei können die Lochtransportschichten auch p-dotiert bzw. die Elektronentransportschichten auch n-dotiert sein. Dabei wird unter einer p-dotierten Schicht eine Schicht verstanden, in der freie Löcher erzeugt werden und deren Leitfähigkeit dadurch erhöht ist. Eine umfassende Diskussion von dotierten Transportschichten in OLEDs findet sich in Chem. Rev. 2007, 107, 1233. Besonders bevorzugt ist der p-Dotand in der Lage, das Lochtransportmaterial in der Lochtransportschicht zu oxidieren, hat also ein ausreichend hohes Redoxpotential, insbesondere ein höheres Redoxpotential als das Lochtransportmaterial. Als Dotanden sind prinzipiell alle Verbindungen geeignet, welche Elektronenakzeptorverbindungen darstellen und die Leitfähigkeit der organischen Schicht durch Oxidation des Hosts erhöhen können. Der Fachmann kann im Rahmen seines allgemeinen Fachwissens ohne größeren Aufwand geeignete Verbindungen identifizieren. Insbesondere geeignet als Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709 und US 2010/0096600 offenbarten Verbindungen.

Der Fachmann kann daher ohne erfinderisches Zutun alle für organische Elektrolumineszenzvorrichtungen bekannten Materialien in Kombination mit der erfindungsgemäßen emittierenden Schicht einsetzen.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektronen (z. B. Al/Ni/NiOₓ, Al/PtOₓ) bevorzugt sein. Dabei muss mindestens eine der Elektroden transparent oder teiltransparent sein, um die Auskopplung von Licht zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Da die fluoreszierende Verbindung mit hoher sterischer Abschirmung typischerweise durch die abschirmenden Gruppen eine gute Löslichkeit in einer Vielzahl gängiger organischer Lösemittel aufweist, ist die Herstellung der emittierenden Schicht aus Lösung bevorzugt.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Ein weiterer Gegenstand der vorliegenden Erfindung ist daher ein Verfahren zur Herstellung einer erfindungsgemäßen organischen Elektrolumineszenzvorrichtung, dadurch gekennzeichnet, dass mindestens eine Schicht mit einem Sublimationsverfahren aufgebracht wird und/oder dass mindestens eine Schicht mit einem OVPD (Organic Vapour Phase Deposition) Verfahren oder mit Hilfe einer Trägergassublimation aufgebracht wird und/oder dass mindestens eine Schicht aus Lösung, durch Spincoating oder mit einem Druckverfahren aufgebracht wird.

Die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen organischen Elektrolumineszenzvorrichtungen weisen gegenüber Vorrichtungen gemäß dem Stand der Technik, die eine TADF-Verbindung als emittierende Verbindung enthalten, jedoch keine sterisch abgeschirmte fluoreszierende Verbindung enthalten, eine verbesserte Lebensdauer auf.
2. Die erfindungsgemäßen organischen Elektrolumineszenzvorrichtungen weisen gegenüber Vorrichtungen gemäß dem Stand der Technik, die eine TADF-Verbindung als emittierende Verbindung enthalten, jedoch keine sterisch abgeschirmte fluoreszierende Verbindung enthalten, häufig ein schmaleres Emissionsspektrum auf, was zu einer größeren Farbreinheit der Emission führt.
3. Die erfindungsgemäßen organischen Elektrolumineszenzvorrichtungen weisen gegenüber Vorrichtungen gemäß dem Stand der Technik, bei denen die fluoreszierende Verbindung keine hohe sterische Abschirmung aufweist, eine wesentlich höhere Effizienz auf.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße organische Elektrolumineszenzvorrichtungen herstellen.

### Beispiele:

### Synthesebeispiele: Synthese einer sterisch abgeschirmten fluoreszierenden Verbindung

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Synthesebeispiel 1:

### a) Synthese von Verbindung 1

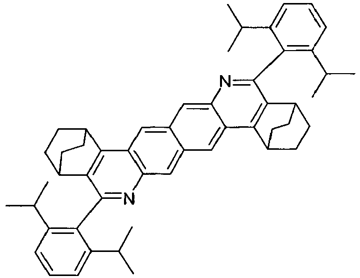

Ein gut gerührtes Gemisch aus 15.8 g (100 mmol) 2,6-Diaminonaphthalin [2243-67-6], 47.6 g (250 mmol) 2,6-Bis(1-methylethyl)-benzaldehyd [179554-06-4], 27.1 g (250 mol) Bicyclo[2.2.2]oct-2-en [931-64-6] und 300 ml Dichlormethan wird tropfenweise mit 2.8 g (20 mmol) Bortrifluoridetherat [60-29-7] versetzt und dann 80 h unter Rückfluss erhitzt. Nach Erkalten wäscht man die Reaktionsmischung zweimal mit je 200 ml Wasser, trocknet die organische Phase über Magnesiumsulfat und entfernt dann das Dichlormethan im Vakuum. Der Rückstand wird in 300 ml o-Dichlorbenzol aufgenommen, mit 87 g (1 mol) Mangandioxid versetzt und 16 h am Wasserabscheider unter Rückfluss erhitzt. Nach Erkalten fügt man 500 ml Ethylacetat zu, saugt vom Mangandioxid über eine Celite-Schicht ab, wäscht das Mangandioxid mit 200 ml Ethylacetat nach und befreit die vereinigten Filtrate im Vakuum von den Lösungsmitteln. Der Rückstand wird an Kieselgel mit n-Heptan / Ethylacetat (2:1) chromatographiert. Ausbeute: 6.4 g (9 mmol) 9 %; Reinheit: ca. 97 %ig nach ¹H-NHR.

### b) Synthese von Verbindung 2

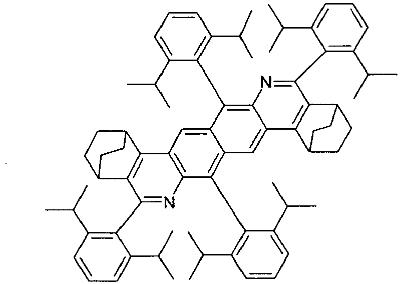

Ein Gemisch aus 6.4 g (9 mmol) Verbindung 1, 4.3 g (22 mmol) 2-Chlor-1,3-bis(1-methylethyl)benzol[54845-36-2], 2.5 g (25 mmol) Natrium-tert-butylat, 44 mg (0.1 mmol) Di-rhodiumtetraacetat [85503-41-3], 52 mg (0.2 mmol) 1,3-Diphenyl-1H-imidazoliumchlorid [26956-10-5], 50 g Glaskugeln (3 mm Durchmesser) und 200 ml o-Xylol wird 48 h unter Rückfluss erhitzt. Nach Erkalten filtriert man von den Salzen über Celite ab, entfernt das Lösungsmittel im Vakuum und chromatographiert den Rückstand an Kieselgel (n-Heptan / Ethylacetat (9:1)) bis eine Reinheit von > 99.8 % nach HPLC erreicht ist. Ausbeute: 1.1 g (1.1 mmol), 12 %.

### Allgemeine Beschreibung der Bestimmung der relevanten Parameter

### 1) Bestimmung der Energieniveaus der Molekülorbitale, Singulett- und Triplettniveau

Die Energieniveaus der Molekülorbitale sowie die Energie des niedrigsten Triplettzustands T₁ bzw. des niedrigsten angeregten Singulettzustands S₁ der Materialien werden über quantenchemische Rechnungen bestimmt. Hierzu wird vorliegend das Programmpaket "Gaussian09, Revision D.01" (Gaussian Inc.) verwendet. Zur Berechnung organischer Substanzen ohne Metalle (mit Methode "org." bezeichnet) wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für den elektronischen Grundzustand und das Triplett-Niveau. Hierbei wird die TDDFT-Methode (time dependent density functional theory) B3PW91 mit dem Basissatz 6-31G(d) (Gaussian-Eingabezeile "# B3PW91/6-31G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31G(d)" verwendet wird (Gaussian-Eingabezeile "#B3PW91/gen pseudo=lanl2 td=(50-50,nstates=4)"). Aus der Energierechnung erhält man z.B. das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Analog erhält man die Energien in Hartree-Einheiten der anderen Energieniveaus wie HOMO-1, HOMO-2,... LUMO+1, LUMO+2 usw.

Im Sinne dieser Anmeldung werden die anhand von CV Messungen kalibrierten Werte ((HEh^{∗}27.212)-0.9899)/1.1206 in eV als Energiniveaus der besetzten Orbitale angesehen.

Im Sinne dieser Anmeldung werden die anhand von CV Messungen kalibrierten Werte ((LEh^{∗}27.212)-2.0041)/1.385 in eV als Energiniveaus der unbesetzten Orbitale angesehen.

Der niedrigste Triplettzustand T₁ eines Materials ist definiert als die relative Anregungsenergie (in eV) des Triplettzustands mit der niedrigsten Energie, der sich aus der quantenchemischen Energierechnung ergibt.

Der niedrigste angeregte Singulettzustand S₁ ist definiert als die relative Anregungsenergie (in eV) des Singulettzustands mit der zweitniedrigsten Energie, der sich aus der quantenchemischen Energierechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian09, Revision D.01" verwendet.

In Tabelle 2 sind die HOMO- und LUMO-Energieniveaus sowie S₁ und T₁ der verschiedenen Materialien angegeben.

### 2) Bestimmung der Photolumineszenz-Quanteneffizienz (PLQE) der TADF Verbindung

Von der Schicht, die ein TADF-Material enthält, wird ein 50 nm dicker Film auf ein Quarzsubstrat aufgebracht. Dieser Film enthält dabei dieselben Materialien in denselben Konzentrationsverhältnissen wie die entsprechende Schicht in der OLED außer der fluoreszenten Verbindung. Enthält z. B. die Schicht in der OLED das Material IC1 zu 85%, das Material D1 zu 10% und das Material SE1 (fluoreszente Verbindung) zu 5%, so enthält der Film zur Bestimmung der PLQE die Materialien IC1 und D1 im Volumenverhältnis 85:10. Zur Herstellung des Films werden die gleichen Herstellungsbedingungen wie zur Herstellung der Emissionsschicht für die OLEDs verwendet.

Von diesem Film wird ein Absorptionsspektrum im Wellenlängenbereich von 350-500 nm gemessen. Hierzu wird das Reflexionsspektrum R(λ) sowie das Transmissionsspektrum T(λ) der Probe unter einem Einfallswinkel von 6° (also nahezu senkrechter Einfall) bestimmt. Als Absorptionsspektrum im Sinne dieser Anmeldung wird A(λ)=1-P(λ)-T(λ) definiert.

Gilt A(λ ≤ 0.3 im Bereich 350-500nm, so wird die zum Maximum des Absorptionsspektrums gehörige Wellenlänge im Bereich 350-500 nm als λ_{exc} definiert. Gilt für irgendeine Wellenlänge A(λ) > 0.3, so wird als λ_{exc} die größte Wellenlänge definiert, bei der A(λ) von einem Wert kleiner 0.3 zu einem Wert größer 0.3 oder von einem Wert größer 0.3 zu einem Wert kleiner 0.3 wechselt.

Zur Bestimmung der PLQE wird ein Messplatz Hamamatsu C9920-02 verwendet. Das Prinzip beruht auf der Anregung der Probe mit Licht definierter Wellenlänge und der Messung der absorbierten und emittierten Strahlung. Die Probe befindet sich während der Messung in einer Ulbrichtkugel ("integrating sphere"). Das Spektrum des Anregungslichts ist in etwa gaußförmig mit einer Halbwertsbreite < 10 nm und Peakwellenlänge λ_{exc} wie oben definiert.

Die PLQE wird nach dem für den genannten Messplatz üblichen Auswerteverfahren bestimmt. Die Messung erfolgt bei Raumtemperatur. Es ist strengstens darauf zu achten, dass die Probe zu keinem Zeitpunkt mit Sauerstoff in Berührung kommt, da die PLQE von Materialien mit kleinem energetischen Abstand zwischen S₁ und T₁ durch Sauerstoff sehr stark reduziert wird (H. Uoyama et al., Nature 2012, Vol. 492, 234).

Die PLQE ist zusammen mit der verwendeten Anregungswellenlänge in den jeweiligen Beispielen angegeben.

### 3) Bestimmung der Peakemissionswellenlänge λₘₐₓ

Zur Bestimmung der Peakemissionswellenlänge der TADF Verbindungen und der fluoreszierenden Verbindung wird das jeweilige Material in Toluol gelöst. Hierbei wird eine Konzentration von 0.5 mg/100 ml verwendet. Die Lösung wird in einem Fluoreszenzspektrometer Hitachi F-4500 mit einer Wellenlänge von 350 nm angeregt. Die Messung erfolgt bei Raumtemperatur. Die Peakemissionswellenlänge λₘₐₓ ist die Wellenlänge, bei der das dabei erhaltenen Emissionsspektrum seinen Maximalwert erreicht.

### 4) Bestimmung der PLQE der fluoreszenten Verbindung

Zur Bestimmung der PLQE der fluoreszenten Verbindung wird das Material in Toluol gelöst. Hierbei wird eine Konzentration von 1 mg/100 ml verwendet. Zur Bestimmung der PLQE wird die Lösung in einem Messplatz Hamamatsu C9920-02 vermessen (Beschreibung siehe oben). Die Messung erfolgt bei Raumtemperatur. Als Anregungswellenlänge wird die Wellenlänge 0.27^{∗}λₘₐₓ+300nm verwendet, wobei λₘₐₓ die Peakemissionswellenlänge der fluoreszenten Verbindung wie oben definiert darstellt.

### 5) Bestimmung der Abklingzeit

Zur Bestimmung der Abklingzeit wird eine Probe verwendet, die wie oben unter "Bestimmung der PL-Quanteneffizienz (PLQE)" beschrieben hergestellt wird. Die Probe wird bei Raumtemperatur durch einen Laserpuls angeregt (Wellenlänge 266 nm, Pulsdauer 1.5 ns, Pulsenergie 200 µJ, Strahldurchmesser 4 mm). Die Probe befindet sich hierbei im Vakuum (<10⁻⁵ mbar). Nach der Anregung (definiert als t = 0) wird der zeitliche Verlauf der Intensität der emittierten Photolumineszenz gemessen. Die Photolumineszenz zeigt am Anfang einen steilen Abfall, der auf die prompte Fluoreszenz der TADF-Verbindung zurückzuführen ist. Im weiteren zeitlichen Verlauf ist ein langsamerer Abfall zu beobachten, die verzögerte Fluoreszenz (siehe z. B. H. Uoyama et al., Nature, vol. 492, no. 7428, 234-238, 2012 sowie K. Masui et al., Organic Electronics, vol. 14, no. 11, pp. 2721-2726, 2013). Die Abklingzeit tₐ im Sinne dieser Anmeldung ist die Abklingzeit der verzögerten Fluoreszenz und wird wie folgt bestimmt: Man wählt einen Zeitpunkt t_{d}, zu dem die prompte Fluoreszenz deutlich unter die Intensität der verzögerten Fluoreszenz abgeklungen ist, so dass die folgende Bestimmung der Abklingzeit nicht von der prompten Fluoreszenz beeinflusst wird. Diese Wahl kann von einem Fachmann durchgeführt werden und gehört zu dessen allgemeinem Fachwissen. Für die Messdaten ab dem Zeitpunkt t_{d} wird die Abklingzeit tₐ = tₑ -t_{d} bestimmt. Dabei ist tₑ derjenige Zeitpunkt nach t = t_{d}, bei dem die Intensität erstmals auf 1/e ihres Wertes bei t = t_{d} abgefallen ist.

### 6) Bestimmung des Abschirmparameters S der fluoreszierenden Verbindung

### a) Bestimmung der aktiven Atome eines Molekülorbitals

Zuerst wird für jedes Molekülorbital einzeln eine Bestimmung durchgeführt, welche Atome aktiv sind. D.h. für jedes Molekülorbital ergibt sich ein im Allgemeinen unterschiedlicher Satz von aktiven Atomen. Im Folgenden wird beispielhaft beschrieben, wie die aktiven Atome des HOMO-Molekülorbitals bestimmt werden. Für alle anderen Molekülorbitale (z. B. HOMO-1, LUMO, LUMO+1 etc.) erfolgt die Bestimmung der aktiven Atome analog.

Für die fluoreszenten Verbindungen wird eine quantenchemische Rechnung wie oben beschrieben durchgeführt. Aus dieser Rechnung ergeben sich die verschiedenen Molekülorbitale, aus denen mit Hilfe der SCPA Population Analysis die Beteiligung aller Atome des Moleküls an den verschiedenen Molekülorbitalen bestimmt wird. Die SCPA Population Analysis, auch C-squared Population Analysis genannt, wird in Ros, P.; Schuit, G. C. A. Theoret. Chim. Acta (Berl.) 1966, 4, 1-12. beschrieben und ist z. B. im Programmpaket AOMix implementiert.

Im Folgenden bezeichnet N die Anzahl der Atome im Molekül, *P'*_{HOMO}(*a*) die Beteiligung des Atoms mit Nummer a (*a* = *1...N*) am HOMO-Molekülorbital (analog für alle anderen Molekülorbitale). Die Beteiligungen sind so definiert, dass gilt ${\sum }_{a = 1}^{N} P {′}_{HOMO} \left(a\right) = 1$ d. h. die Summe aller Beteiligungen ergibt eins. Die auf eins normierten Atombeteiligungen werden mit *P*_{HOMO}(a) bezeichnet, d. h. ${P}_{HOMO} \left(a\right) = P {′}_{HOMO} \left(a\right) / MAX \left(P{′}_{HOMO}\left(a\right),a=1…N\right)$ Gilt ${P}_{HOMO} \left(a\right) \geq 0.2 ,$ so gilt das Atom a als aktiv bezüglich des HOMO-Molekülorbitals. Eine analoge Definition gilt für alle anderen Molekülorbitale.

### b) Ladungstauschende Molekülorbitale der fluoreszenten Verbindung

Als ladungstauschende Molekülorbitale gelten HOMO und LUMO sowie alle Molekülorbitale, die energetisch um 75 meV oder weniger von HOMO oder LUMO entfernt liegen.
Weiterhin gelten alle besetzten Molekülorbitale als aktiv, für deren Energie E gilt: E(LUMO)-E ≤ Gap(TADF). Weiterhin gelten alle unbesetzten Molekülorbitale als aktiv, für deren Energie E gilt: E- E(HOMO) ≤ Gap(TADF). Dabei ist E(LUMO) das LUMO Energieniveau und E(HOMO) das HOMO Energieniveau der fluoreszenten Verbindung. Weiterhin ist Gap(TADF) = E(LUMO,TADF) - E(HOMO,TADF). Dabei ist E(LUMO,TADF) das LUMO Energiniveau der TADF-Verbindung und E(HOMO,TADF) das HOMO Energieniveaus der TADF-Verbindung. Enthält die OLED mehrere TADF-Verbindungen, so ist Gap(TADF) der größte der Werte für die enthaltenen TADF-Verbindungen.

### c) Bestimmung der aktiven Atome der fluoreszenten Verbindung

Ist ein Atom in wenigstens einem ladungstauschenden Molekülorbital aktiv, so gilt es als aktiv für die fluoreszente Verbindung. Nur Atome, die inaktiv (nicht aktiv) in allen ladungstauschenden Molekülorbitalen sind, sind inaktiv für die fluoreszente Verbindung.

### Beispiel: Aktive und inaktive Atome von Rubren

Im Folgenden ist das Material Rubren mit den Nummern *a* der enthaltenen Kohlenstoffatome gezeigt. Die Nummern der Wasserstoffatome sind nicht angegeben, da diese ausnahmslos nicht aktiv sind.

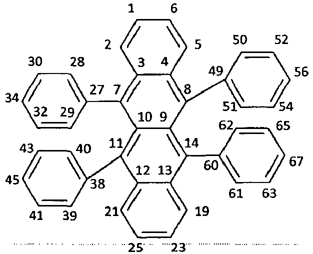

Die folgende Tabelle zeigt die Beteiligungen *P'* sowie die normierten Beteiligungen *P* der Kohlenstoffatome am HOMO bzw. LUMO Molekülorbital, berechnet nach der oben beschriebenen Methode. Die Beteiligungen der Wasserstoffatome sind nicht gezeigt. Weiterhin ist in der letzten Spalte gezeigt, ob die Atome aktiv ("A") bzw. nicht aktiv ("N") sind unter der Annahme, dass nur HOMO und LUMO ladungstauschend sind.

| *a* | *P'*_{HOMO}(*a*) | *P'*_{LUMO}(*a*) | *P*_{HOMO}(*a*) | *P*_{LUMO}(*a*) | aktiv/nicht aktiv |
|---|---|---|---|---|---|
| 1 | 0.035 | 0.034 | 0.31 | 0.29 | A |
| 2 | 0.042 | 0.040 | 0.38 | 0.33 | A |
| 3 | 0.020 | 0.025 | 0.18 | 0.20 | A |
| 4 | 0.020 | 0.025 | 0.18 | 0.20 | A |
| 5 | 0.042 | 0.040 | 0.38 | 0.33 | A |
| 6 | 0.035 | 0.034 | 0.31 | 0.29 | A |
| 7 | 0.112 | 0.120 | 1.00 | 1.00 | A |
| 8 | 0.112 | 0.120 | 1.00 | 1.00 | A |
| 9 | 0.004 | 0.002 | 0.04 | 0.01 | N |
| 10 | 0.004 | 0.002 | 0.04 | 0.01 | N |
| 11 | 0.112 | 0.120 | 1.00 | 1.00 | A |
| 12 | 0.020 | 0.025 | 0.18 | 0.20 | A |
| 13 | 0.020 | 0.025 | 0.18 | 0.20 | A |
| 14 | 0.112 | 0.120 | 1.00 | 1.00 | A |
| 19 | 0.042 | 0.040 | 0.38 | 0.33 | A |
| 21 | 0.042 | 0.040 | 0.38 | 0.33 | A |
| 23 | 0.035 | 0.034 | 0.31 | 0.29 | A |
| 25 | 0.035 | 0.034 | 0.31 | 0.29 | A |
| 27 | 0.003 | 0.003 | 0.03 | 0.02 | N |
| 28 | 0.012 | 0.007 | 0.10 | 0.06 | N |
| 29 | 0.012 | 0.009 | 0.10 | 0.07 | N |
| 30 | 0.004 | 0.002 | 0.03 | 0.01 | N |
| 32 | 0.002 | 0.004 | 0.02 | 0.03 | N |
| 34 | 0.004 | 0.006 | 0.04 | 0.05 | N |
| 38 | 0.003 | 0.003 | 0.03 | 0.02 | N |
| 39 | 0.012 | 0.007 | 0.10 | 0.06 | N |
| 40 | 0.012 | 0.009 | 0.10 | 0.07 | N |
| 41 | 0.004 | 0.002 | 0.03 | 0.01 | N |
| 43 | 0.002 | 0.004 | 0.02 | 0.03 | N |
| 45 | 0.004 | 0.006 | 0.04 | 0.05 | N |
| 49 | 0.003 | 0.003 | 0.03 | 0.02 | N |
| 50 | 0.012 | 0.007 | 0.10 | 0.06 | N |
| 51 | 0.012 | 0.009 | 0.10 | 0.07 | N |
| 52 | 0.004 | 0.002 | 0.03 | 0.01 | N |
| 54 | 0.002 | 0.004 | 0.02 | 0.03 | N |
| 56 | 0.004 | 0.006 | 0.04 | 0.05 | N |
| 60 | 0.003 | 0.003 | 0.03 | 0.02 | N |
| 61 | 0.012 | 0.007 | 0.10 | 0.06 | N |
| 62 | 0.012 | 0.009 | 0.10 | 0.07 | N |
| 63 | 0.004 | 0.002 | 0.03 | 0.01 | N |
| 65 | 0.002 | 0.004 | 0.02 | 0.03 | N |
| 67 | 0.004 | 0.006 | 0.04 | 0.05 | N |

### d) Bestimmung von V(d_{cut})

Die aktive Oberfläche bezeichnet die Van-der-Waals Oberfläche der aktiven Atome der fluoreszenten Verbindung. Es handelt sich dabei um die Oberfläche des Van-der-Waals Volumens der aktiven Atome. Legt man um jedes aktive Atom eine Kugel mit dem zur jeweiligen Atomsorte gehörenden Van-der-Waals Radius, wobei das Atom den Mittelpunkt der Kugel bildet, so ist die Vereinigungsmenge all dieser Kugeln das Van-der-Waals Volumen der aktiven Atome. Die Van-der-Waals Radien r_{VDW} der verschiedenen Elemente sind in der folgenden Tabelle in Angström angegeben.

| Element | H | He | Li | Be | B | C | N | O | F | Ne | Na | Mg | Al |
|---|---|---|---|---|---|---|---|---|---|---|---|---|---|
| r_{VDW} (Å) | 1.2 | 1.22 | 1.52 | 1.7 | 2.08 | 1.85 | 1.54 | 1.4 | 1.35 | 1.6 | 2.31 | 1.73 | 2.05 |
| Element | Si | P | S | Cl | Ar | K | Ca | Sc | Ti | V | Cr | Mn | Fe |
| r_{VDW} (Å) | 2 | 1.9 | 1.85 | 1.81 | 1.91 | 2.31 | 1.97 | 1.7 | 1.7 | 1.7 | 1.7 | 1.7 | 1.7 |
| Element | Co | Ni | Cu | Zn | Ga | Ge | As | Se | Br | Kr | Rb | Sr | Y |
| r_{VDW} (Å) | 1.7 | 1.7 | 1.7 | 1.7 | 1.7 | 1.7 | 2 | 2 | 2.1 | 2.1 | 1.7 | 1.7 | 1.7 |
| Element | Zr | Nb | Mo | Tc | Ru | Rh | Pd | Ag | Cd | In | Sn | Sb | Te |
| r_{VDW} (Å) | 1.7 | 1.7 | 1.7 | 1.7 | 1.7 | 1.7 | 1.7 | 1.7 | 1.7 | 1.7 | 1.7 | 2.2 | 2.2 |

| Element | I | Xe | Cs | Ba | La | Ce | Pr | Nd | Pm | Sm | Eu | Gd | Tb |
|---|---|---|---|---|---|---|---|---|---|---|---|---|---|
| r_{VDW} (Å) | 2.15 | 2.16 | 1.7 | 1.7 | 1.7 | 1.7 | 1.7 | 1.7 | 1.7 | 1.7 | 1.7 | 1.7 | 1.7 |
| Element | Dy | Ho | Er | Tm | Yb | Lu | Hf | Ta | W | Re | Os | Ir | Pt |
| r_{VDW} (Å) | 1.7 | 1.7 | 1.7 | 1.7 | 1.7 | 1.7 | 1.7 | 1.7 | 1.7 | 1.7 | 1.7 | 1.7 | 1.72 |
| Element | Au | Hg | Tl | Pb | Bi | Po | At | Rn | Fr | Ra | Ac | Th | Pa |
| r_{VDW} (Å) | 1.66 | 1.55 | 1.7 | 1.7 | 1.7 | 1.7 | 1.7 | 1.7 | 1.7 | 1.7 | 1.7 | 1.7 | 1.7 |
| Element | u | Np | Pu | Am | Cm | Bk | Cf | | | | | | |
| r_{VDW} (Å) | 1.7 | 1.7 | 1.7 | 1.7 | 1.7 | 1.7 | 1.7 | | | | | | |

Die Connolly-Oberfläche (auch als solvent-excluded surface bezeichnet) der gesamten fluoreszenten Verbindung bezeichnet die Oberfläche des solvent-excluded volume (Micheal L. Connolly, "Computation of Molecular Volume", J. Am. Chem. Soc., 1985, Vol. 107, p. 1118-1124). Betrachtet man das Van-der-Waals Volumen der gesamten fluoreszenten Verbindung (definiert analog zum oben beschriebenen Van-der-Waals Volumen der aktiven Atome) als hart, d.h. als Volumen, das nicht durchdrungen werden kann, so ist das solvent-excluded volume im Sinne der vorliegenden Erfindung der Teil des Raums, der von einer harten Kugel mit Radius 0.4 nm nicht eingenommen werden kann. Die Fläche der Connolly-Oberfläche der gesamten fluoreszente Verbindung wird mit *A*_{con} bezeichnet.

Die Fläche desjenigen Anteils der Connolly-Oberfläche, welcher einen Abstand kleiner oder gleich *d*_{cut} von der aktiven Oberfläche hat, wird mit *A*_{cut} bezeichnet. Für einen gegebenen Wert von *d*_{cut} wird der Parameter *V*(*d*_{cut}) = *A*_{cut}/*A*_{con} definiert.

### e) Bestimmung des Abschirmparameters S für die fluoreszente Verbindung

Der Parameter *S*, der die sterische Abschirmung der fluoreszenten Verbindungen dieser Erfindung beschreibt, ist definiert als $S = \left(V\left(0.2 nm\right)/2+V\left(0.3 nm\right)/3+V\left(0.4 nm\right)/4+V\left(0.5 nm\right)/5+V\left(0.6 nm\right)/6\right) ⋅ \left(20/29\right)$

Der Parameter S hängt von der Struktur der fluoreszenten Verbindung sowie von der verwendeten TADF Verbindung ab (siehe "Ladungstauschende Molekülorbitale der fluoreszenten Verbindung" weiter oben).

Eine fluoreszierende Verbindung gilt als sterisch abgeschirmt im Sinne der vorliegenden Anmeldung, wenn der oben definierte Abschirmparameter *S* ≤ 0.6 ist.

### Beispiele für organische Elektrolumineszenzvorrichtungen

### Herstellung der OLEDs

Glasplättchen, die mit strukturiertem ITO (Indium Zinn Oxid) der Dicke 50 nm beschichtet sind, werden nass gereinigt (Spülmaschine, Reiniger Merck Extran), anschließend 15 min lang bei 250 °C in einer Stickstoffatmosphäre ausgeheizt und vor der Beschichtung 130 s lang mit einem Sauerstoffplasma behandelt. Diese mit Plasma behandelten Glasplättchen bilden die Substrate, auf welche die OLEDs aufgebracht werden. Die Substrate verbleiben vor der Beschichtung im Vakuum. Die Beschichtung beginnt spätestens 10 min nach der Plasmabehandlung. Nach der Herstellung werden die OLEDs zum Schutz gegen Sauerstoff und Wasserdampf verkapselt. Der genaue Schichtaufbau der OLEDs ist den Beispielen zu entnehmen. Die zur Herstellung der OLEDs benötigten Materialien sind in Tabelle 1 gezeigt.

Alle Materialien werden in einer Vakuumkammer thermisch aufgedampft. Dabei besteht die Emissionsschicht bzw. die Emissionsschichten immer aus mindestens einem Matrixmaterial (Hostmaterial, Wirtsmaterial) dem emittierenden Material. Dieses wird dem Matrixmaterial bzw. den Matrixmaterialien durch Coverdampfung in einem bestimmten Volumenanteil beigemischt. Eine Angabe wie IC1:D1:SE1 (92%:5%:3%) bedeutet hierbei, dass das Material IC1 in einem Volumenanteil von 92%, D1 in einem Anteil von 5% und SE1 in einem Anteil von 3% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren bei 1000 cd/m² sowie Spannungs-Strom-Leuchtdichte (UIL) Kennlinien gemessen, woraus sich unter Annahme einer lambertschen Abstrahlcharakteristik die externe Quanteneffizienz (EQE, gemessen in Prozent) bestimmen lässt. Die Angabe U1000 bezeichnet die Spannung, die für eine Leuchtdichte von 1000 cd/m² benötigt wird. EQE1000 bezeichnet die externe Quanteneffizienz bei einer Betriebsleuchtdichte von 1000 cd/m².

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von j0 = 10mA/cm², L1 = 80% bedeutet, dass die Leuchtdichte bei Betrieb mit 10mA/cm² nach der Zeit LD auf 80% ihres Anfangswertes absinkt.

Als TADF Material wird die Verbindung D1 eingesetzt. Diese weist einen energetischen Abstand zwischen S₁ und T₁ von 0.09 eV auf.

Für die beiden Materialien Rubren und SE1 sind in Verbindung mit dem Material D1 die HOMO und LUMO Molekülorbitale ladungstauschend. Der Abschirmparameter beträgt S = 0.755 für Rubren und 0.518 für SE1.

Für das Emissionsmaximum der Verbindung D1 gilt λₘₐₓ = 511 nm, für Rubren ist λₘₐₓ = 555 nm, für SE1 ist λₘₐₓ = 572 nm.

### Beispiel 1: Fluoreszente Verbindungen mit unterschiedlichem Abschirmparameter S in der Emissionsschicht enthaltend eine TADF-Verbindung

Die OLEDs bestehen aus folgender Schichtabfolge, die nach der Plasmabehandlung auf das Substrat aufgebracht wird: 85 nm SpMA1, 15 nm IC1:D1:Emitter (92%:5%:3%), 10 nm IC1, 45 nm ST2:LiQ (50%:50%), Aluminium (100 nm).

Die PLQE der Schicht, welche die TADF Verbindung enthält, beträgt 94% (λ_{exc} = 350 nm), die Abklingzeit 5.4 µs (t_{d} = 7 µs).

Verwendet man Rubren als Emitter (*S* = 0.755), so erhält man EQE1000 = 4.6 %, U1000 = 4.6 V und LD = 220 h für j0 = 10 mA/cm², L1 = 85 %. Mit dem Emitter SE1 (*S* = 0.518) erhält man deutlich bessere Werte von EQE1000 = 8.2 %, U1000 = 4.4 V und LD = 440 h für j0 = 10 mA/cm², L1 = 85 %. In beiden Fällen erhält man orange Emission.

Für eine ansonsten gleich aufgebaute OLED, die in der Emissionsschicht keine fluoreszente Verbindung enthält, also nur IC1 und D1 im Volumenverhältnis 92:5 erhält man eine Lebensdauer LD = 37 h für j0 = 10 mA/cm², L1 = 85 %.

Die PLQE von Rubren beträgt 94%, Anregungswellenlänge 450nm. Die PLQE von SE1 beträgt 97%, Anregungswellenlänge 454nm.

### Beispiel 1a: Höhere Konzentration der fluoreszenten Verbindung

Die OLEDs werden wie in Beispiel 1 hergestellt, wobei für die 15nm dicke Schicht IC1:D1:Emitter (92%:5%:3%) eine Schicht IC1:D1:SE1 (89%:5%:6%) verwendet wird. Die Lebensdauer nimmt im Vergleich zu Beispiel 1 deutlich zu, man erhält LD = 1120 h für j0 = 10 mA/cm², L1 = 85%. Weiterhin verbessert sich die Farbreinheit. Während in Beispiel 1 die Restemission der TADF Verbindung bei 500 nm 9% der Peakemission (bei 567nm) beträgt, reduziert sich diese für 6% SE1 auf nur noch 3% der Peakemission (bei 571nm).

### Beispiel 1b: Höhere Konzentration der TADF Verbindung

Die OLEDs werden wie in Beispiel 1 hergestellt, wobei für die 15nm dicke Schicht IC1:D1:Emitter (92%:5%:3%) eine Schicht IC1:D1:SE1 (87%:10%:3%) verwendet wird. Die Lebensdauer nimmt im Vergleich zu Beispiel 1 deutlich zu, man erhält LD = 655 h für j0 = 10 mA/cm², L1 = 85%.

Die PLQE der Schicht, welche die TADF Verbindung enthält beträgt für dieses Beispiel 87% (λ_{exc} = 350 nm), die Abklingzeit 4.9 µs (t_{d} = 7 µs).

### Beispiel 1c: Höhere Konzentration der fluoreszierenden und der TADF Verbindung

Die OLEDs werden wie in Beispiel 1 hergestellt, wobei für die 15nm dicke Schicht IC1:D1:Emitter (92%:5%:3%) eine Schicht IC1:D1:SE1 (84%:10%:6%) verwendet wird. Die Lebensdauer nimmt im Vergleich zu Beispiel 1 deutlich zu, man erhält LD = 1605 h für j0 = 10 mA/cm², L1 = 85%. Die Farbreinheit gegenüber Beispiel 1 verbessert sich in gleichem Maße wie in Beispiel 1a beschrieben.

### Beispiel 1d: Verwendung einer anderen Elektronentransportschicht

Die OLEDs werden wie in Beispiel 1 hergestellt mit dem Unterschied, dass die 45 nm dicke Schicht ST2:LiQ (50%:50%) durch die Abfolge 45nm ST2, 3nm LiQ ersetzt wird.

Verwendet man Rubren als Emitter (S = 0.755), so erhält man EQE1000 = 4.8 %, U1000 = 4.6 V und LD = 115 h für j0 = 10 mA/cm², L1 = 85 %. Mit dem Emitter SE1 (S = 0.518) erhält man deutlich bessere Werte von EQE1000 = 8.4 %, U1000 = 3.6 V und LD = 150 h für j0 = 10 mA/cm², L1 = 85 %. In beiden Fällen erhält man orange Emission.

### Beispiel 2: Vergleich mit einer OLED enthaltend eine Anthracen-Matrix

Die OLEDs werden mit der fluoreszenten Verbindung SE1 wie in Beispiel 1 hergestellt, mit dem Unterschied, dass die 15 nm dicke Schicht IC1:D1:SE1 (92%:5%:3%) durch eine 30 nm dicke Schicht AM1:SE1 (97%:3%) ersetzt werden. Die Schichtdicke der Emissionsschicht ist für das verwendete Matrixmaterial AM1 optimiert und besser als bei Verwendung einer 15 nm dicken Emissionsschicht. Anthracenhaltige Materialen wie AM1 sind im Stand der Technik sehr häufig verwendete Matrixmaterialien für fluoreszente Verbindungen, wie z.B. das Material SE1. Trotzdem erhält man mit U1000 = 4.9 V, EQE1000 = 4.8 % deutlich schlechtere Werte als in Beispiel 1.

### Beispiel 3: Fluoreszente Verbindungen mit unterschiedlichem Abschirmparameter S in einer Schicht, die an die Schicht angrenzt die das TADF-Material enthält

Die OLEDs werden wie in Beispiel 1 hergestellt mit dem Unterschied, dass die 15 nm dicke Schicht IC1:D1:Emitter (92%:5%:3%) durch die Abfolge 7.5 nm IC1:D1 (95%:5%), 7.5 nm IC1:Emitter (97%:3%), also zwei aneinander angrenzende Schichten, ersetzt wird.

Die PLQE der Schicht, welche die TADF Verbindung enthält beträgt 92% (λ_{exc} = 350 nm), die Abklingzeit 5.4 µs (t_{d} = 7 µs).

Verwendet man Rubren als Emitter (S = 0.755), so erhält man EQE1000 = 8.8%, U1000 = 3.8 V und LD = 130 h für j0 = 10 mA/cm², L1 = 80%. Mit dem Emitter SE1 (S = 0.518) ergibt sich eine deutlich bessere Effizienz, man erhält EQE1000 = 14.2%, U1000 = 3.7 V und LD = 135 h für j0 = 10 mA/cm², L1 = 80%.

### Beispiel 4:

Die OLEDs werden wie in Beispiel 3 hergestellt mit dem Unterschied, dass die 85 nm SpMA1 durch 75 nm SpMA1 und 10 nm SpMA2 ersetzt werden.

Verwendet man Rubren als Emitter (S = 0.755), so erhält man EQE1000 = 8.0%, U1000 = 3.8 V und LD = 150 h für j0 = 10 mA/cm², L1 = 80%. Mit dem Emitter SE1 (S = 0.518) ergibt sich eine deutlich bessere Effizienz, man erhält EQE1000 = 15.1%, U1000 = 3.8 V und LD = 150 h für j0 = 10 mA/cm², L1 = 80%.

**Tabelle 1: Strukturformeln der Materialien für die OLEDs**

| | |
|---|---|
| 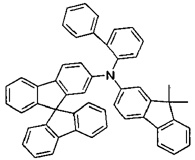 | 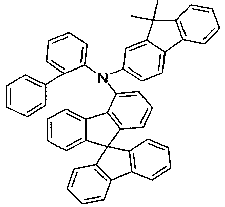 |
| SpMA1 | SpMA2 |
| 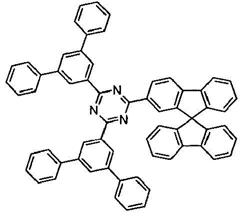 | 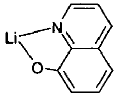 |
| ST2 | LiQ |
| 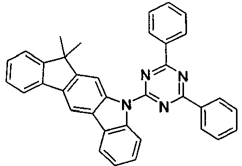 | 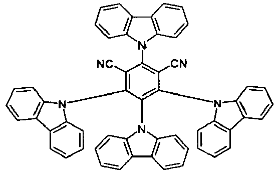 |
| IC1 | D1 |
| 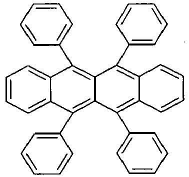 | 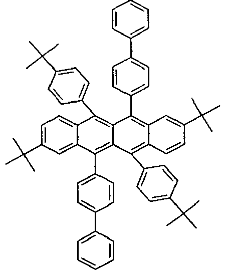 |
| Rubren | SE1 |
| 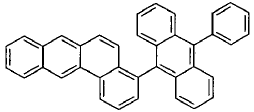 | |
| AM1 | |

**Tabelle 2: HOMO, LUMO, T₁, S₁ der relevanten Materialien**

| Material | Methode | HOMO (eV) | LUMO (eV) | S₁ (eV) | T₁ (eV) |
|---|---|---|---|---|---|
| D1 | org. | -6.11 | -3.40 | 2.50 | 2.41 |
| IC1 | org. | -5.79 | -2.83 | 3.09 | 2.69 |
| SpMA1 | org. | -5.25 | -2.18 | 3.34 | 2.58 |
| SpMA2 | org. | -5.35 | -2.34 | 3.14 | 2.62 |
| ST2 | org. | -6.03 | -2.82 | 3.32 | 2.68 |
| LiQ | M-org. | -5.17 | -2.39 | 2.85 | 2.13 |

### Vergleichsbeispiel gemäß dem Stand der Technik

In US 2012/248968 sind OLEDs beschrieben, die eine TADF Verbindung und ein fluoreszentes Emittermaterial enthalten. Die verwendete Verbindung GH-4 weist einen energetischen Abstand von 0.0 4eV zwischen S₁ und T₁ auf. Für den fluoreszenten Emitter GD-1 mit GH-4 als TADF Verbindung gilt *S* = 0.834. Die gezeigte externe Quanteneffizienz für diese Kombination beträgt für 1 mA/cm² (entspricht 174 cd/m²) EQE = 5.04 % und für 10 mA/cm² (1585 cd/m²) EQE = 4.59 %. Da die Effizienz zu höheren Leuchtdichten generell abnimmt, sind die in der vorliegenden Erfindung gezeigten EQE für 1000 cd/m² somit deutlich höher.

Die im Stand der Technik verwendeten Verbindungen GH-4 und GD-1 sind im Folgenden abgebildet:

| | |
|---|---|
| 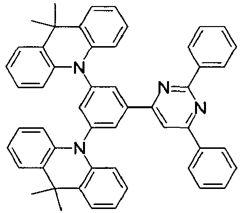 | 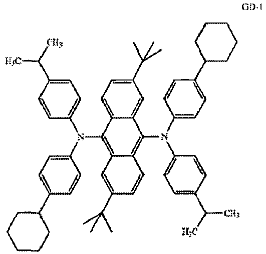 |
| GH-4 | GD-1 |

## Patentansprüche

1. Organische Elektrolumineszenzvorrichtung, enthaltend Kathode, Anode und mindestens eine emittierende Schicht, die eine sterisch abgeschirmte fluoreszierende Verbindung enthält, wobei die emittierende Schicht oder eine an die emittierende Schicht angrenzende Schicht eine lumineszente organische Verbindung enthält, die einen Abstand zwischen dem niedrigsten Triplettzustand T₁ und dem ersten angeregten Singulettzustand S₁ von ≤ 0.30 eV aufweist (TADF-Verbindung), wobei die Peakemissionswellenlänge der sterisch abgeschirmten fluoreszierenden Verbindung größer oder gleich der Peakemissionswellenlänge der TADF-Verbindung ist; dabei wird zur Bestimmung der Peakemissionswellenlänge das Material in einer Konzentraion von 0.5mg/100ml in Toluol gelöst und die Lösung in einem Fluoreszenzspektrometer bei Raumtemperatur mit einer Wellenlänge von 350 nm angeregt, wobei die Peakemissionswellenlänge die Wellenlänge ist, bei der das dabei erhaltene Emissionsspektrum seinen Maximalwert erreicht, **dadurch gekennzeichnet, dass** die sterisch abgeschirmte fluoreszierende Verbindung einen Abschirmparameter S ≤ 0.6 aufweist, wobei der Abschirmparamter bestimmt wird, wie unter 6) in der allgemeinen Beschreibung der Bestimmung der relevanten Parameter definiert.

2. Organische Elektrolumineszenzvorrichtung nach Anspruch 1, **dadurch gekennzeichnet, dass** die Peakemissionswellenlänge der sterisch abgeschirmten fluoreszierenden Verbindung mindestens 10 nm größer ist als die der TADF-Verbindung.

3. Organische Elektrolumineszenzvorrichtung nach Anspruch 1 oder 2, **dadurch gekennzeichnet, dass** die emittierende Schicht eine Mischung aus der sterisch abgeschirmten fluoreszierenden Verbindung und der TADF-Verbindung enthält oder dass die organische Elektrolumineszenzvorrichtung angrenzend an die emittierende Schicht auf Anodenseite oder auf Kathodenseite eine Schicht enthält, welche die TADF-Verbindung enthält

4. Organische Elektrolumineszenzvorrichtung nach einem oder mehreren der Ansprüche 1 bis 3, **dadurch gekennzeichnet, dass** die TADF-Verbindung eine Lumineszenzquanteneffizienz von mindestens 40 % aufweist; zur Bestimmung der Lumineszenzquanteneffizienz wird von der Schicht, die ein TADF-Material enthält, ein 50 nm dicker Film auf ein Quarzsubstrat aufgebracht, wobei der Film dieselben Materialien in denselben Konzentrationsverhältnissen wie die entsprechende Schicht in der OLED außer der flucreszenten Verbindung enthält und zur Herstellung des Films die gleichen Herstellungsbedingungen wie zur Herstellung der Emissionsschicht für die OLEDs verwendet werden; von dem Film wird ein Absorptionsspektrum im Wellenlängenbereich von 350-500 nm gemessen, wobei das Reflexionsspektrum R(λ) sowie das Transmissionsspektrum T(λ) der Probe unter einem Einfallswinkel von 6° bestimmt wird und als Absorptionsspektrum A(λ)=1-P(λ)-T(λ) definiert wird; gilt A(λ) ≤ 0.3 im Bereich 350-500nm, so wird die zum Maximum des Absorptionsspektrums gehörige Wellenlänge im Bereich 350-500 nm als λ_{exc} definiert; gilt für irgendeine Wellenlänge A(λ) > 0.3, so wird als λ_{exc} die größte Wellenlänge definiert, bei der A(λ) von einem Wert kleiner 0.3 zu einem Wert größer 0.3 oder von einem Wert größer 0.3 zu einem Wert kleiner 0.3 wechselt; dabei wird ein Messplatz verwendet, wobei sich die Probe während der Messung in einer Ulbrichtkugel befindet; das Spektrum des Anregungslichts ist in etwa gaußförmig mit einer Halbwertsbreite < 10 nm und Peakwellenlänge λ_{exc}; die PLQE wird nach dem für den genannten Messplatz üblichen Auswerteverfahren bestimmt, wobei die Messung bei Raumtemperatur erfolgt und die Probe zu keinem Zeitpunkt mit Sauerstoff in Berührung kommt.

5. Organische Elektrolumineszenzvorrichtung nach einem oder mehreren der Ansprüche 1 bis 4, **dadurch gekennzeichnet, dass** die TADF-Verbindung eine Abklingzeit von ≤ 50 µs aufweist, wobei zur Bestimmung der Abklingzeit eine Probe verwendet wird, die wie in Anspruch 4 beschrieben hergestellt wird, die sich im Vakuum <10⁻⁵ mbar befindet und bei Raumtemperatur durch einen Laserpuls (Wellenlänge 266 nm, Pulsdauer 1.5 ns, Pulsenergie 200 µJ, Strahldurchmesser 4 mm) angeregt wird; nach der Anregung (definiert als t = 0) wird der zeitliche Verlauf der Intensität der emittierten Photolumineszenz gemessen; die Abklingzeit tₐ wird wie folgt bestimmt: Man wählt einen Zeitpunkt t_{d}, zu dem die prompte Fluoreszenz deutlich unter die Intensität der verzögerten Fluoreszenz abgeklungen ist und bestimmt für die Messdaten ab dem Zeitpunkt t_{d} die Abklingzeit tₐ = tₑ -t_{d}, wobei tₑ derjenige Zeitpunkt nach t = t_{d} ist, bei dem die Intensität erstmals auf 1/e ihres Wertes bei t = t_{d} abgefallen ist.

6. Organische Elektrolumineszenzvorrichtung nach einem oder mehreren der Ansprüche 1 bis 5, **dadurch gekennzeichnet, dass** der Abstand zwischen S₁ und T₁ der TADF-Verbindung ≤ 0.25 eV ist, bevorzugt ≤ 0.15 eV und besonders bevorzugt ≤ 0.10 eV.

7. Organische Elektrolumineszenzvorrichtung nach einem oder mehreren der Ansprüche 1 bis 6, **dadurch gekennzeichnet, dass** die sterisch abgeschirmte fluoreszierende Verbindung eine Lumineszenzquanteneffizienz von mindestens 60 % aufweist, bevorzugt von mindestens 80 %, besonders bevorzugt von mindestens 90 %;
dabei wird zur Bestimmung der PLQE die Lösung der fluoreszenten Verbindung in Toluol in einer Konzentration von 1 mg/100 ml in einem Messplatz bei Raumtemperatur vermessen, wie in Anspruch 4 beschrieben, wobei als Anregungswellenlänge die Wellenlänge 0.27^{∗}λₘₐₓ+300nm verwendet wird, wobei λₘₐₓ die Peakemissionswellenlänge der fluoreszenten Verbindung, wie in Anspruch 1 definiert, darstellt.

8. Organische Elektrolumineszenzvorrichtung nach einem oder mehreren der Ansprüche 1 bis 7, **dadurch gekennzeichnet, dass** als sterisch abgeschirmte fluoreszierende Verbindung steife π-Systeme eingesetzt werden, die mit aliphatischen, cycloaliphatischen oder aromatischen Substituenten, die durch aliphatische oder cycloaliphatlsche Reste substituiert sein können, substituiert sind.

9. Organische Elektrolumineszenzvorrichtung nach einem oder mehreren der Ansprüche 1 bis 8, **dadurch gekennzeichnet, dass** der fluoreszierende Grundkörper der sterisch abgeschirmten Verbindung ausgewählt ist aus den Gruppen der Formeln (1) bis (70), 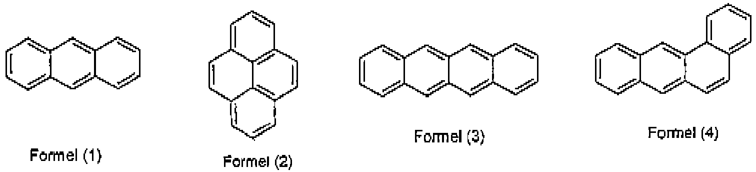 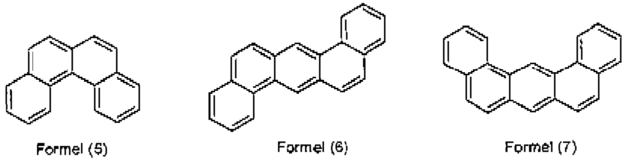 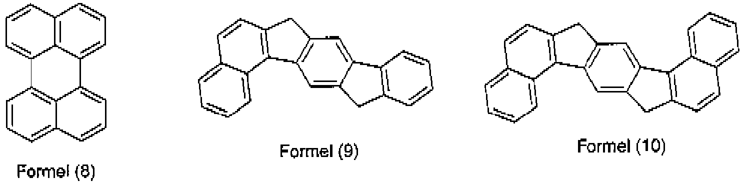 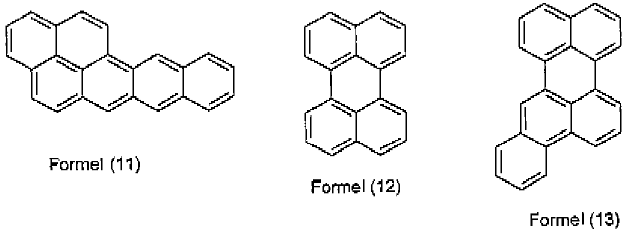 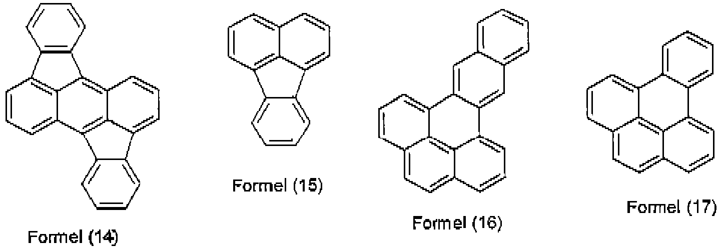 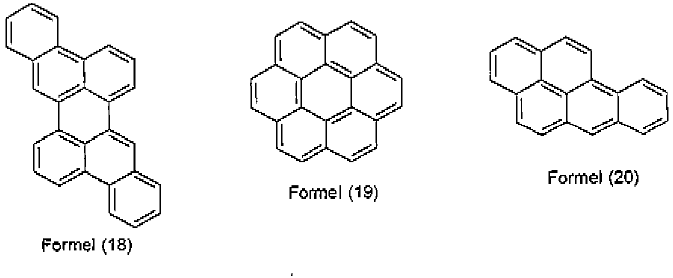 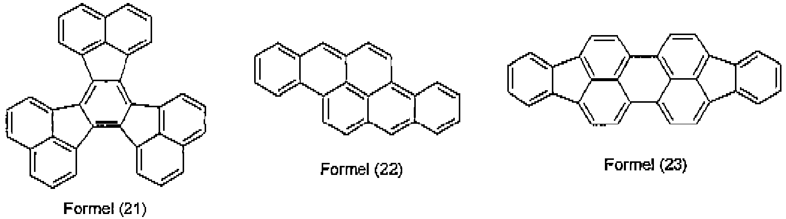 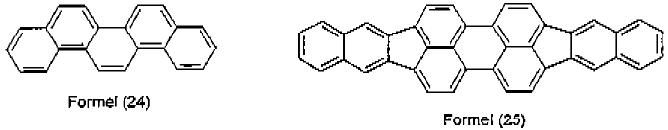 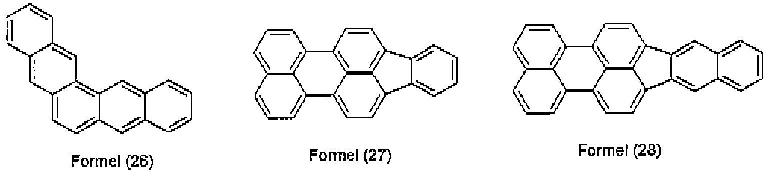 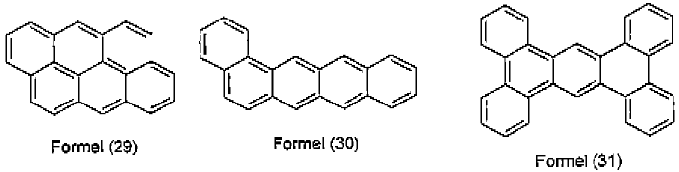 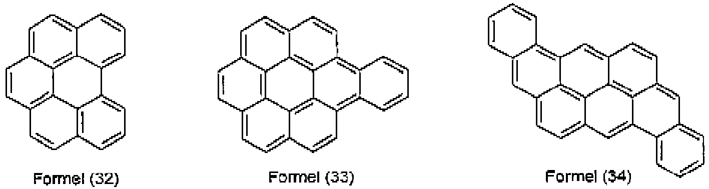 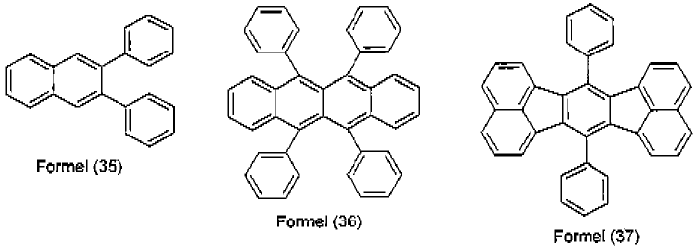 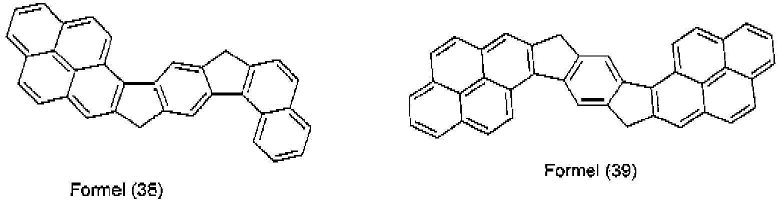 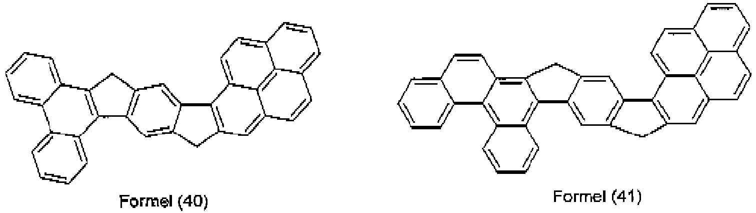 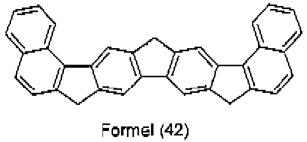 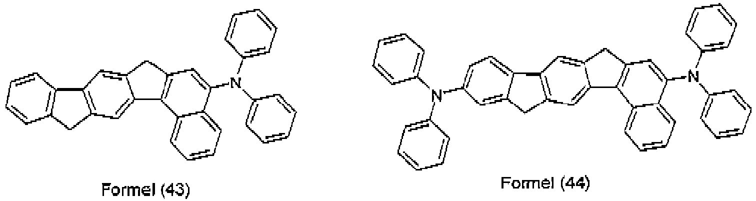 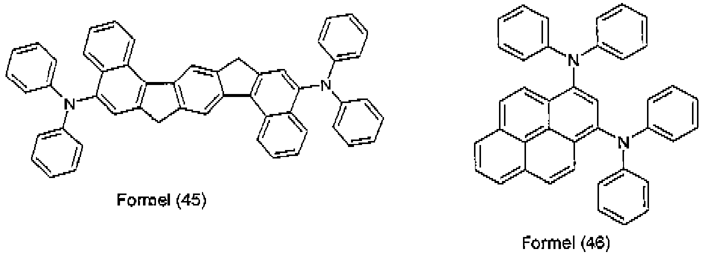 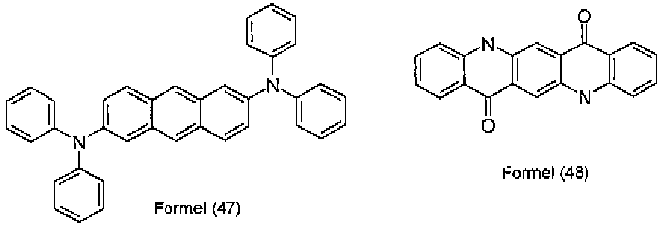 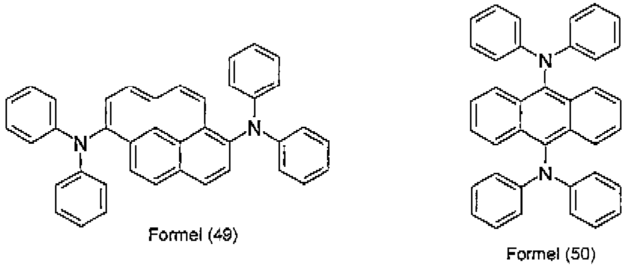 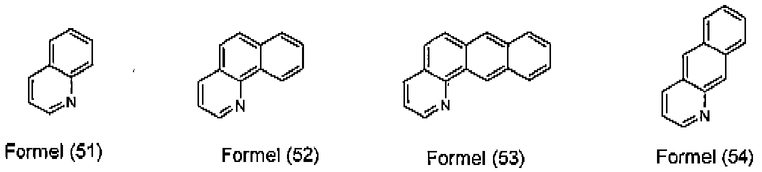 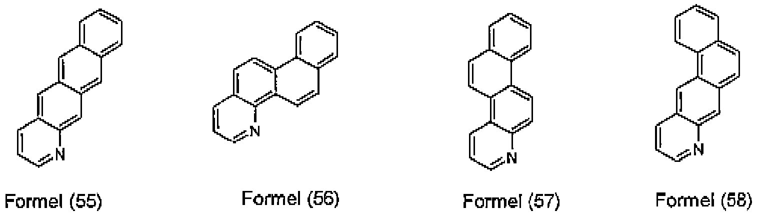 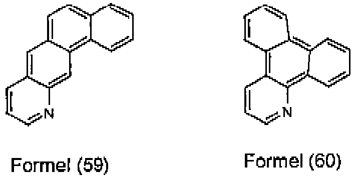 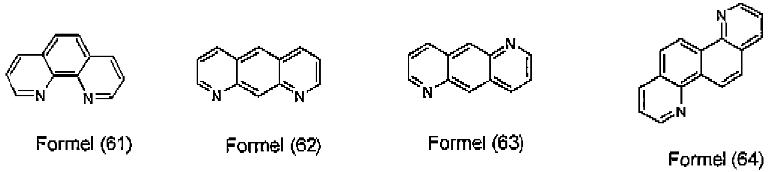 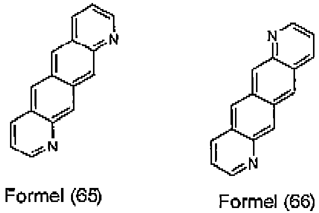 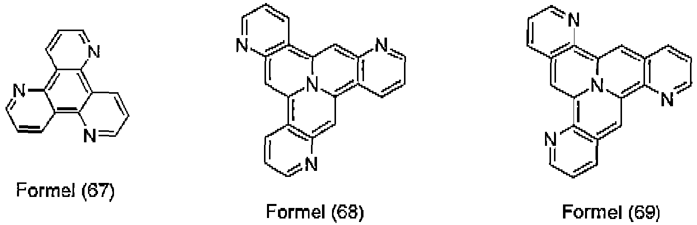 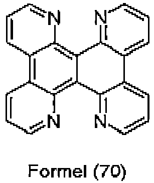 wobei in dem kondensierten aromatischen Grundkörper in den Gruppen der Formeln (1) bis (50) auch ein, zwei, drei oder vier Kohlenstoffatome durch Stickstoff ersetzt sein können.

10. Organische Elektrolumineszenzvorrichtung nach einem oder mehreren der Ansprüche 1 bis 9, **dadurch gekennzeichnet, dass** als Substituenten zur Abschirmung in der sterisch abgeschirmten fluoreszierenden Verbindung Alkylgruppen, insbesondere mit 3 bis 20 C-Atomen, in denen auch H-Atome durch F ersetzt sein können, Alkoxygruppen, insbesondere mit 3 bis 20 C-Atomen, Aralkylgruppen, insbesondere mit 7 bis 30 C-Atomen, und aromatische Ringsysteme, insbesondere mit 6 bis 30 C-Atomen, wobei in den Aralkylgruppen und aromatischen Ringsystemen die Arylgruppen auch durch ein oder mehrere Alkylgruppen mit 1 bis 10 C-Atomen substituiert sein können, eingesetzt werden;
und/oder dass als Substituenten zur Abschirmung in der sterisch abgeschirmten fluoreszierenden Verbindung eine oder mehrere ankondensierte aliphatische Gruppen der Formeln (Ring-1) bis (Ring-7) eingesetzt werden, 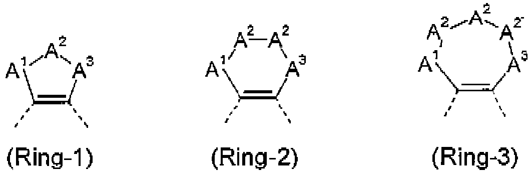 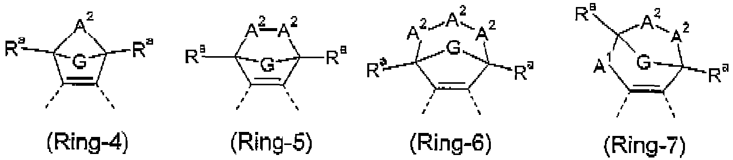 wobei die gestrichelten Bindungen die Verknüpfung der beiden Kohlenstoffatome im fluoreszierenden Grundkörper andeuten und weiterhin gilt:
A¹, A², A³ ist gleich oder verschieden bei jedem Auftreten C(R^{a})₂, O oder S;
G ist eine Alkylengruppe mit 1, 2 oder 3 C-Atomen, welche mit einem oder mehreren Resten R^{b} substituiert sein kann oder eine orthoverknüpfte Arylengruppe mit 5 bis 14 aromatischen Ringatomen, welche durch einen oder mehrere Reste R^{b} substituiert sein kann;
R^{a} ist bei jedem Auftreten gleich oder verschieden ausgewählt aus der Gruppe bestehend aus H, D, F, einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R^{b} substituiert sein kann, einem aromatischen Ringsystem mit 5 bis 60 aromatischen Ringatomen, das jeweils mit einem oder mehreren Resten R^{b} substituiert sein kann, oder einer Aralkylgruppe mit 5 bis 60 aromatischen Ringatomen, die mit einem oder mehreren Resten R^{b} substituiert sein kann, wobei optional zwei oder mehr benachbarte Substituenten R^{a} ein Ringsystem bilden können, das mit einem oder mehreren Resten R^{b} substituiert sein kann;
R^{b} ist ausgewählt aus der Gruppe bestehend aus H, D, F, einem aliphatischem Kohlenwasserstoffrest mit 1 bis 20 C-Atomen, einem aromatischem Ringsystem mit 5 bis 30 aromatischen Ringatomen, wobei zwei oder mehr benachbarte Substituenten R^{b} miteinander ein Ringsystem bilden können;
mit der Maßgabe, dass in diesen Gruppen nicht zwei Heteroatome direkt aneinander gebunden sind.

11. Organische Elektrolumineszenzvorrichtung nach Anspruch 10, **dadurch gekennzeichnet, dass** die Gruppen (Ring-1) bis (Ring-7) keine aziden benzylischen Protonen aufweisen.

12. Organische Elektrolumineszenzvorrichtung nach Anspruch 10 oder 11, **dadurch gekennzeichnet, dass** die Strukturen (Ring-1) bis (Ring-7) ausgewählt sind aus den folgenden Strukturen (Ring-1A) bis (Ring-7-A), 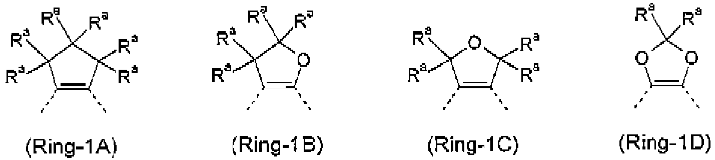 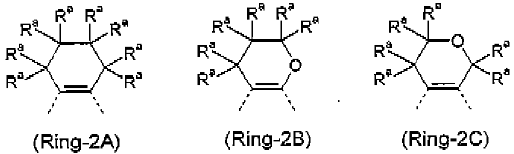 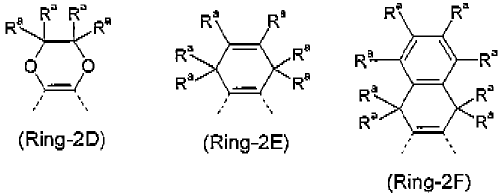 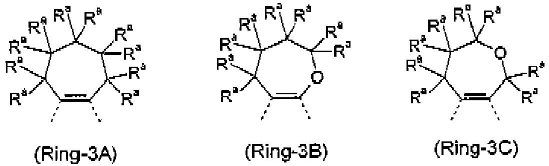 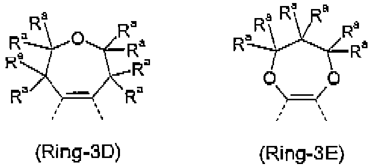 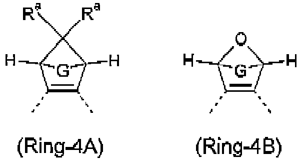 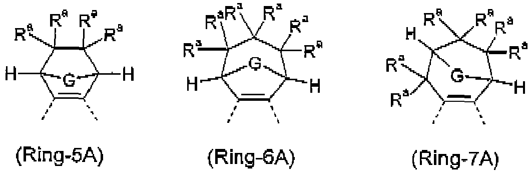 wobei R^{a} und G die in Anspruch 10 genannten Bedeutungen aufweisen und R^{a} in den benzylischen Positionen bevorzugt nicht für H oder D steht.

13. Organische Elektrolumineszenzvorrichtung nach einem oder mehreren der Ansprüche 1 bis 12, **dadurch gekennzeichnet, dass** die emittierende Schicht mindestens eine Matrixverbindung enthält.

14. Organische Elektrolumineszenzvorrichtung nach Anspruch 13, **dadurch gekennzeichnet, dass** die Matrixverbindung eine oder mehrere Verbindungen sind, die ausgewählt sind aus der Gruppe bestehend aus Ketonen, Phosphinoxiden, Sulfoxiden, Sulfonen, Triarylaminen, Carbazolen, Dibenzofuranen, Indolocarbazolen, Indenocarbazolen, Azacarbazolen, bipolaren Matrixmaterialien, Silanen, Azaborolen, Boronestern, Diazasilolen, Diazaphospholen, Triazinen, Pyrimidinen, Chinoxalinen, Zn-, AI- oder Be-Komplexen oder verbrückten Carbazolen.

15. Verfahren zur Herstellung einer organischen Elektrolumineszenzvorrichtung nach einem oder mehreren der Ansprüche 1 bis 14, **dadurch gekennzeichnet, dass** mindestens eine Schicht mit einem Sublimationsverfahren aufgebracht wird und/oder dass mindestens eine Schicht mit dem Organic Vapour Phase Deposition Verfahren oder mit Hilfe einer Trägergassublimation aufgebracht wird und/oder dass mindestens eine Schicht aus Lösung, durch Spincoating oder mit einem Druckverfahren aufgebracht wird.

## Claims

1. Organic electroluminescent device comprising cathode, anode and at least one emitting layer comprising a sterically shielded fluorescent compound,
wherein the emitting layer or a layer adjoining the emitting layer contains a luminescent organic compound having a gap between the lowest triplet state T₁ and the first excited singlet state S₁ of ≤ 0.30 eV (TADF compound), where the peak emission wavelength of the sterically shielded fluorescent compound is greater than or equal to the peak emission wavelength of the TADF compound;
where the peak emission wavelength is determined by dissolving the material in toluene in a concentration of 0.5 mg/100 ml and exciting the solution with a wavelength of 350 nm at room temperature in a fluorescence spectrometer, where the peak emission wavelength is the wavelength at which the emission spectrum obtained reaches its maximum value, **characterized in that** the sterically shielded fluorescent compound has a shielding parameter S ≤ 0.6, where the shielding parameter is determined as defined in 6) in the general description of the determination of the relevant parameters.

2. Organic electroluminescent device according to Claim 1, **characterized in that** the peak emission wavelength of the sterically shielded fluorescent compound is at least 10 nm greater than that of the TADF compound.

3. Organic electroluminescent device according to Claim 1 or 2, **characterized in that** the emitting layer comprises a mixture of the sterically shielded fluorescent compound and the TADF compound, or **in that** the organic electroluminescent device adjoining the emitting layer on the anode side or on the cathode side comprises a layer comprising the TADF compound.

4. Organic electroluminescent device according to one or more of Claims 1 to 3, **characterized in that** the TADF compound has a luminescence quantum efficiency of at least 40%; the luminescence quantum efficiency of the layer comprising a TADF material is determined by applying a 50 nm-thick film to a quartz substrate, said film comprising the same materials in the same concentration ratios as the corresponding layer in the OLED except for the fluorescent compound and said film being produced using the same production conditions as for production of the emission layer for the OLEDs; an absorption spectrum of this film is measured in the wavelength range of 350-500 nm by determining the reflection spectrum R(λ) and the transmission spectrum T(λ) of the sample at an angle of incidence of 6°, the absorption spectrum being defined as A(λ) = 1 - R(λ) - T(λ) ; if A(λ) ≤ 0.3 in the range of 350-500 nm, the wavelength corresponding to the maximum of the absorption spectrum in the range of 350-500 nm is defined as λ_{exc}; if, for any wavelength, A(λ) > 0.3, λ_{exc} is defined as being the greatest wavelength at which A(λ) changes from a value of less than 0.3 to a value of greater than 0.3 or from a value of greater than 0.3 to a value of less than 0.3; this is done using a measurement system wherein the sample is within an Ulbricht sphere during the measurement; the spectrum of the excitation light is approximately Gaussian with a half-height width of < 10 nm and a peak wavelength λ_{exc} as defined above; the PLQE is determined by the evaluation method customary for said measurement system, wherein the measurement is effected at room temperature and the sample does not come into contact with oxygen at any time.

5. Organic electroluminescent device according to one or more of Claims 1 to 4, **characterized in that** the TADF compound has a decay time of ≤ 50 µs, where the decay time is determined using a sample that has been produced as described in Claim 4, which is at a reduced pressure of < 10⁻⁵ mbar and is excited at room temperature by a laser pulse (wavelength 266 nm, pulse duration 1.5 ns, pulse energy 200 µJ, beam diameter 4 mm); after excitation (defined as t = 0), the progression of the intensity of the photoluminescence emitted against time is measured; the decay time tₐ is determined as follows: a time t_{d} at which the prompt fluorescence has abated to well below the intensity of the delayed fluorescence is chosen and, for the measurement data from the time t_{d}, the decay time tₐ = tₑ - t_{d} is determined, where tₑ is that time after t = t_{d} at which the intensity has for the first time dropped to 1/e of its value at t = t_{d}.

6. Organic electroluminescent device according to one or more of Claims 1 to 5, **characterized in that** the gap between S₁ and T₁ of the TADF compound is ≤ 0.25 eV, preferably ≤ 0.15 eV and more preferably ≤ 0.10 eV.

7. Organic electroluminescent device according to one or more of Claims 1 to 6, **characterized in that** the sterically shielded fluorescent compound has a luminescence quantum efficiency of at least 60%, preferably of at least 80%, more preferably of at least 90%;
the PLQE of the fluorescent compound is determined by analysing the solution of the fluorescent compound in toluene in a concentration of 1 mg/100 ml in a measurement system at room temperature, as described in Claim 4, using, as the excitation wavelength, the wavelength 0.27 ^{∗} λₘₐₓ + 300 nm, where λₘₐₓ represents the peak emission wavelength of the fluorescent compound as defined in Claim 1.

8. Organic electroluminescent device according to one or more of Claims 1 to 7, **characterized in that** the sterically shielded fluorescent compounds used comprise rigid π systems substituted by aliphatic, cycloaliphatic or aromatic substituents which may be substituted by aliphatic or cycloaliphatic radicals.

9. Organic electroluminescent device according to one or more of Claims 1 to 8, **characterized in that** the fluorescent base skeleton of the sterically shielded compound is selected from the groups of the formulae (1) to (70) : 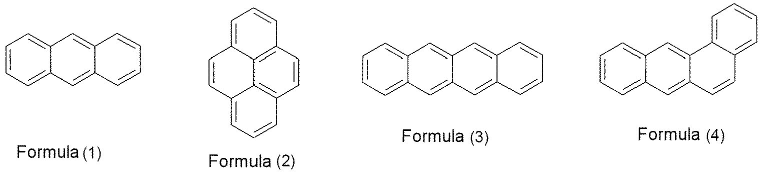 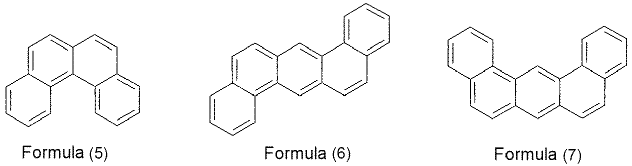 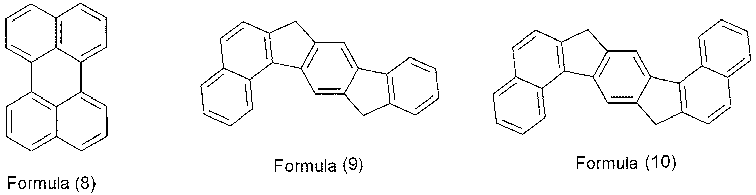 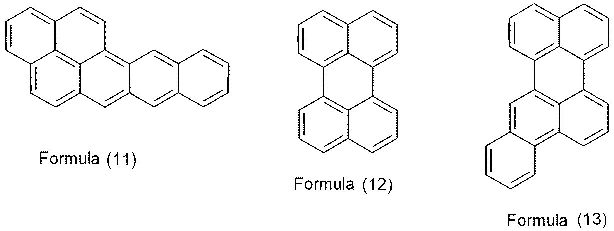 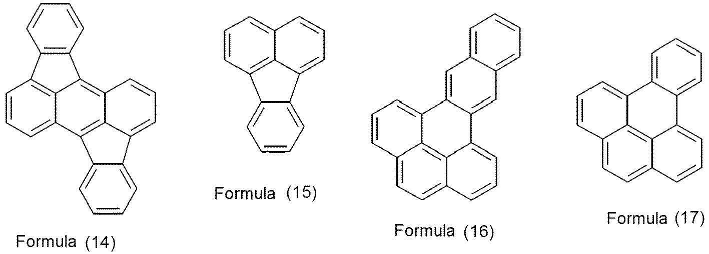 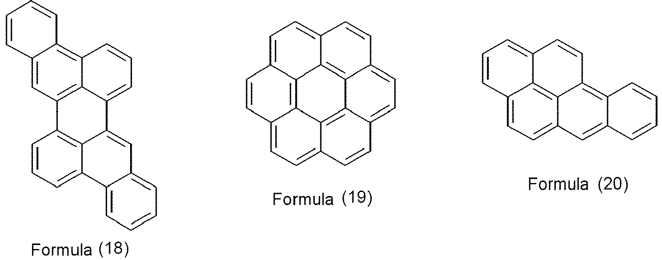 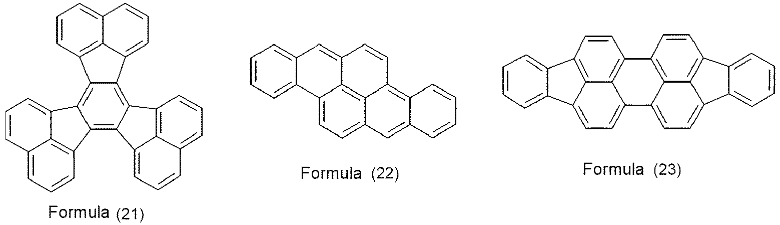 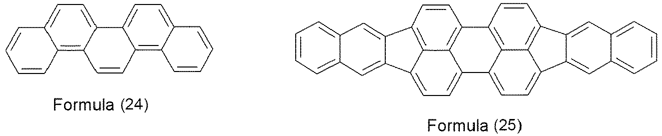 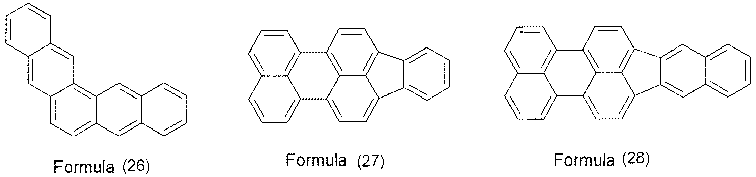 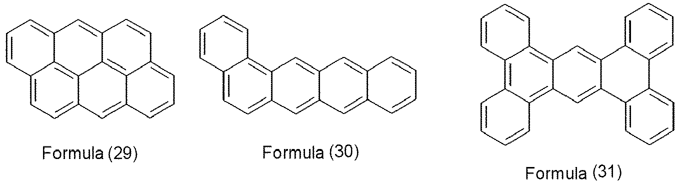 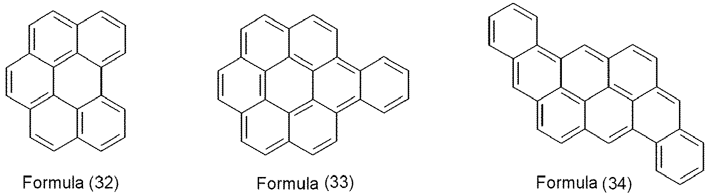 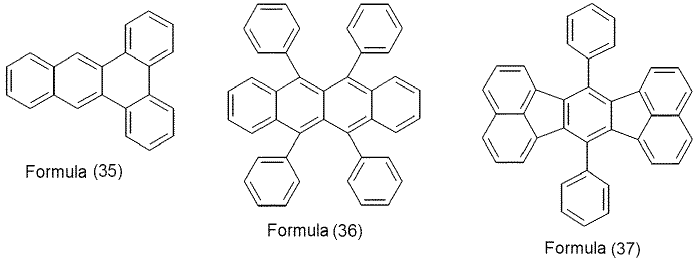 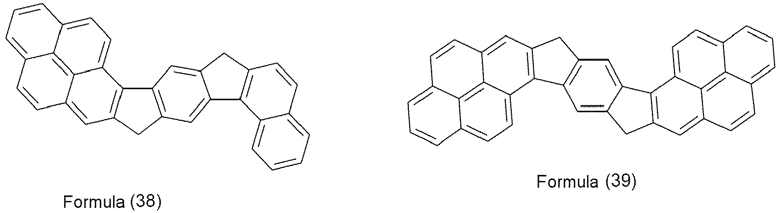 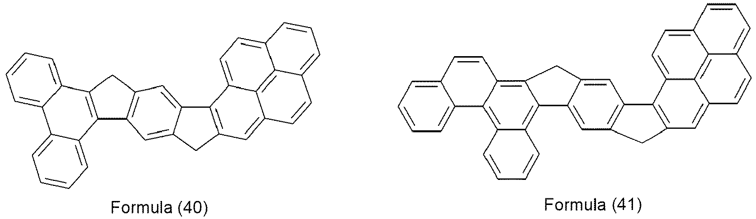 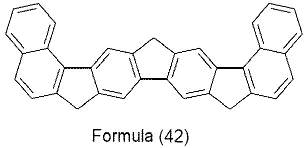 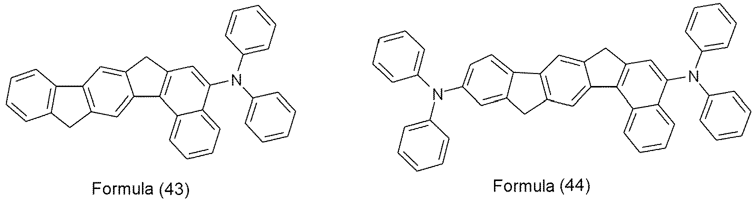 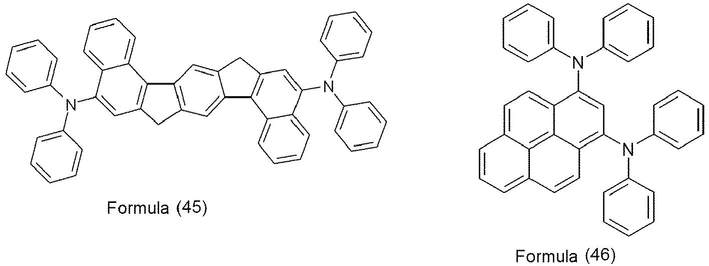 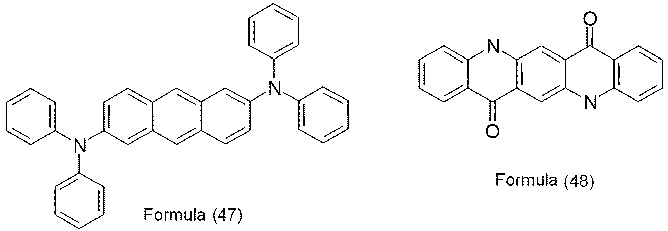 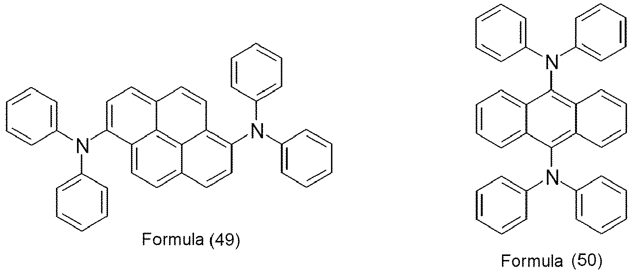 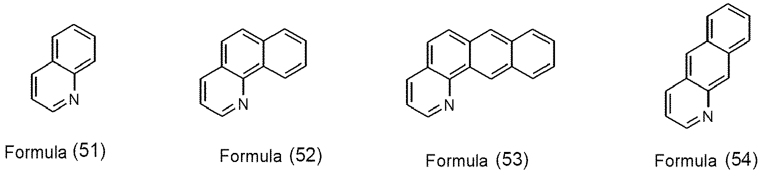 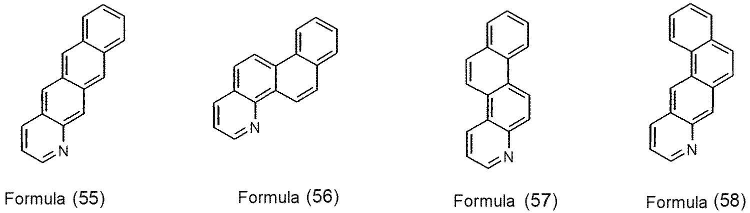 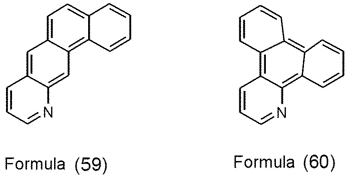 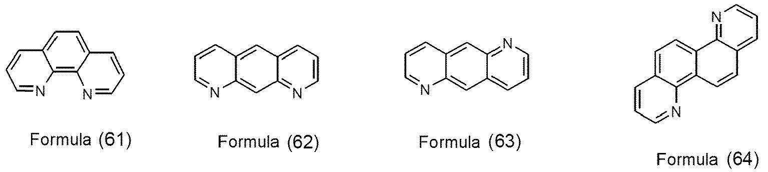 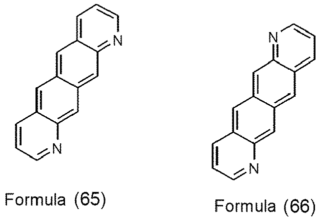 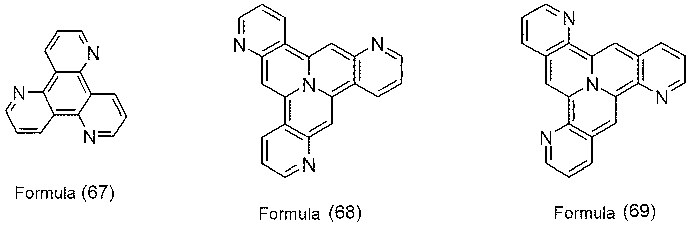 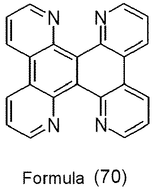 where it is also possible for one, two, three or four carbon atoms in the fused aromatic base skeleton in the groups of the formulae (1) to (50) to be replaced by nitrogen.

10. Organic electroluminescent device according to one or more of Claims 1 to 9, **characterized in that** substituents used for shielding in the sterically shielded fluorescent compound are alkyl groups, especially having 3 to 20 carbon atoms, in which hydrogen atoms may also be replaced by F, alkoxy groups, especially having 3 to 20 carbon atoms, aralkyl groups, especially having 7 to 30 carbon atoms, and aromatic ring systems, especially having 6 to 30 carbon atoms, where it is also possible for the aryl groups in the aralkyl groups and aromatic ring systems to be substituted by one or more alkyl groups having 1 to 10 carbon atoms;
and/or **in that** substituents used for shielding in the sterically shielded fluorescent compound are one or more fused-on aliphatic groups of the formulae (Ring-1) to (Ring-7) 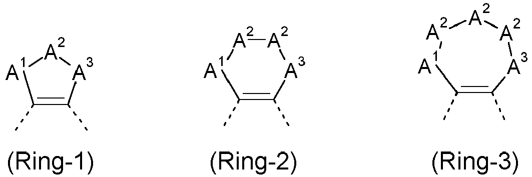 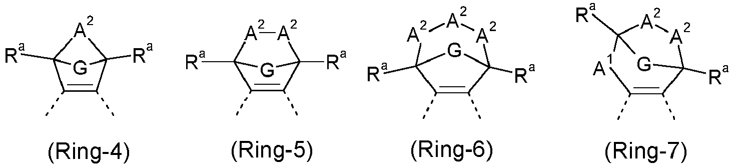 where the dotted bonds indicate the linkage of the two carbon atoms within the fluorescent base skeleton and in addition:
A¹, A², A³ is the same or different at each instance and is C(R^{a})₂, O or S;
G is an alkylene group which has 1, 2 or 3 carbon atoms and may be substituted by one or more R^{b} radicals or an ortho-bonded arylene group which has 5 to 14 aromatic ring atoms and may be substituted by one or more R^{b} radicals;
R^{a} is the same or different at each instance and is selected from the group consisting of H, D, F, a straight-chain alkyl group having 1 to 40 carbon atoms or a branched or cyclic alkyl group having 3 to 40 carbon atoms, each of which may be substituted by one or more R^{b} radicals, an aromatic ring system having 5 to 60 aromatic ring atoms, each of which may be substituted by one or more R^{b} radicals, or an aralkyl group which has 5 to 60 aromatic ring atoms and may be substituted by one or more R^{b} radicals, where it is optionally possible for two or more adjacent R^{a} substituents to form a ring system which may be substituted by one or more R^{b} radicals;
R^{b} is selected from the group consisting of H, D, F, an aliphatic hydrocarbyl radical having 1 to 20 carbon atoms, an aromatic ring system having 5 to 30 aromatic ring atoms, where two or more adjacent R^{b} substituents together may form a ring system;
with the proviso that no two heteroatoms in these groups are bonded directly to one another.

11. Organic electroluminescent device according to Claim 10, **characterized in that** the (Ring-1) to (Ring-7) groups do not have any acidic benzylic protons.

12. Organic electroluminescent device according to Claim 10 or 11, **characterized in that** the (Ring-1) to (Ring-7) structures are selected from the following structures (Ring-1A) to (Ring-7A): 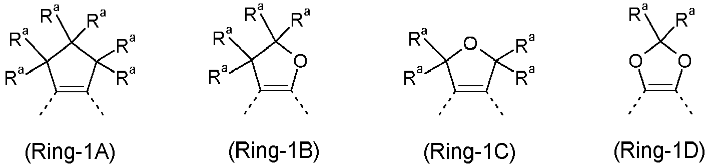 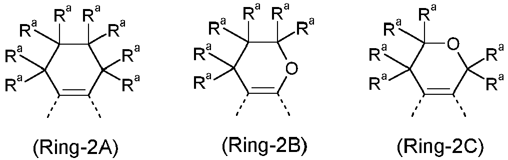 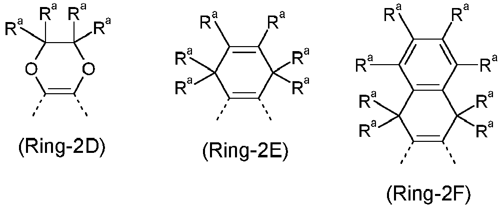 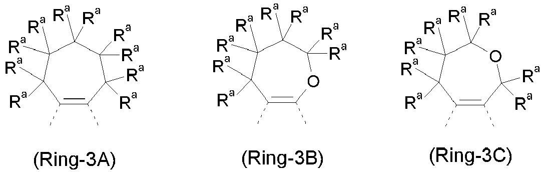 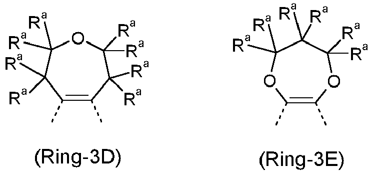 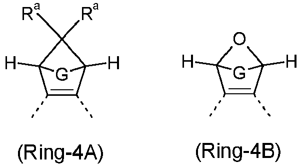 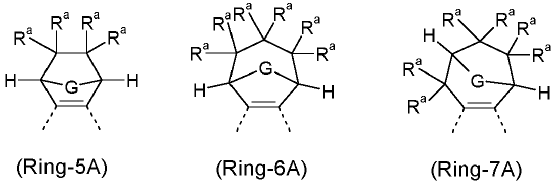 where R^{a} and G have the definitions given in Claim 10 and R^{a} in the benzylic positions is preferably not H or D.

13. Organic electroluminescent device according to one or more of Claims 1 to 12, **characterized in that** the emitting layer comprises at least one matrix compound.

14. Organic electroluminescent device according to Claim 13, **characterized in that** the matrix compound is one or more compounds selected from the group consisting of ketones, phosphine oxides, sulfoxides, sulfones, triarylamines, carbazoles, dibenzofurans, indolocarbazoles, indenocarbazoles, azacarbazoles, bipolar matrix materials, silanes, azaboroles, boronic esters, diazasiloles, diazaphospholes, triazines, pyrimidines, quinoxalines, Zn complexes, Al complexes or Be complexes, or bridged carbazoles.

15. Process for producing an organic electroluminescent device according to one or more of Claims 1 to 14, **characterized in that** at least one layer is applied by a sublimation method and/or **in that** at least one layer is applied by an organic vapour phase deposition method or with the aid of a carrier gas sublimation and/or **in that** at least one layer is applied from solution, by spin-coating or by a printing method.

## Revendications

1. Dispositif électroluminescent organique contenant une cathode, une anode et au moins une couche émettrice qui contient un composé fluorescent à blindage stérique, la couche émettrice ou une couche adjacente à la couche émettrice contenant un composé organique luminescent qui présente une distance ≤ 0,30 eV entre l'état triplet le plus bas T₁ et le premier état singulet excité S₁ (composé TADF), la longueur d'onde d'émission de crête du composé fluorescent à blindage stérique étant supérieure ou égale à la longueur d'onde d'émission de crête du composé TADF ; pour déterminer la longueur d'onde d'émission de crête, le matériau est dissous à une concentration de 0,5 mg/100 ml dans du toluène et la solution est excitée dans un spectromètre à fluorescence à température ambiante à une longueur d'onde de 350 nm, la longueur d'onde d'émission de crête étant la longueur d'onde à laquelle le spectre d'émission ainsi obtenu atteint sa valeur maximale, **caractérisé en ce que** le composé fluorescent à blindage stérique présente un paramètre de blindage S ≤ 0,6, les paramètres de blindage étant déterminés de manière définie en 6) dans la description générale de la détermination des paramètres pertinents.

2. Dispositif électroluminescent organique selon la revendication 1, **caractérisé en ce que** la longueur d'onde d'émission de crête du composé fluorescent à blindage stérique est supérieure d'au moins 10 nm à celle du composé TADF.

3. Dispositif électroluminescent organique selon la revendication 1 ou 2, **caractérisé en ce que** la couche émettrice contient un mélange comprenant le composé fluorescent à blindage stérique et le composé TADF ou **en ce que** le dispositif électroluminescent organique contient une couche adjacente à la couche émettrice, côté anode ou côté cathode, qui contient le composé TADF.

4. Dispositif électroluminescent organique selon une ou plusieurs des revendications 1 à 3, **caractérisé en ce que** le composé TADF a un rendement quantique de luminescence d'au moins 40 % ; pour déterminer le rendement quantique de luminescence, un film de 50 nm d'épaisseur de la couche contenant un matériau TADF est appliqué sur un substrat de quartz, le film contenant les mêmes matériaux dans les mêmes rapports de concentration que la couche correspondante dans l'OLED, à l'exception du composé fluorescent et les mêmes conditions de production étant utilisées pour produire le film que pour produire la couche d'émission pour les OLED ; un spectre d'absorption dans la gamme de longueurs d'onde de 350 à 500 nm étant mesuré pour le film, le spectre de réflexion R(λ) et le spectre de transmission T(λ) de l'échantillon étant déterminés sous un angle d'incidence de 6° et étant définis comme le spectre d'absorption A(λ) = 1-R(λ)-T(λ) ; si on a A(λ) ≤ 0,3 dans la gamme allant de 350 à 500 nm, la longueur d'onde appartenant au maximum du spectre d'absorption dans la gamme allant de 350 à 500 nm étant définie comme λ_{exc} ; si on a A(λ) > 0,3 pour n'importe quelle longueur d'onde, alors λexc est défini comme la plus grande longueur d'onde à laquelle A(λ) passe d'une valeur inférieure à 0,3 à une valeur supérieure à 0,3 ou d'une valeur supérieure à 0,3 à une valeur inférieure à 0,3 ; une station de mesure étant utilisée pour cela, l'échantillon étant situé dans une sphère d'Ulbricht pendant la mesure ; le spectre de la lumière d'excitation étant à peu près gaussien avec une demi-largeur < 10 nm et une longueur d'onde de crête λ_{exc} ; le PLQE étant déterminé selon le procédé d'évaluation habituelle pour la station de mesure spécifiée, la mesure étant effectuée à température ambiante et l'échantillon ne venant jamais en contact avec l'oxygène.

5. Dispositif électroluminescent organique selon une ou plusieurs des revendications 1 à 4, **caractérisé en ce que** le composé TADF a un temps de décroissance ≤ 50 µs, un échantillon étant utilisé pour déterminer le temps de décroissance, lequel échantillon est produit de la manière décrite dans la revendication 4, est placé dans un vide < 10⁻⁵ mbar et est excité à température ambiante par une impulsion laser (longueur d'onde 266 nm, durée d'impulsion 1,5 ns, énergie d'impulsion 200 µJ, diamètre du faisceau 4 mm) ; après l'excitation (définie par t = 0), la variation dans le temps de l'intensité de la photoluminescence émise étant mesurée ; le temps de décroissance tₐ étant déterminé de la manière suivante : on choisit un instant t_{d} auquel la fluorescence rapide a décru bien au-dessous de l'intensité de la fluorescence retardée et on détermine le temps de décroissance tₐ = tₑ - t_{d} pour les données de mesure à partir de l'instant t_{d}, tₑ étant l'instant après t = t_{d} auquel l'intensité est tombée pour la première fois à 1/e de sa valeur pour t = t_{d}.

6. Dispositif électroluminescent organique selon une ou plusieurs des revendications 1 à 5, **caractérisé en ce que** la distance entre S₁ et T₁ du composé TADF est de ≤ 0,25 eV, de préférence ≤ 0,15 eV et de manière particulièrement préférée ≤ 0,10 eV.

7. Dispositif électroluminescent organique selon une ou plusieurs des revendications 1 à 6, **caractérisé en ce que** le composé fluorescent à blindage stérique présente un rendement quantique de luminescence d'au moins 60 %, de préférence d'au moins 80 %, de manière particulièrement préférée d'au moins 90 % ; pour déterminer le PLQE, la solution du composé fluorescent dans du toluène est alors mesurée à une concentration de 1 mg/100 ml dans une station de mesure à température ambiante, de la manière décrite dans la revendication 4, la longueur d'onde de 0,27*λₘₐₓ + 300 nm étant utilisée comme longueur d'onde d'excitation, λₘₐₓ étant la longueur d'onde d'émission de crête du composé fluorescent tel que défini dans la revendication 1.

8. Dispositif électroluminescent organique selon une ou plusieurs des revendications 1 à 7, **caractérisé en ce que** des systèmes π rigides sont utilisés comme composé fluorescent à blindage stérique, lesquels sont substitués par des substituants aliphatiques, cycloaliphatiques ou aromatiques qui peuvent être substitués par des radicaux aliphatiques ou cycloaliphatiques.

9. Dispositif électroluminescent organique selon une ou plusieurs des revendications 1 à 8, **caractérisé en ce que** le corps de base fluorescent du composé à blindage stérique est choisi parmi les groupes de formules (ci-dessous 'Formel') (1) à (70), 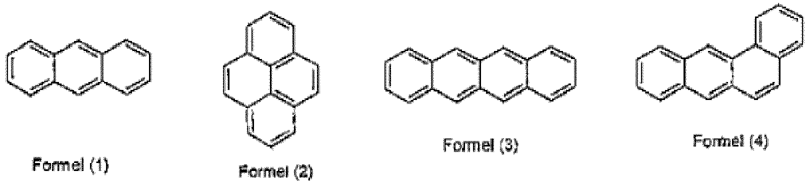 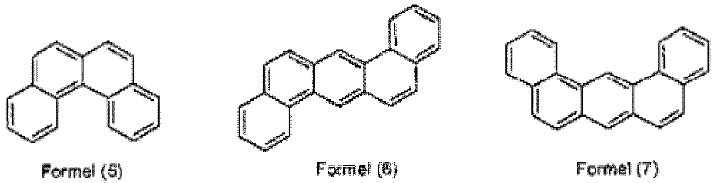 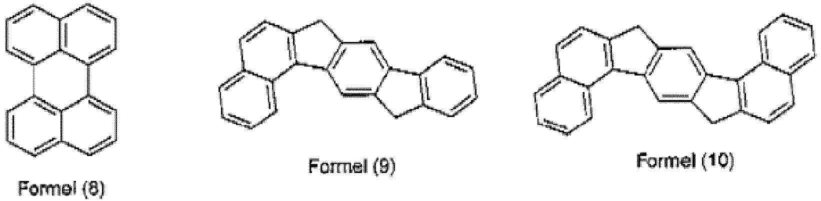 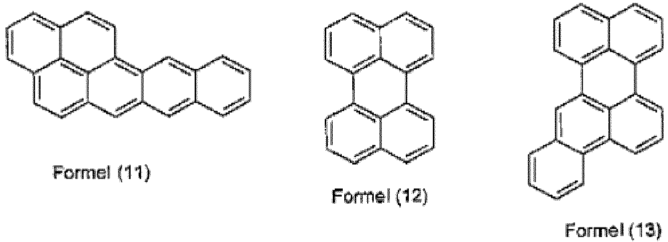 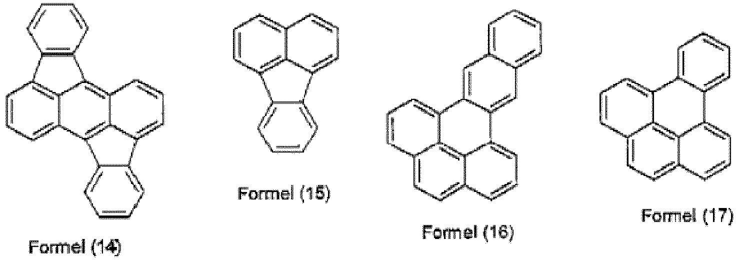 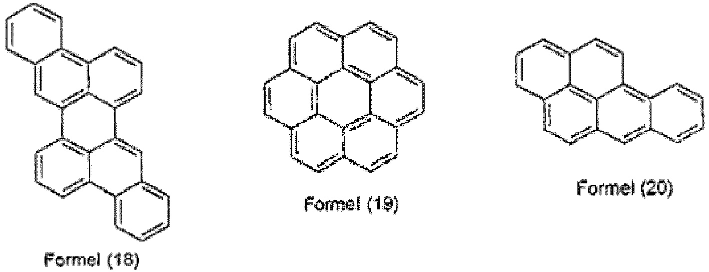 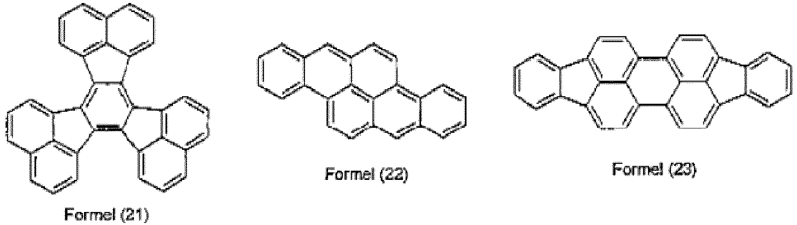 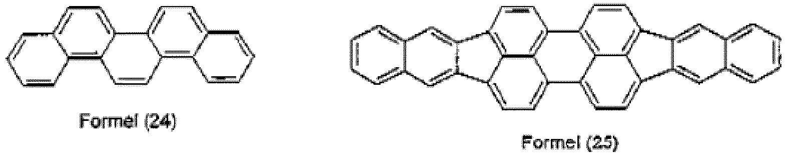 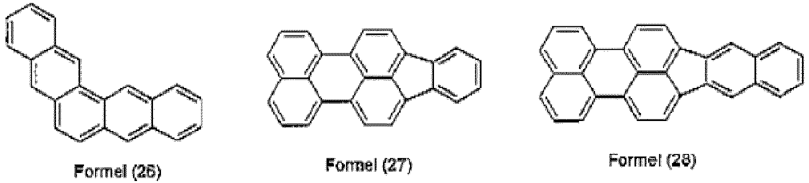 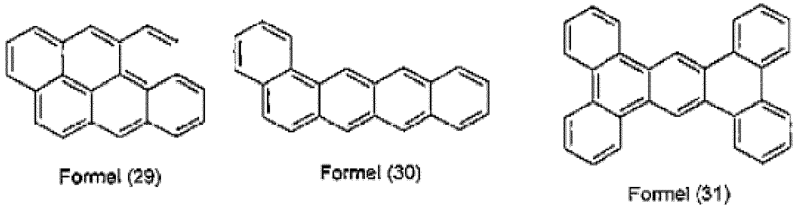 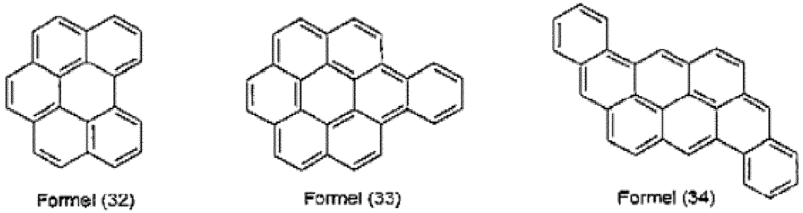 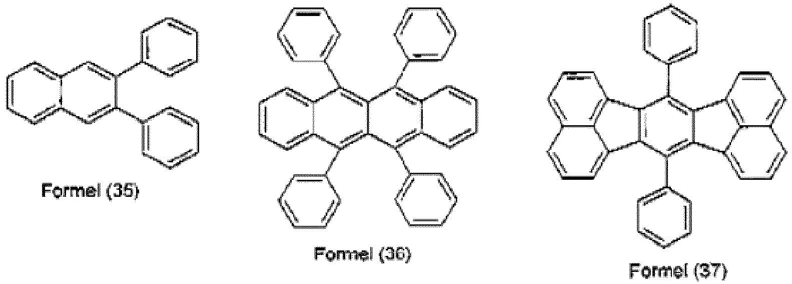 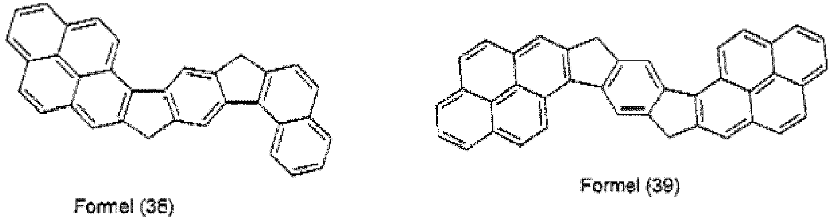 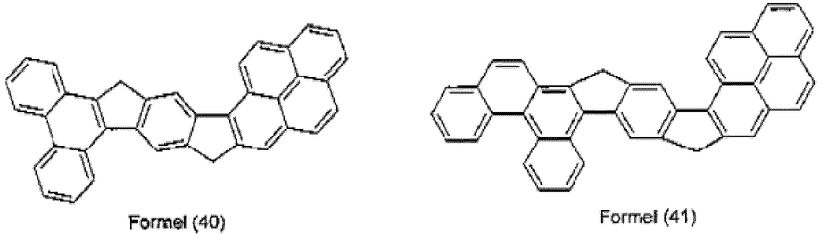 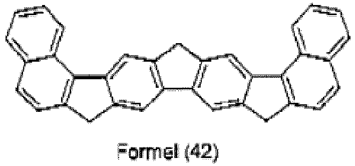 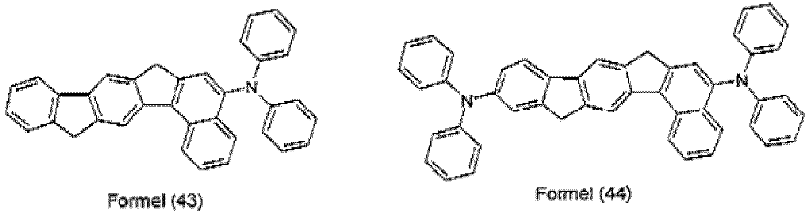 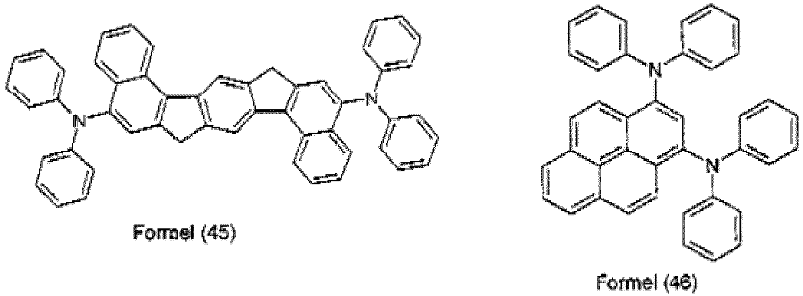 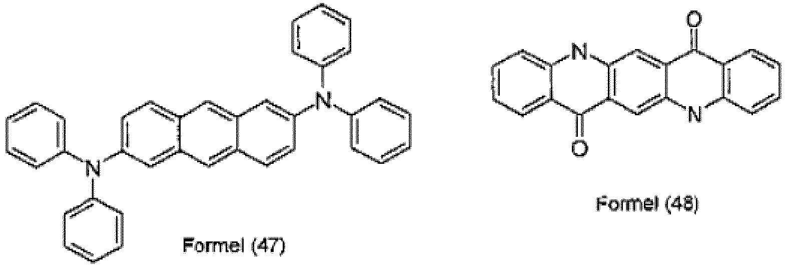 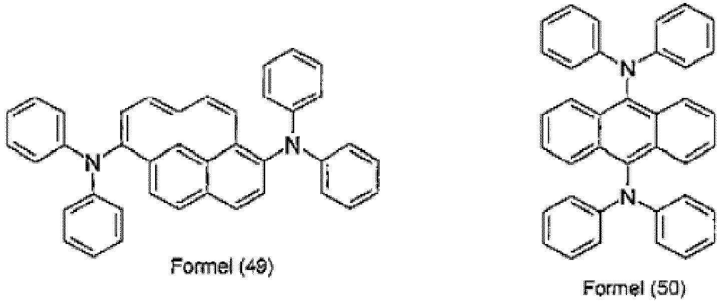 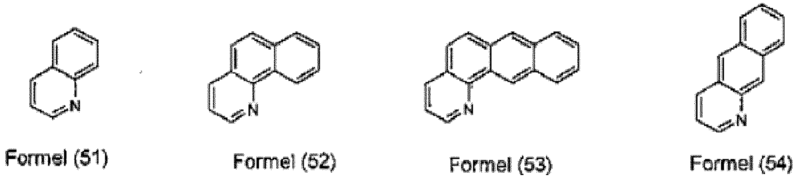 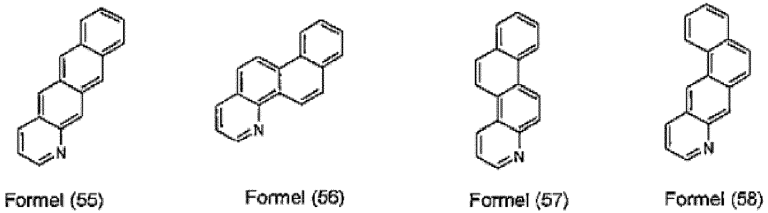 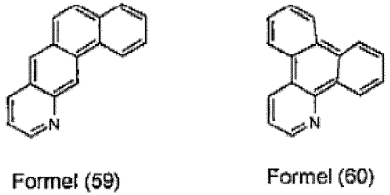 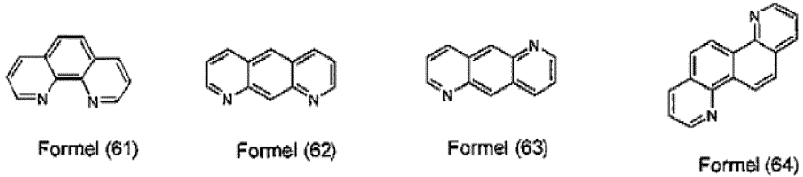 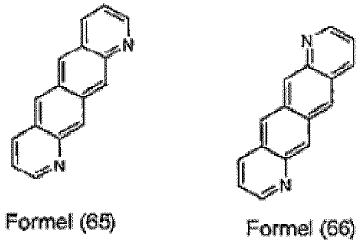 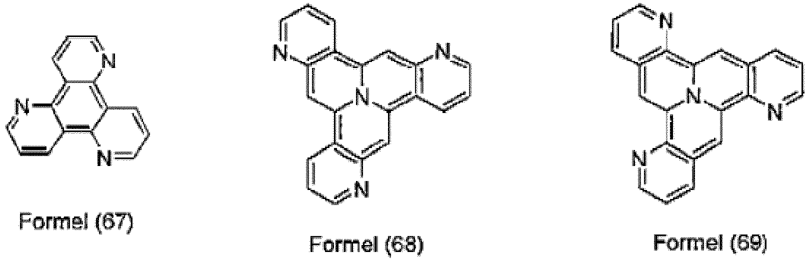 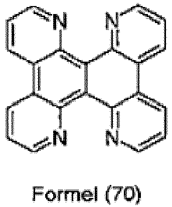 un, deux, trois ou quatre atomes de carbone dans la structure de base aromatique condensée des groupes de formules (1) à (50) pouvant être remplacés par de l'azote.

10. Dispositif électroluminescent organique selon une ou plusieurs des revendications 1 à 9, **caractérisé en ce que** des groupes alkyles, notamment de 3 à 20 atomes de carbone, dans lesquels des atomes d'hydrogène peuvent également être remplacés par F, des groupes alcoxy, en particulier de 3 à 20 atomes de carbone, des groupes aralkyle, en particulier de 7 à 30 atomes de carbone, et des systèmes cycliques aromatiques, en particulier de 6 à 30 atomes de carbone, peuvent être utilisés comme substituants de blindage dans le composé fluorescent à blindage stérique, les groupes aryle dans les groupes aralkyle et les systèmes cycliques aromatiques pouvant également être substitués par un ou plusieurs groupes alkyle ayant 1 à 10 atomes de carbone ;
et/ou **en ce qu'**un ou plusieurs groupes aliphatiques condensés des formules (Cycle-1) à (Cycle-7) (ci-dessous 'Ring-1' à 'Ring-7') sont utilisés comme substituants pour le blindage dans le composé fluorescent à blindage stérique, 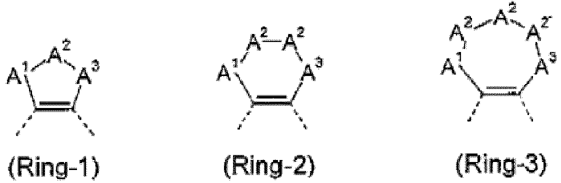 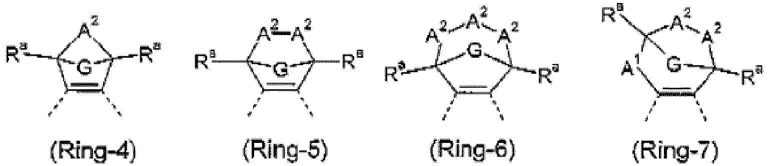 les liaisons en pointillés indiquant la combinaison des deux atomes de carbone dans le corps de base fluorescent et en outre :
A¹, A², A³, étant identiques ou différents pour chaque occurrence C(R^{a})₂, O ou S ;
G étant un groupe alkylène ayant 1, 2 ou 3 atomes de carbone, qui peut être substitué par un ou plusieurs radicaux R^{b}, ou un groupe arylène ortho-lié ayant 5 à 14 atomes de cycle aromatique qui peut être substitué par un ou plusieurs radicaux R^{b} ;
R^{a} étant choisi pour être identique ou différent à chaque occurrence dans le groupe comprenant H, D, F, un groupe alkyle à chaîne droite ayant 1 à 40 atomes de carbone ou un groupe alkyle ramifié ou cyclique ayant 3 à 40 atomes de carbone, qui peuvent chacun être substitués par un ou plusieurs radicaux R^{b}, un système cyclique aromatique ayant 5 à 60 atomes de cycle aromatique, qui peuvent chacun être substitués par un ou plusieurs radicaux R^{b}, ou un groupe aralkyle ayant 5 à 60 atomes de cycle aromatique qui peuvent être substitués par un ou plusieurs radicaux R^{b}, éventuellement deux ou plusieurs substituants R^{a} adjacents pouvant former un système cyclique qui peut être substitué par un ou plusieurs radicaux R^{b} ;
Rb étant choisi dans le groupe comprenant H, D, F, un radical hydrocarboné aliphatique ayant 1 à 20 atomes de carbone, un système cyclique aromatique ayant 5 à 30 atomes de cycle aromatique, deux ou plusieurs substituants R^{b} adjacents pouvant former l'un avec l'autre un système cyclique ;
à condition que, dans ces groupes, deux hétéroatomes ne soient pas directement liés l'un à l'autre.

11. Dispositif électroluminescent organique selon la revendication 10, **caractérisé en ce que** les structures (Cycle-1) à (Cycle-7) ne comportent pas de protons benzyliques acides.

12. Dispositif électroluminescent organique selon la revendication 10 ou 11, **caractérisé en ce que** les structures (Cycle-1) à (Cycle-7) sont choisies parmi les structures suivantes (Cycle-1A) à (Cycle-7-A) (ci-dessous (Ring-1A) à (Ring-7A)), 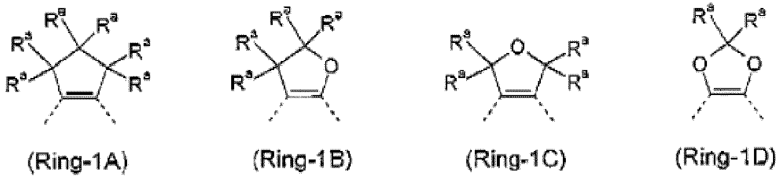 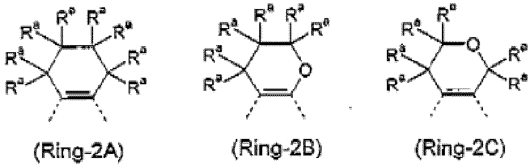 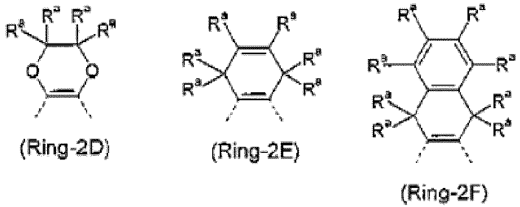 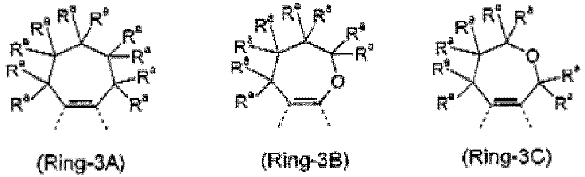 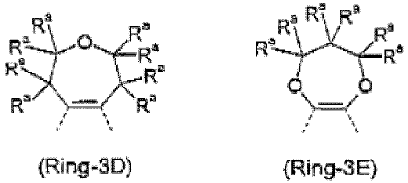 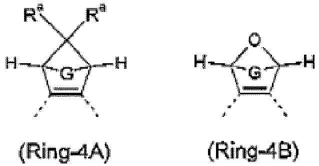 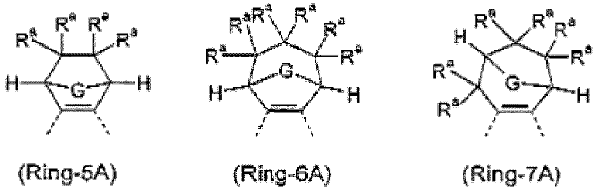 R^{a} et G ayant les significations données dans la revendication 10 et R^{a} n'étant de préférence pas H ou D dans les positions benzyliques.

13. Dispositif électroluminescent organique selon une ou plusieurs des revendications 1 à 12, **caractérisé en ce que** la couche émettrice contient au moins un composé matriciel.

14. Dispositif électroluminescent organique selon la revendication 13, **caractérisé en ce que** le composé matriciel est un ou plusieurs composés choisis dans le groupe comprenant les cétones, les oxydes de phosphine, les sulfoxydes, les sulfones, les triarylamines, les carbazoles, les dibenzofuranes, les indolocarbazoles, les indénocarbazoles, les azacarbazoles, les matériaux de matrice bipolaire, les silanes, les azaboroles, les esters boroniques, les diazasiloles, les diazaphospholes, les triazines, les pyrimidines, les quinoxalines, les complexes de Zn, Al ou Be ou les carbazoles pontés.

15. Procédé de production d'un dispositif électroluminescent organique selon une ou plusieurs des revendications 1 à 14, **caractérisé en ce qu'**au moins une couche est appliquée par un procédé de sublimation et/ou **en ce qu'**au moins une couche est appliquée par voie de dépôt organique en phase vapeur ou à l'aide d'une sublimation par gaz porteur et/ou **en ce qu'**au moins une couche est appliquée à partir d'une solution, par enduction centrifuge ou par un procédé d'impression.
